# EUROPEAN PATENT SPECIFICATION

(11) **EP 3 150 606 B1**
(45) Date of publication and mention of the grant of the patent: **14.08.2019**
(21) Application number: 15187954.1
(22) Date of filing: 01.10.2015
(51) Int. Cl.: C07D 487/04, C09K 11/06

(54) **BENZIMIDAZOLO[1,2-A]BENZIMIDAZOLES CARRYING BENZOFURANE OR BENZOTHIOPHENE GROUPS FOR ORGANIC LIGHT EMITTING DIODES**
BENZIMIDAZOLO[1,2-A]BENZIMIDAZOLES MIT BENZOFURAN-ODER BENZOTHIOPHEN GRUPPEN FÜR ORGANISCHE LICHT EMITTIERENDE DIODEN
BENZIMIDAZOLO[1,2-A]BENZIMIDAZOLES AVEC DES GROUPEMENTS BENZOFURANE OU BENZOTHIOPHÈNE POUR DES DIODES ÉMITTANT DE LA LUMIÈRE

(43) Date of publication of application: 05.04.2017
(73) Proprietor: IDEMITSU KOSAN CO., LTD., Tokyo 100-8321 (JP)
(72) Inventor: SCHÄFER, Thomas, 4410 Liestal (CH); KAWAMURA, Masahiro, Chiba 299-0293 (JP); NAGASHIMA, Hideaki, 4057 Basel (CH); NAKANO, Yuki, Chiba 299-0293 (JP); BARDON, Kristina, 79761 Waldshut-Tiengen (DE)
(74) Representative: Hollah, Dorothee

(56) References cited:
- WO-A1-2012/130709
- WO-A1-2014/009317
- WO-A1-2014/044722

## Description

The present invention relates to compounds of formula (**1**) and their use in electronic devices, especially electroluminescent devices. When used as charge transport material, charge blocker material and/or host material in electroluminescent devices, the compounds of formula I may provide improved efficiency, stability, manufacturability, or spectral characteristics of electroluminescent devices and reduced driving voltage of electroluminescent devices.

Khan, Misbahul Ain; Ribeiro, Vera Lucia Teixeira, Pakistan Journal of Scientific and Industrial Research 43 (2000) 168-170 describes the synthesis of benzimidazo[1,2-a]benzimadozoles 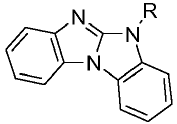 (R = H, Me, Et) by trialkyl phosphite-induced deoxygenation and thermolysis of 1-(o-nitrophenyl)- and 1-(o-azidophenyl)benzimidazoles.

Pedro Molina et al. Tetrahedron (1994) 10029-10036 reports that aza Wittig-type reaction of bis(iminophosphoranes), derived from bis(2-aminophenyl)amine with two equivalents of isocyanate directly provided benzimidazo[1,2,a]benzimidazole derivatives. 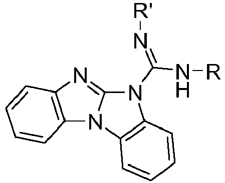 (R = R' = 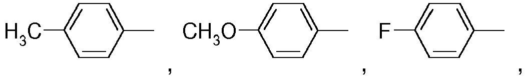 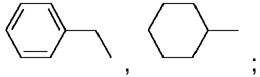 R = 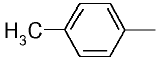 and R' = 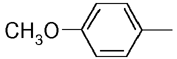 , R = iso-propyl and R' = ethyl)

Kolesnikova, I. V.; Zhurnal Organicheskoi Khimii 25 (1989) 1689-95 describes the synthesis of 5H-benzimidazo[1,2-a]benzimidazole 1,2,3,4,7,8,9,10-octafluoro-5-(2,3,4,5,6-pentafluorophenyl).

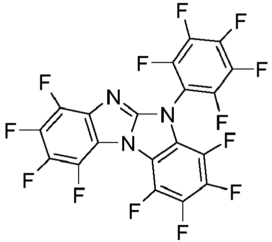

Achour, Reddouane; Zniber, Rachid, Bulletin des Societes Chimiques Belges 96 (1987) 787-92 describes the synthesis of benzimidazobenzimidazoles 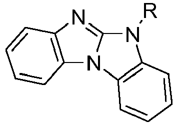 (R = H,-CH(CH₃)₂) which were prepared from benzimidazolinone derivatives.

Hubert, Andre J.; Reimlinger, Hans, Chemische Berichte 103 (1970) 2828-35 describes the synthesis of benzimidazobenzimidazoles 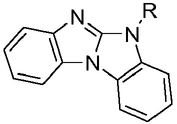 (R = H, CH₃, 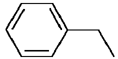 ).

X. Wang et al. Org. Lett. 2012, 14, 452-455 discloses a highly efficient copper-catalyzed synthesis for compounds of formula 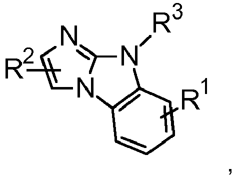 wherein compounds of formula 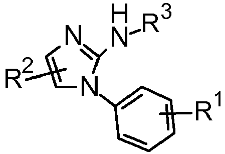 are reacted in the presence of copper acetate (Cu(OAc)₂)/PPh₃/1,10-phenathroline/sodium acetate and oxygen in m-xylene (1 atm) at elevated temperature. Among others the following compounds can be prepared by the described synthesis method: 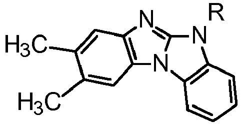 (R = 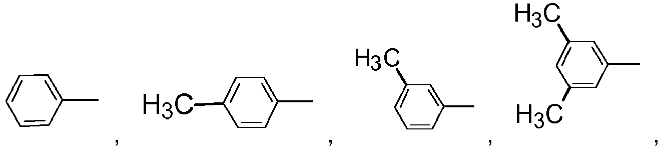 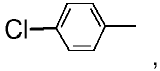 or 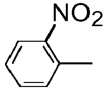 ).

In Eur. J. Org. Chem. 2014, 5986-5997 a new synthesis of benzimidazolo[1,2-a]benzimidazole is described.

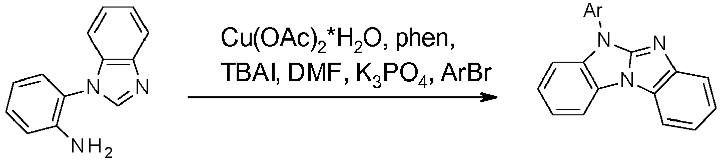

In RSC Advances 2014, 4, 21904-21908 a new synthesis of benzimidazolo[1,2-a]benzimidazole is described. 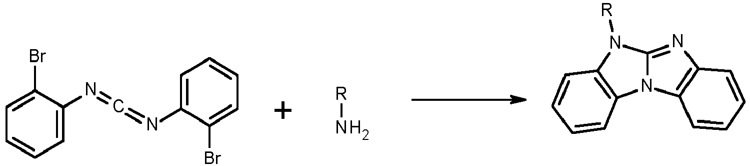 It is mentioned - as a general statement - that these polycyclic molecules have - besides other applications - also attracted great interest in the field of electroluminescent devices.

WO2011/160757 relates to an electronic device comprising an anode, cathode and at least one organic layer which contains a compound of formulae 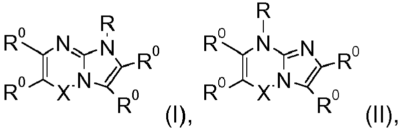 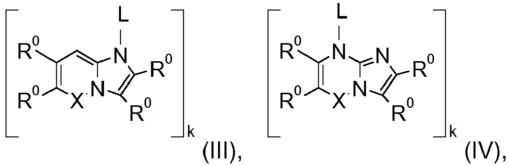 wherein X may be a single bond and L may be a divalent group. The following 4H-Imidazo[1,2-a]imidazole compounds are explicitly disclosed: 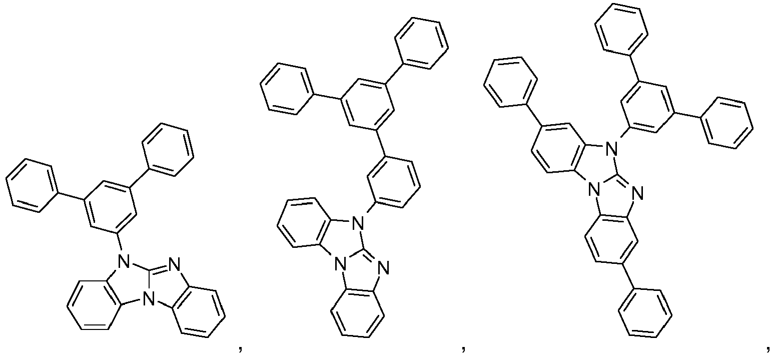 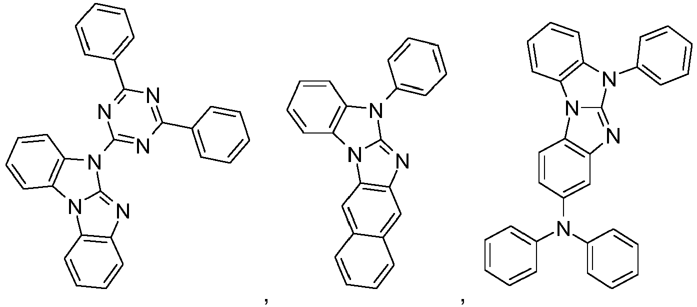 and

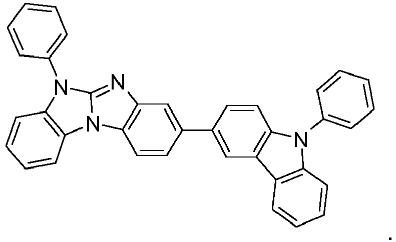

WO2012/130709 relates to 4H-Imidazo[1,2-a]imidazoles, 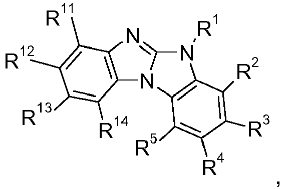 such as , for example, 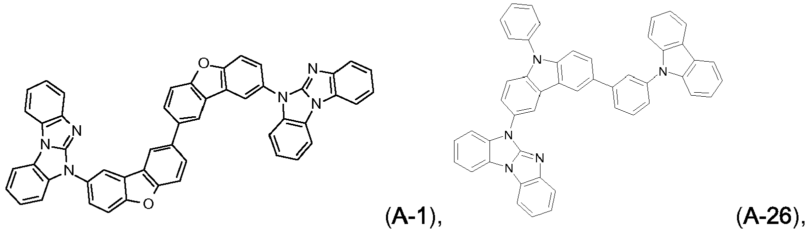 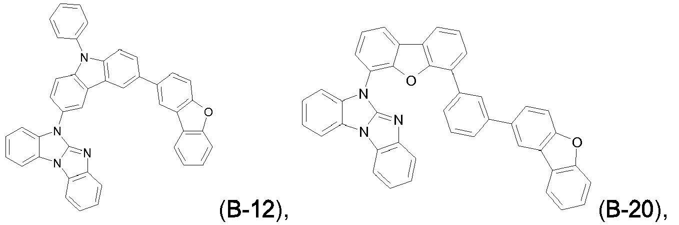 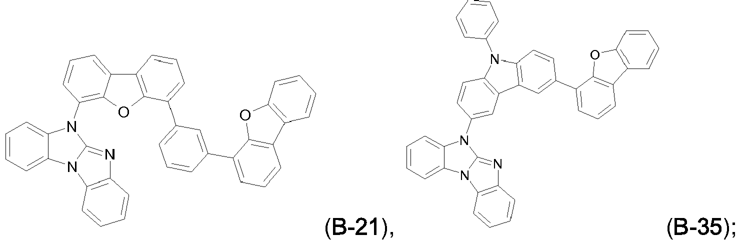 a process for their production and their use in electronic devices, especially electroluminescent devices.

WO2014/009317 relates to compounds of formula 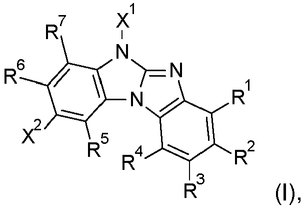 especially compounds of formula 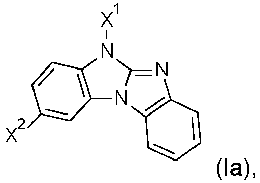 such as, for example, 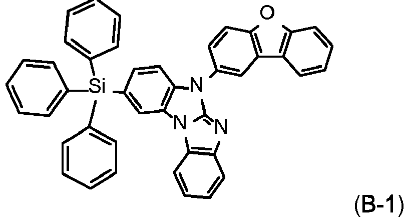 and 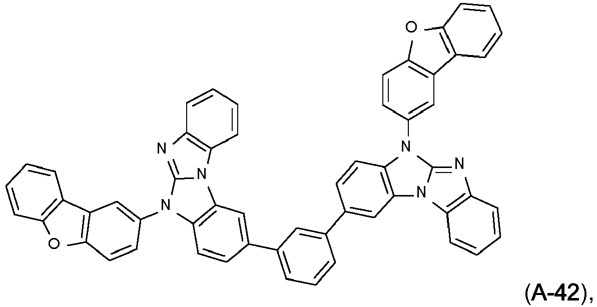 a process for their production and their use in electronic devices, especially electroluminescent devices. The 2,5-disubstituted benzimidazo[1,2-a]benzimidazole derivatives are suitable hole transporting materials, or host materials for phosphorescent emitters.

WO2014/044722 relates to compounds of formula 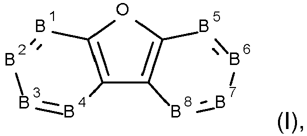 which are characterized in that they substituted by benzimidazo[1,2-a]benzimidazo-5-yl and/or benzimidazo[1,2-a]benzimidazo-2,5-ylene groups and in that at least one of the substituents B¹, B², B³, B⁴, B⁵, B⁶, B⁷ and B⁸ represents N, a process for their production and their use in electronic devices, especially electroluminescent devices.

European patent application no. 13191100.0 relates to compounds of formula 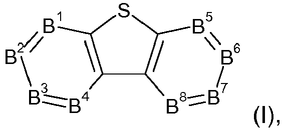 which are characterized in that they are substituted by benzimidazo[1,2-a]benzimidazo-5-yl and/or benzimidazo[1,2-a]benzimidazo-2,5-ylene groups and in that at least one of the substituents B¹, B², B³, B⁴, B⁵, B⁶, B⁷ and B⁸ represents N; a process for their production and their use in electronic devices, especially electroluminescent devices.

Benzimidazo[1,2-a]benzimidazo-5-yl and benzimidazo[1,2-a]benzimidazo-2-ylsubstituted benzimidazolo[2,1-b][1,3]benzothiazole derivatives are described in WO2015/014791.

European patent application no. EP14197947.9 describes carbazol compounds carrying benzimidazolo[1,2-a]benzimidazole groups of the following structure. 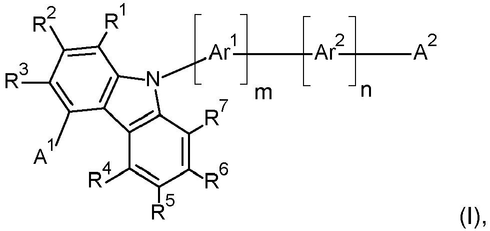 wherein
m is 1, or 2, n is 0, 1, or 2,
Ar¹ and Ar² are independently of each other a C₆-C₂₄aryl group, which can optionally be substituted by G, a C₁₂-C₃₀heteroaryl group, which can optionally be substituted by G,
A¹ is a group of formula

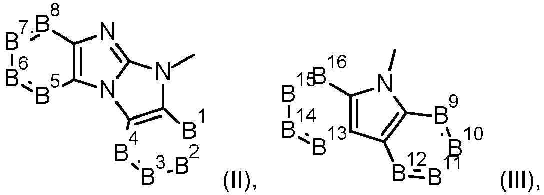

European patent application no. EP14197952.6 describes dibenzofurane compounds carrying benzimidazolo[1,2-a]benzimidazole groups of the following structure. 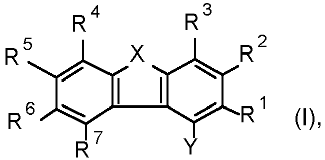 wherein
X is O or S;
Y is a group of formula -[Ar¹]ₐ-[Ar²]_{b}-[Ar³]_{c}-A¹;

A¹ is a group of formula 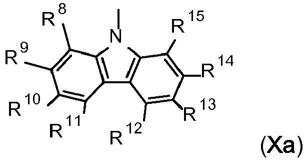 or

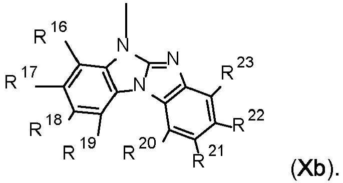

Notwithstanding these developments, there remains a need for organic light emitting devices comprising new materials, especially host ( = matrix) materials, hole transport materials and/or electron/exciton blocker materials to provide long lifetimes, improved efficiency, stability, manufacturability, driving voltage and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide hole transport materials, electron/exciton blocker materials and host (= matrix) materials for use in OLEDs. The materials should be suitable especially for OLEDs which comprise at least one emitter, which is preferably a phosphorescence emitter, for example at least one green, red or yellow emitter, especially at least one green emitter or at least one red emitter.

Furthermore, the materials should be suitable for providing OLEDs which ensure good efficiencies, good operative lifetimes and a high stability to thermal stress, and a low use and operating voltage of the OLEDs. Examples or embodiments not covered by the scope of the claims do not form part of the invention.

Said object is solved by heterocyclic derivatives of formula (1);

**A-[(B₁)ₒ-(B₂)ₚ-(B₃)_{q}-(B₄)ᵣ-Z]_{z}** **(1)**

wherein
B₁, B₂, B₃ and B₄ are independently of each other a C₆-C₂₄arylene group, which can optionally be substituted by G, or a 6 membered heteroarylene group, which can optionally be substituted by G;
o is 0 or 1, p is 0 or 1, q is 0 or 1, r is 0 or 1, wherein at least one of o, p, q and r is 1;
Z represents a dibenzofuran group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G; or a dibenzothiophene group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
   and/or
   two adjacent substituents of the dibenzofuran group or of the dibenzothiophene group may form together with the atoms to which they are bonded a ring structure, which can optionally be substituted by G;
A is a heterocyclic group represented by formula (2) or formula (3); 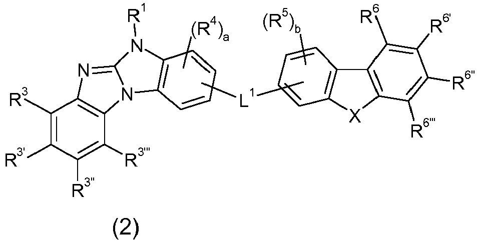 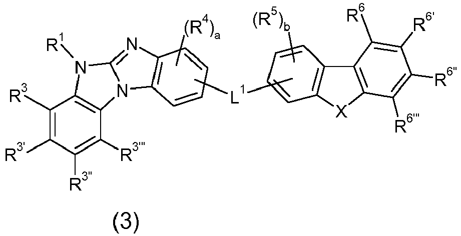 wherein X is NR⁷;
   L¹ is single bond, a C₆-C₂₄arylene group, which can optionally be substituted by G, or a C₁-C₂₄heterocyclic group, which can optionally be substituted by G;
   R¹, R³, R^{3'}, R^{3"}, R^{3'''}, R⁴, R⁵, R⁶, R^{6'}, R^{6"}, and R6''', are independently of each other **H or** a group of formula -(B⁵)ₛ-(B⁶)ₜ-(B⁷)ᵤ-(B⁸)ᵥ-R¹⁰;
   B⁵, B⁶, B⁷ and B⁸ are independently of each other a C₆-C₂₄arylene group, which can optionally be substituted by G, or a C₂-C₃₀heteroarylene group, which can optionally be substituted by G; s is 0 or 1, t is 0 or 1, u is 0 or 1, v is 0 or 1;
   R¹⁰ is H, a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G;
      and/or
   two adjacent groups of the groups R¹, R³, R^{3'}, R^{3"}, R^{3'''}, R⁴, R⁵, R⁶, R^{6'}, R^{6"}, R^{6'''} may form together with the atoms to which they are bonded a ring structure, which can optionally be substituted by G;
   a is 1, 2 or 3;
   b is 1, 2 or 3;
   D is -CO-, -COO-, -S-, -SO-, -SO₂-, -O-, -NR⁶⁵-, -SiR⁷⁰R⁷¹-, -POR⁷²-, -CR⁶³=CR⁶⁴- or -C≡C;
   E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
   G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
   R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
   R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
   R⁶⁵ and R⁶⁶ may form together with the atom to which they are bonded a five or six membered ring,
   R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
   R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
   R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by-O-,
   R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
   R⁷² is a C₁-C₁₈alkyl group, a C₆-C₁₈aryl group, or a C₆-C₁₈aryl group, which is substituted by C₁-C₁₈alkyl;
   z is 1; wherein R⁷ is replaced by-(B₁)ₒ-(B₂)ₚ-(B₃)_{q}-(B₄)ᵣ-Z.

The combination of the linking group B₁, B₂, B₃ and/or B₄ with the group Z gives rise to materials that are highly suitable in devices that emit green, red or yellow light, preferably green or red light, more preferably green light. Moreover, a balanced hole transport and/or electron/exciton blocking in devices is achieved resulting in low voltages and high external quantum efficiencies (EQE's) and/or long lifetimes.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device, such as an organic light-emitting diode (OLED).

The compounds of formula (**1**) can in principal be used in any layer of an EL device, but are preferably used as host, hole transport and/or electron/exciton blocking material. Particularly, the compounds of formula (1) are used as host material for green, red and yellow, preferably green and red, more preferably green light emitting phosphorescent emitters.

Hence, a further subject of the present invention is directed to a hole transport layer, comprising a compound of formula (**1**) according to the present invention.

A further subject of the present invention is directed to an emitting layer, comprising a compound of formula (**1**) according to the present invention. In said embodiment a compound of formula (**1**) is preferably used as host material or as co-host material together with one or more, preferably one, further host materials. More preferably, a combination of a compound of formula (**1**) and a co-host material together with a phosphorescent emitter is used.

A further subject of the present invention is directed to an electron/exciton blocking layer, comprising a compound of formula (**1**) according to the present invention.

The terms halogen, alkyl, alkoxy, cycloalkyl, aryl, aryloxy, aralkyl, heteroaryl, arylene, heteroarylene generally have the following meaning, if said groups are not further specified in specific embodiments mentioned below:
Halogen is fluorine, chlorine, bromine and iodine, preferably fluorine.

C₁-C₂₅alkyl, preferably C₁-C₂₄alkyl and more preferably C₁-C₁₈alkyl are typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

The alkyl groups mentioned above can optionally be substituted by E and/or interrupted by D. Preferably, the alkyl groups mentioned above are unsubstituted or can optionally be substituted by E.

C₁-C₂₅alkoxy groups and preferably C₁-C₁₈alkoxy groups are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is preferably C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted by G.

C₆-C₃₀aryl, preferably C₆-C₂₄aryl and more preferably C₆-C₁₈aryl, which is unsubstituted or optionally can be substituted by G, is most preferably phenyl, 4-methylphenyl, 4-methoxyphenyl, naphthyl, especially 1-naphthyl, or 2-naphthyl, biphenylyl, triphenylyl, fluoranthenyl, terphenylyl, pyrenyl, 2- or 9-fluorenyl, phenanthryl, or anthryl, which may be unsubstituted or substituted by G. Phenyl, 1-naphthyl and 2-naphthyl are examples of a C₆-C₁₀aryl group.

C₂-C₆₀heteroaryl, preferably C₂-C₃₀heteroaryl, more preferably C₂-C₁₃ heteroaryl represents a ring with five, six or seven ring atoms or a condensed ring system, wherein nitrogen, oxygen or sulfur are the possible heteroatoms, and is typically a heterocyclic group with five to 60 atoms, preferably with five to 30 atoms, more preferably with five to 13 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl, dibenzo[f,h]quinazolinyl or phenoxazinyl, which can be unsubstituted or substituted by G. Benzimidazo[1,2-a]benzimidazo-5-yl, benzimidazo[1,2-a]benzimidazo-2-yl, carbazolyl and dibenzofuranyl are examples of a C₂₋C₁₄heteroaryl group.

The group C₁₋C₆₀heteroaryl, preferably C₁₋C₃₀heteroaryl, more preferably C₁₋C₂₄heteroaryl, most preferably C₂-C₁₃ heteroaryl, even more preferably C₂-C₆₀heteroaryl, C₂₋C₃₀heteroaryl, C₂₋C₂₄heteroaryl, C₂₋C₁₃heteroaryl may be unsubstituted or substituted by G.

A C₂-C₁₃heteroaryl group is for example, benzimidazo[1,2-a]benzimidazo-5-yl ( 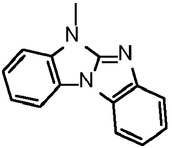 ), benzimidazo[1,2-a]benzimidazo-2-yl ( 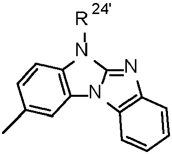 ), benzimidazolo[2,1-b][1,3]benzothiazolyl, benzimidazolo[2,1-b][1,3]benzoxazole, carbazolyl, dibenzofuranyl, or dibenzotihophenyl, which can be unsubstituted or substituted by G, especially by C₆-C₁₀aryl, or C₆-C₁₀aryl, which is substituted by C₁-C₄alkyl; or C₂-C₁₃heteroaryl.

C₁-C₆₀heteroaryl, preferably C₁-C₃₀heteroaryl, more preferably C₁-C₂₄heteroaryl, most preferably C2-C13 heteroaryl, even more preferably C₂-C₆₀heteroaryl, C₂-C₃₀heteroaryl, C₂-C₂₄heteroaryl, C₂-C₁₃heteroaryl means that the heteroaryl residue comprises at least one, preferably at least 2 carbon atoms and at most 60 carbon atoms in the base skeleton (without substituents). The further atoms in the heteroaryl base skeleton are heteroatoms (N, O and/or S).

R^{24'} is in each case independently C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, phenanthronyl, triphenylenyl, fluoranthenyl or biphenylyl.

C₁₋C₂₄heterocyclic group, preferably C₁₋C₁₃heterocyclic group, more preferably C₂-C₁₃ heterocyclic group represents a ring with five, six or seven ring atoms or a condensed ring system, wherein nitrogen, oxygen or sulfur are the possible heteroatoms, and is typically a heterocyclic group with five to 24 atoms, preferably with five to 13 atoms. The heterocyclic group may be a C₁₋C₂₄heteroaryl group as defined above or a C₁₋C₂₄heterocycloalkyl group which may be unsubstituted or substituted by G. Typical C₁₋C₂₄heterocycloalkyl groups are oxetan, tetrahydrofuran, tetrahydropyran, oxepane, dioxane, azetidine, pyrrolidine, piperidine, hexahydroazepine, hexahydrodiazepin, tetrahydrothiophene, thietan, tetrahydrothiopyran, thiepan, morpholine as well as bridged heterocycloalkyl systems such as oxabicyclo[4.4.0]decane and azabicyclo[2,2,1]undecane.

C₆-C₂₄arylene groups, preferably C₆-C₁₀arylene groups, which optionally can be substituted by G, preferably C₆-C₁₀arylene groups, which optionally can be substituted by G, are more preferably phenylene, 4-methylphenylene, 4-methoxyphenylene, naphthylene, especially 1-naphthylene, or 2-naphthylene, biphenylylene, triphenylylene, fluoranthenylene, terphenylylene, pyrenylene, 2- or 9-fluorenylene, phenanthrylene, or anthrylene, which may be unsubstituted or substituted by G. Preferred C₆-C₂₄arylen groups, preferably C₆-C₁₀arylene groups are 1,3-phenylene, 3,3'-biphenylylene, 3,3'-m-terphenylene, 2- or 9-fluorenylene, phenanthrylene, which may be unsubstituted or substituted by G.

C₂-C₃₀heteroarylene groups, preferably C₂-C₁₄heteroarylene groups, which are unsubstituted or optionally can be substituted by G, represent a ring with five to seven ring atoms or a condensed ring system, wherein nitrogen, oxygen or sulfur are the possible heteroatoms, and is typically a heterocyclic group with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted by G. Preferred C₂-C₃₀heteroarylen groups are pyridylene, triazinylene, pyrimidinylene, carbazolylene, dibenzofuranylene, azatriphenylylene, azadibenzofurylene, azadibenzothiophenylene, azacarbazolylene, quinolonylene, isoquinolinylene, quinoxalinylene, quinazolinylene, phenanthrolinylene, phenanthridinylene, benzo[h]quinolonylene, benz[h]isoquinolinylene, benzo[f]isoquinolinylene, benzo[f]quinolinylene, benzo[h]quinazolinylene, benzo[f]quinazolinylene, dibenzo[f,h]quinolonylene, dibenzo[f,h]isoquinolonylene, dibenzo[f,h]quinoxalinylene, dibenzo[f,h]quinazolinylene and benzimidazo[1,2-a]benzimidazo-2,5-ylene ( 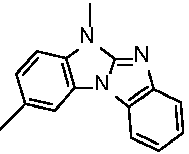 ), which can be unsubstituted or substituted by G, preferably substituted by C₆-C₁₀aryl, C₆-C₁₀aryl which is substituted by C₁-C₄alkyl; or C₂₋C₁₃heteroaryl.

A 6 membered heteroarylene group which can optionally be substituted by G represents a ring with six ring atoms, which is not a condensed ring system, preferably comprising one, two or three heteroatoms, wherein nitrogen is a preferred heteroatom. Examples are pyridylene, pyrimidylene, pyridazinylene, pyrazinylene and triazinylene which may optionally substituted by G, preferably pyrimidylene, pyridylene and triazinylene.

If a substituent occurs more than one time in a group, it can be different in each occurrence.

Halo-C₁-C₈alkyl is an alkyl group (as defined above) where at least one of the hydrogen atoms is replaced by a halogen atom. Examples are -CF₃, -CF₂CF₃, -CF₂CF₂CF₃, -CF(CF₃)₂, -(CF₂)₃CF₃, and -C(CF₃)₃.

The wording "substituted by G" means that one, or more, especially one, two or three substituents G might be present. Preferred substituents G are mentioned below.

The wording "substituted by E" means that one, or more, especially one, two or three substituents E might be present. Preferred substituents E are mentioned below.

As described above, the aforementioned alkyl groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y}')-CH₂-O-R^{y}, where R^{y} is C₁-C₁₈alkyl, C₅-C₁₂cycloalkyl, phenyl, C₇-C₁₅phenylalkyl, and R^{y}' embraces the same definitions as R^{y} or is H.

An alkyl group substituted by E is, for example, an alkyl group where at least one of the hydrogen atoms is replaced by F. Examples are -CF₃, -CF₂CF₃, -CF₂CF₂CF₃, -CF(CF₃)₂, -(CF₂)₃CF₃, and -C(CF₃)₃.

D is -CO-, -COO-, -S-, -SO-, -SO₂-, -O-, -NR⁶⁵-, -SiR⁷⁰R⁷¹-, -POR⁷²-, -CR⁶³=CR⁶⁴- or -C≡C. Suitable residues R⁶³, R⁶⁴, R⁶⁵, R⁷⁰ R⁷¹ and R⁷² are mentioned above. D is preferably -CO-,-COO-, -S-, -SO-, -SO₂-, -O-, -NR⁶⁵-, wherein R⁶⁵ is preferably C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, triphenylyl or biphenylyl, or C₂-C₃₀heteroaryl, such as, for example, benzimidazo[1,2-a]benzimidazo-2-yl ( 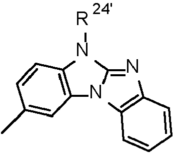 ), carbazolyl, dibenzofuranyl, which can be unsubstituted or substituted especially by C₆-C₁₀aryl, or C₆-C₁₀aryl, which is substituted by C₁-C₄alkyl; or C₂-C₁₃heteroaryl.

E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen. E is preferably-OR⁶⁹; -SR⁶⁹; -NR⁶⁵R⁶⁶; -COR⁶⁸; -COOR⁶⁷; -CON⁶⁵R⁶⁶; or-CN; wherein R⁶⁵, R⁶⁶, R⁶⁷, R⁶⁸ and R⁶⁹ are preferably independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, triphenylyl or biphenylyl.

G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O. G is preferably - OR⁶⁹, -SR⁶⁹, -NR⁶⁵R⁶⁶; a C₁-C₁₈alkyl group, a C₆-C₁₈aryl group, a C₆-C₁₈aryl group, which is substituted by F, or C₁-C₁₈alkyl; a C₂-C₂₄heteroaryl group, or a C₂-C₂₄heteroaryl group, which is substituted by F, or C₁-C₁₈alkyl; wherein R⁶⁵, R⁶⁶ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, G is a C₆-C₁₈aryl group like phenyl, tolyl, triphenylyl or biphenylyl, or a C₆-C₂₄heteroaryl group like dibenzothiophenylyl, dibenzofuranyl, pyridyl, triazinyl, pyrimidinyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl or dibenzo[f,h]quinazolinyl.

### Group A

A is a heterocyclic group represented by formula (2) or formula (3); 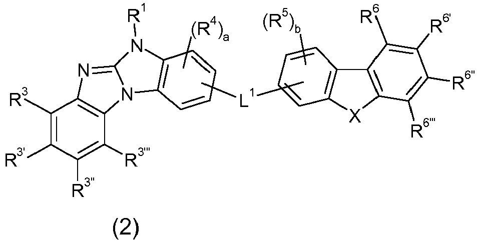 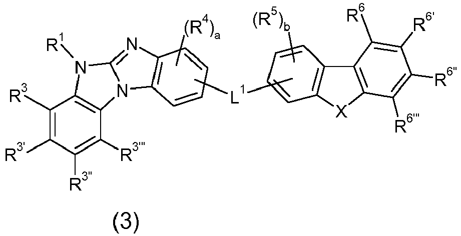
wherein X is NR⁷;
L¹ is single bond, a C₆-C₂₄arylene group, which can optionally be substituted by G, or a C₁-C₂₄heterocyclic group, which can optionally be substituted by G; preferably L¹ is a single bond, 1,2-phenylene, 1,3-phenylene or 1,4-phenylene, more preferably a single bond;
R¹, R³, R^{3'}, R^{3"}, R^{3'''}, R⁴, R⁵, R⁶, R^{6'}, R^{6"}, and R6''' are independently of each other **H or** a group of formula -(B⁵)ₛ-(B⁶)ₜ-(B⁷)ᵤ-(B⁸)ᵥ-R¹⁰, preferably, R¹, R³, R^{3'}, R^{3"}, R^{3'''}, R⁴, R⁵, R⁶, R^{6'}, R^{6"}, R^{6'''}, are independently of each other **H or** a group of formula -(B⁵)ₛ-(B⁶)ₜ-(B⁷)ᵤ-(B⁸)ᵥ-R¹⁰. B⁵, B⁶, B⁷ and B⁸ are independently of each other a C₆-C₂₄arylene group, which can optionally be substituted by G, or a C₂-C₃₀heteroarylene group, which can optionally be substituted by G; s is 0 or 1, t is 0 or 1, u is 0 or 1, v is 0 or 1;
R¹⁰ is H, a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G;
   and/or
   two adjacent groups of the groups R¹, R³, R^{3'}, R^{3"}, R^{3'''}, R⁴, R⁵, R⁶, R^{6'}, R^{6"}, R6''' may form together with the atoms to which they are bonded a ring structure, which can optionally be substituted by G;
a is 0, 1, 2 or 3, preferably 0 or 1, more preferably 0;
b is 0, 1, 2 or 3, preferably 0 or 1, more preferably 0;
D is -CO-, -COO-, -S-, -SO-, -SO₂-, -O-, -NR⁶⁵-, -SiR⁷⁰R⁷¹-, -POR⁷²-, -CR⁶³=CR⁶⁴- or -C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl or C₁-C₁₈alkoxy; C₁-C₁₈alkyl; or C₁-C₁₈alkyl which is interrupted by-O-;
R⁶⁵ and R⁶⁶ are independently of each other a C₆-C₁₈aryl group; a C₆-C₁₈aryl which is substituted by C₁-C₁₈alkyl or C₁-C₁₈alkoxy; a C₁-C₁₈alkyl group; or a C₁-C₁₈alkyl group, which is interrupted by-O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; or
R⁶⁵ and R⁶⁶ may form together with the atom to which they are bonded a five or six membered ring;
R⁶⁷ is a C₆-C₁₈aryl group; a C₆-C₁₈aryl group, which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R⁶⁸ is H; a C₆-C₁₈aryl group; a C₆-C₁₈aryl group, which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R⁶⁹ is C₆-C₁₈aryl; a C₆-C₁₈aryl, which is substituted by C₁-C₁₈alkyl or C₁-C₁₈alkoxy; a C₁-C₁₈alkyl group; or a C₁-C₁₈alkyl group, which is interrupted by -O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl;
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; and
R⁷² is a C₁-C₁₈alkyl group, a C₆-C₁₈aryl group, or a C₆-C₁₈aryl group, which is substituted by C₁-C₁₈alkyl, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl, more preferably phenyl;
   wherein one and/or two of R¹, R³, R^{3'}, R^{3"}, R^{3'''}, R⁴, R⁵, R⁶, R^{6'}, R^{6"}, R^{6'''} or R⁷, preferably 1, is/are replaced by -(B₁)ₒ-(B₂)ₚ-(B₃)_{q}-(B₄)ᵣ-Z.

The heterocyclic groups represented by formula (2) or (3) are isomeric groups and can also be depicted as follows:

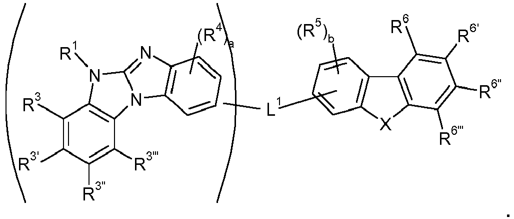

Preferred groups D, E and G are mentioned above.

R¹, R³, R^{3'}, R^{3"}, R^{3'''}, R⁴, R⁵, R⁶, and R6''' are independently of each other H or a group of formula -(B⁵)ₛ-(B⁶)ₜ-(B⁷)ᵤ-(B⁸)ᵥ-R¹⁰.

Preferred groups B⁵, B⁶, B⁷ and B⁸ are independently of each other a C₆-C₁₀arylene group, which can optionally be substituted by G, or a C₂-C₁₈heteroarylene group, which can optionally be substituted by G.

More preferably, the groups B⁵, B⁶, B⁷ and B⁸ are independently of each other:
Phenylene, naphthylene, especially 1-naphthylene, or 2-naphthylene, biphenylylene, triphenylylene, terphenylylene, pyrenylene, 2- or 9-fluorenylene, phenanthrylene, or anthrylene, which may be unsubstituted or substituted by G;
benzothiophenylene, thianthrenylene, furylene, furfurylene, 2H-pyranylene, benzofuranylene, isobenzofuranlene, dibenzofuranylene ( 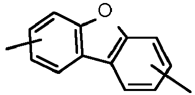 ), dibenzothiophenylene ( 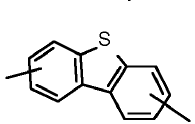 ), carbazolylene ( 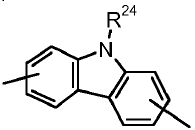 or 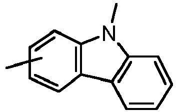 ), imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, pyrimidinylene, benzimidazo[1,2-a]benzimidazo-2,5-ylene ( 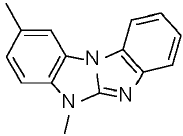 ),which can be unsubstituted or substituted by G. R²⁴ is a C₆-C₂₄aryl group, or a C₂-C₃₀heteroaryl group, which can optionally be substituted by G, wherein G is as defined in above; wherein the lines are bonding sites; 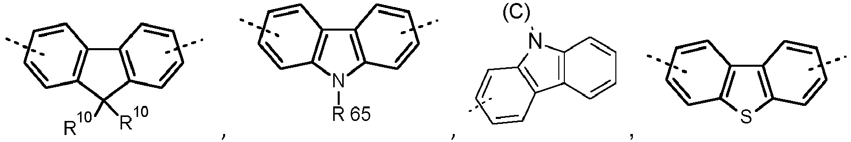 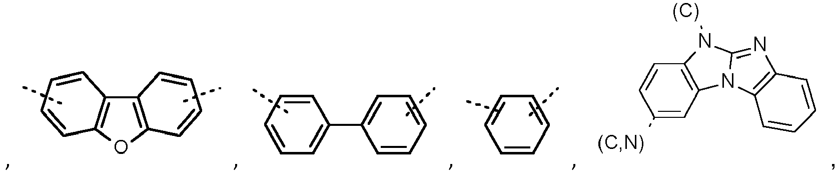 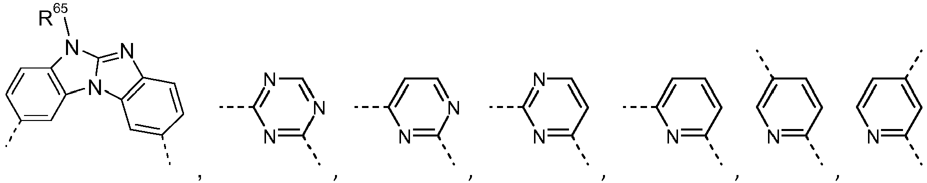 which can be unsubstituted or substituted by G. R⁶⁵ is a C₆-C₁₈aryl group; a C₆-C₁₈aryl which is substituted by C₁-C₁₈alkyl or C₁-C₁₈alkoxy; a C₁-C₁₈alkyl group; or a C₁-C₁₈alkyl group, which is interrupted by-O-, preferably C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; R¹⁰ a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G; and/or two adjacent groups of the groups R¹⁰ may form together with the atom to which they are bonded a ring structure, which can optionally be substituted by G; wherein G is as defined in above; wherein the dotted lines are bonding sites;
wherein (C)- has the meaning that the bonding site of the group B⁵, B⁶, B⁷ or B⁸ is linked to a C-atom, and (N)- has the meaning that the bonding site of the group B⁵, B⁶, B⁷ or B⁸ is linked to a N-atom, and (C,N) has the meaning that the bonding site of the group B⁵, B⁶, B⁷ or B⁸ is linked to a C or N-atom.

B⁵, B⁶, B⁷ and B⁸ are most preferably in each occurrence independently of each other a group of the formula: 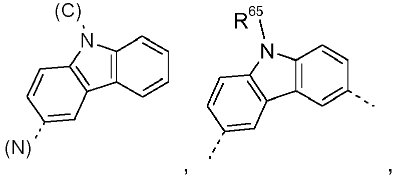 preferably 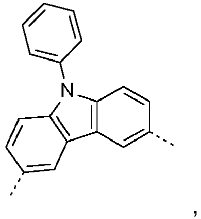 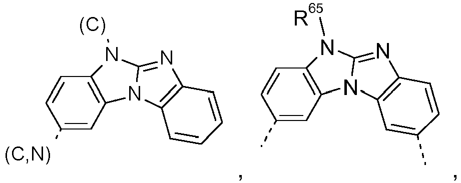 preferably 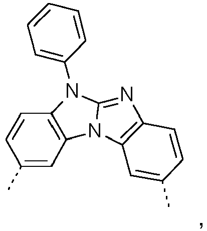 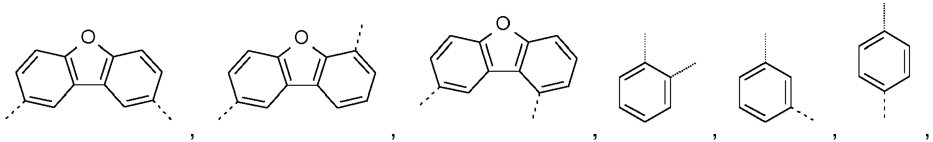 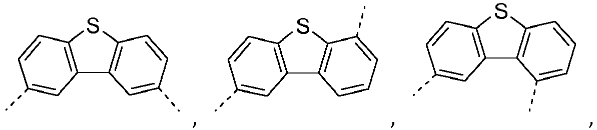 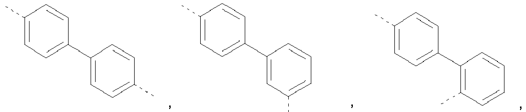 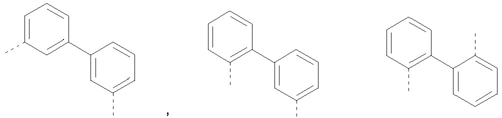 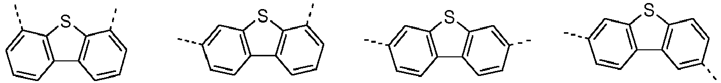 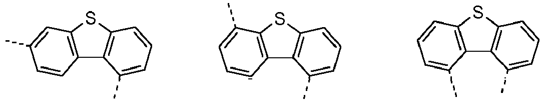 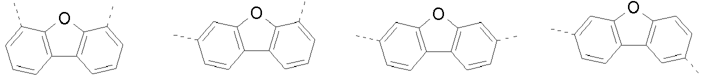 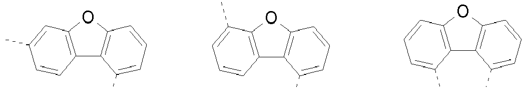 wherein (C)- has the meaning that the bonding site of the group B⁵, B⁶, B⁷ or B⁸ is linked to a C-atom, and (N)- has the meaning that the bonding site of the group B⁵, B⁶, B⁷ or B⁸ is linked to a N-atom, and (C,N) has the meaning that the bonding site of the group B⁵, B⁶, B⁷ or B⁸ is linked to a C or N-atom; and the dotted lines are bonding sites.

s is 0 or 1, t is 0 or 1, u is 0 or 1, v is 0 or 1, preferably, s is 0 or 1, t is 0 or 1 and u and v are 0, more preferably, s is 0 or 1 and t, u and v are 0, most preferably s, t, u and v are 0.

R¹⁰ is H, a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G.

R¹⁰ is preferably H, phenyl, phenyl which is substituted by one or two phenyl groups or a group of the following formula: 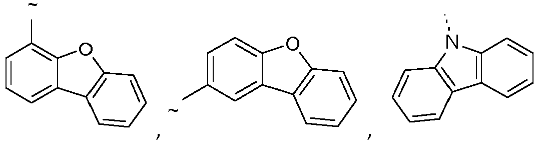 or 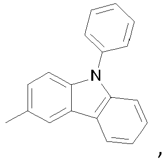 wherein ∼ is a bonding site and the aforementioned groups may be unsubstituted or substituted by G.

Most preferably, R¹, R³, R^{3'}, R^{3"}, R^{3'''}, R⁴, R⁵, R⁶, R^{6'}, R^{6"}, and R6'''. are independently of each other H, phenyl or a group of the following formula 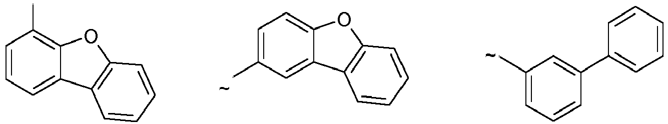 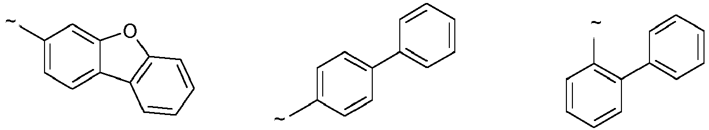 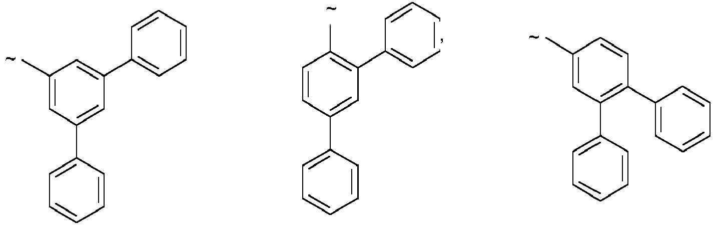 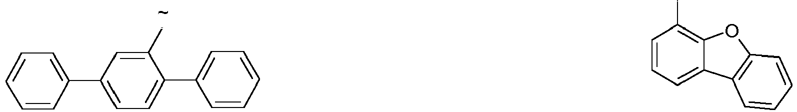 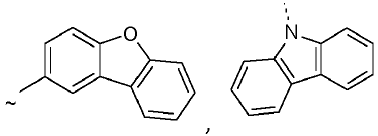 or 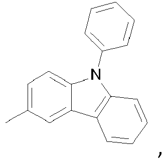 wherein R⁷, is replaced by -(B₁)ₒ-(B₂)ₚ-(B₃)_{q}-(B₄)ᵣ-Z; and ∼ are bonding sites, Even more preferably, R¹, R³, R^{3'}, R^{3"}, R^{3'''}, R⁴, R⁵, R⁶, R^{6'}, R^{6"}, and R6''' are independently of each other H, phenyl or a group of the following formula 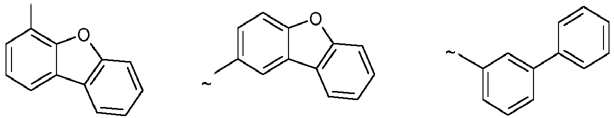 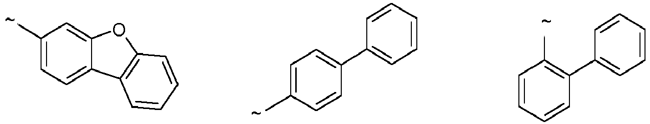 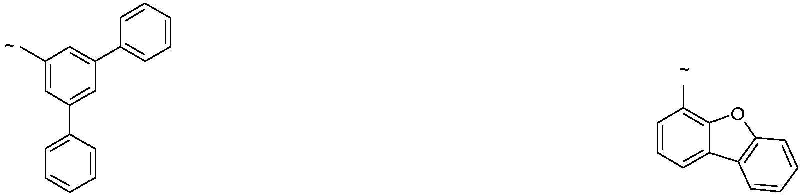 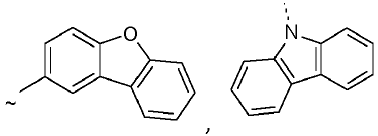 or 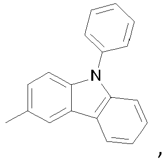 wherein R⁷, is replaced by -(B₁)ₒ-(B₂)ₚ-(B₃)_{q}-(B₄)ᵣ-Z; and ∼ are bonding sites.

Further most preferably, R¹, R^{3"}, R^{6'} are independently of each other H, phenyl or a group of the following formula 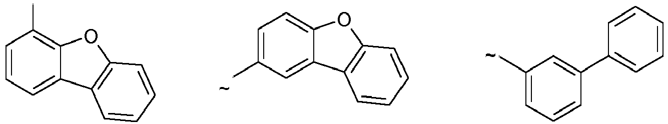 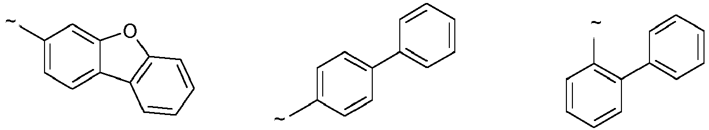 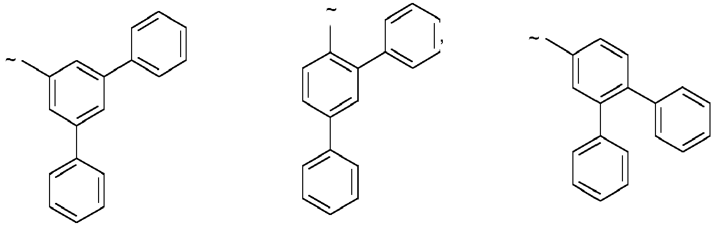 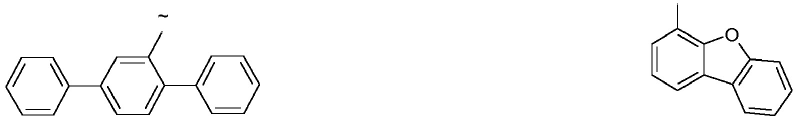 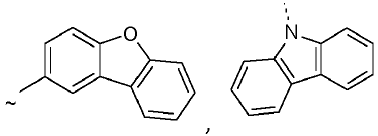 or 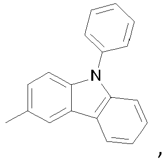 and R³, R^{3'}, R^{3'''}, R⁴, R⁵, R⁶, R^{6"}, R^{6'''} are H; and wherein one and/or two of R¹, R^{3"}, R^{6'}, R^{6"} and ∼ are bonding sites; preferably H, phenyl or a group of the following formula 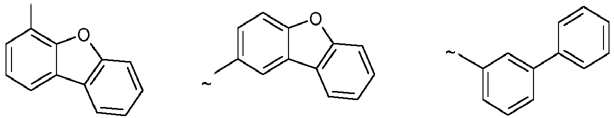 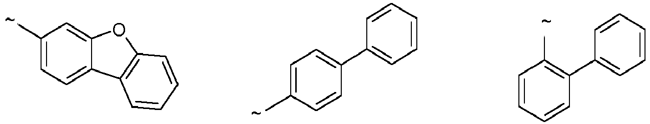 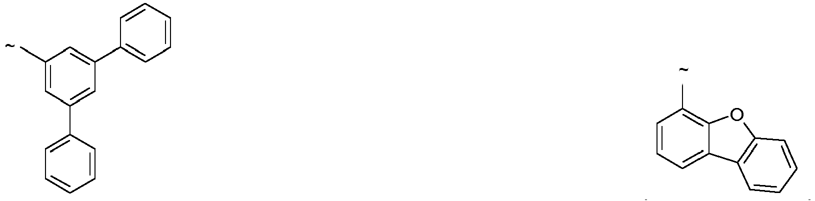 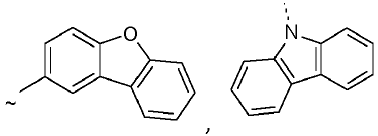 or 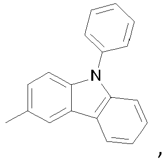 and R³, R^{3'}, R^{3'''}, R⁴, R⁵, R⁶, R^{6"} nd R^{6'''} are H; and
wherein R⁷, is by -(B₁)ₒ-(B₂)ₚ-(B₃)_{q}-(B₄)ᵣ-Z; and ∼ are bonding sites.

Even further most preferably, R¹ is independently of each other phenyl or a group of the following formula 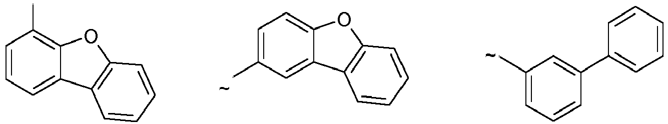 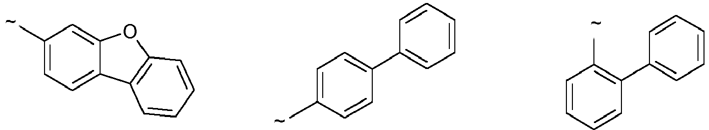 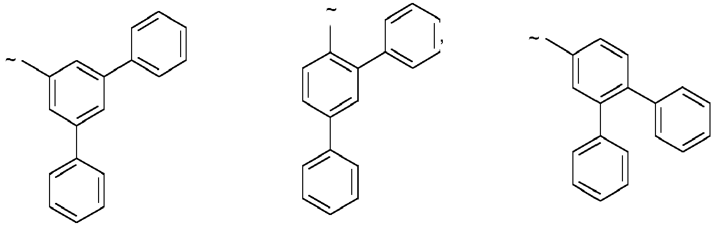 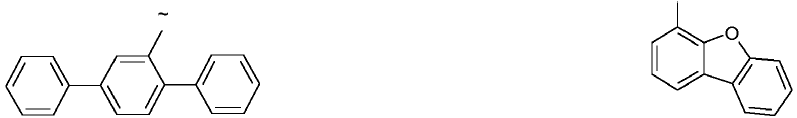 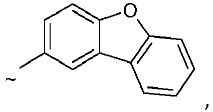 -or 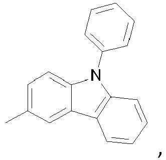 and R³, R^{3'}, R^{3"}, R^{3'''}, R⁴, R⁵, R⁶, R^{6'}, R^{6"} and R^{6'''} are H; and
Even further most preferably, R¹ is independently of each other phenyl or a group of the following formula 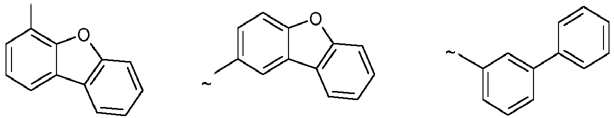 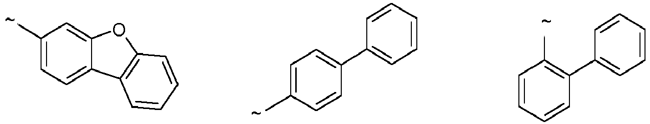 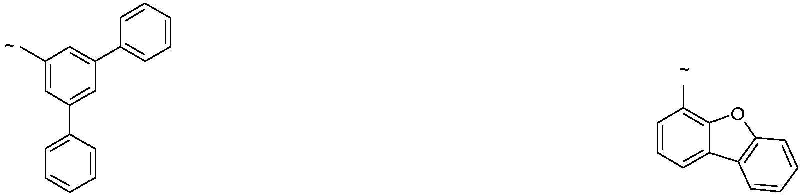 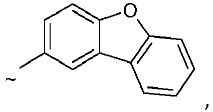 or 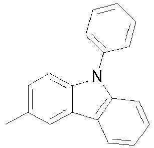 and R³, R^{3'}, R^{3"}, R^{3'''}, R⁴, R⁵, R⁶, R^{6'}, R^{6"} and R^{6'''} are H; and
R7 is replaced by -(B₁)ₒ-(B₂)ₚ-(B₃)_{q}-(B₄)ᵣ-Z; and ∼ are bonding sites;
X is NR⁷, wherein-R⁷, is replaced by -(B₁)ₒ-(B₂)ₚ-(B₃)_{q}-(B₄)ᵣ-Z.

L¹ in the heterocyclic derivative of formula (1) is a single bond, a C₆-C₂₄arylene group, which can optionally be substituted by G, or a C₁-C₂₄heterocyclic group, which can optionally be substituted by G; preferably L¹ is a single bond, 1,2-phenylene, 1,3-phenylene or 1,4-phenylene, more preferably a single bond.

Particularly preferred groups A are therefore represented by formula (4), formula (5), formula (6), formula (7), formula (8), formula (9), formula (10), formula (11), formula (12), formula (13), formula (14) or formula (15) and by formula (20), formula (21), formula (22), formula (23), formula (24), formula (25), formula (26), formula (27), formula (28), formula (29), formula (30), formula (31), formula (32), formula (33), formula (34), formula (35), formula (36), formula (37), formula (38) or formula (39): 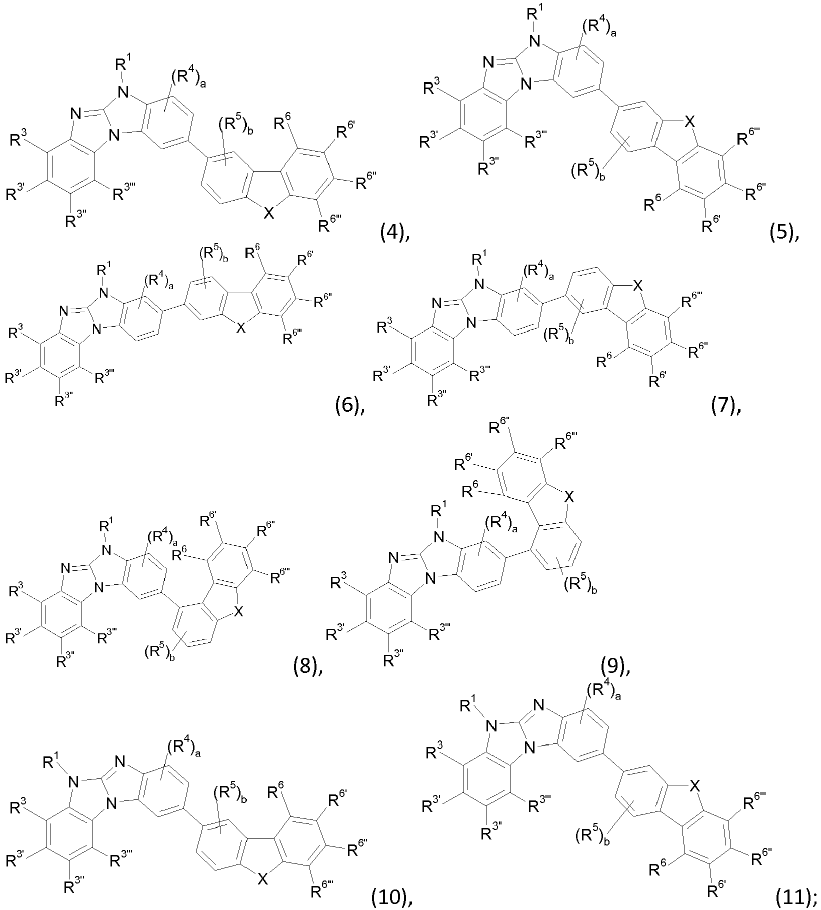 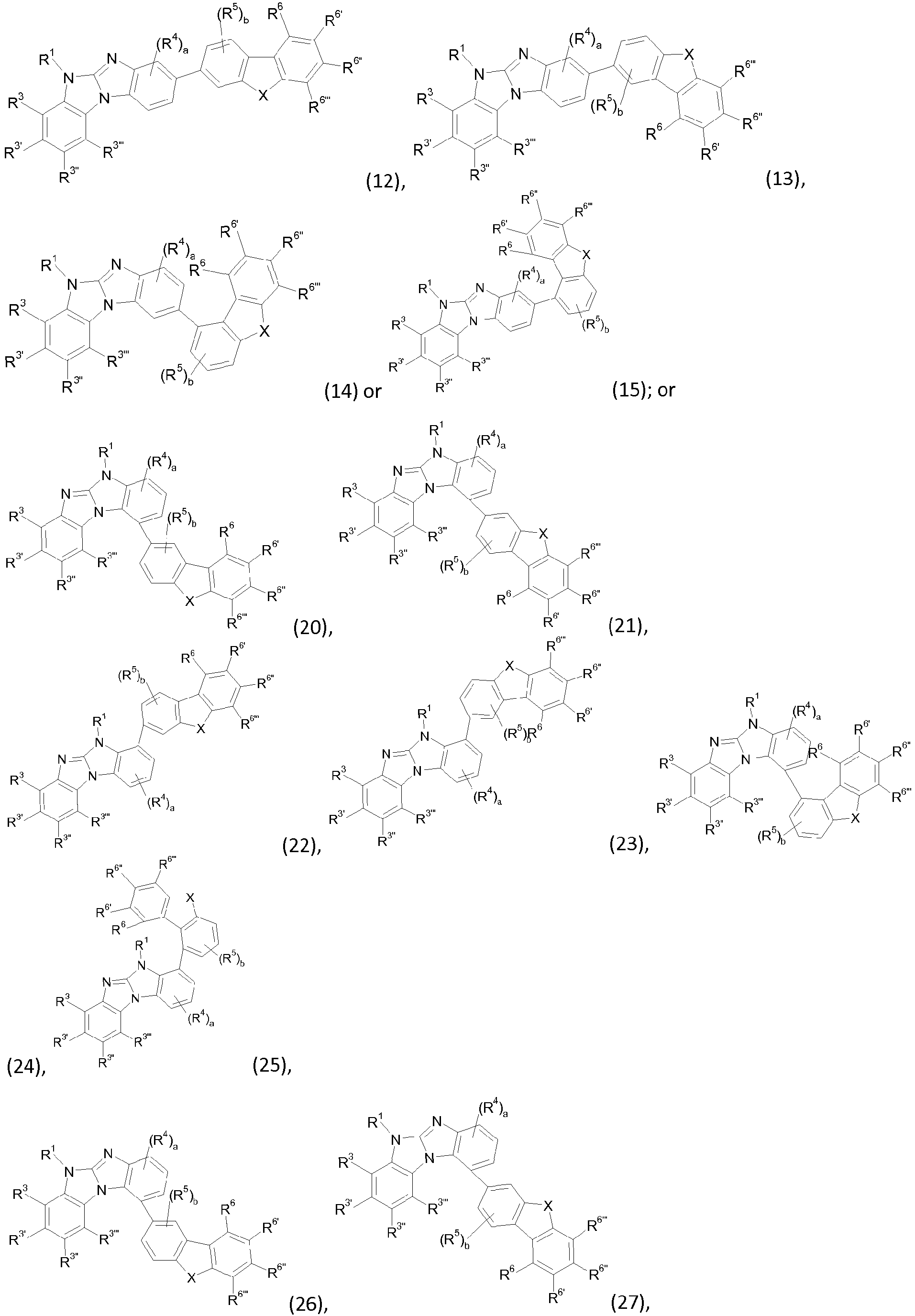 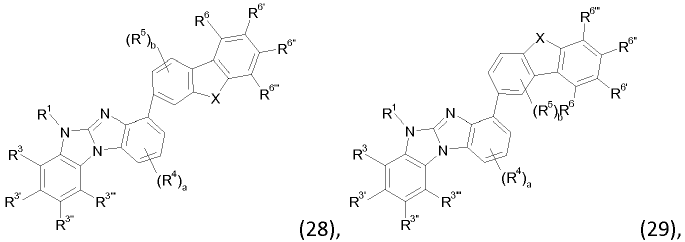 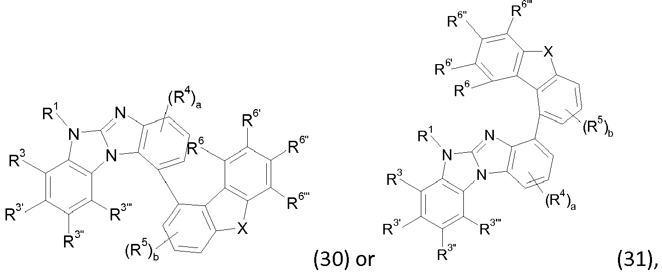 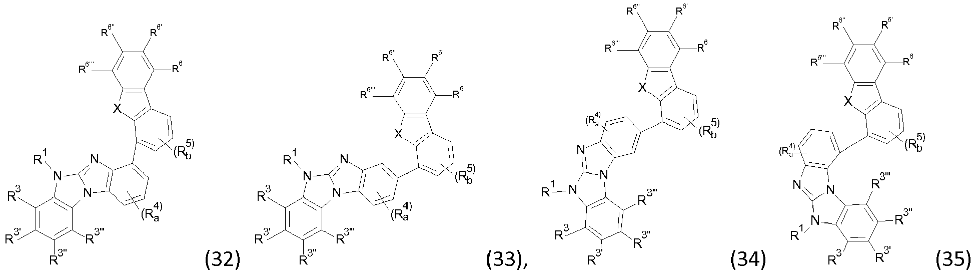 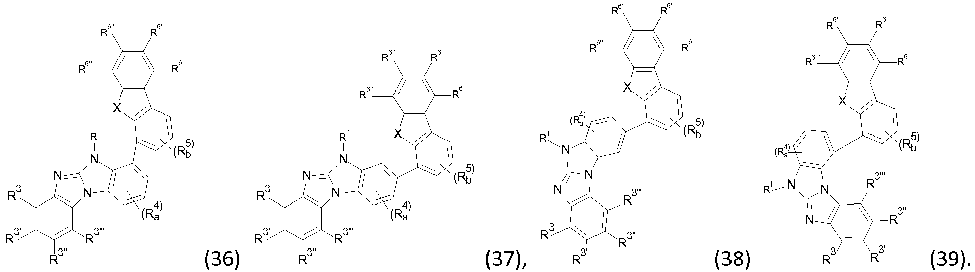 wherein X is NR⁷.

Preferred residues R¹, R³, R^{3'}, R^{3"}, R^{3'''}, R⁴, R⁵, R⁶, R^{6'}, R^{6"}, and R6''' preferred indices a and b and a preferred group X of the compounds of formula (4), formula (5), formula (6), formula (7), formula (8), formula (9), formula (10), formula (11), formula (12), formula (13), formula (14) or formula (15), formula (20), formula (21), formula (22), formula (23), formula (24), formula (25), formula (26), formula (27), formula (28), formula (29), formula (30), formula (31), formula (32), formula (33), formula (34), formula (35), formula (36), formula (37), formula (38) and formula (39) are the residues, indices and groups mentioned before;
wherein R⁷, is replaced by -(B₁)ₒ-(B₂)ₚ-(B₃)_{q}-(B₄)ᵣ-Z.

Even more preferred groups A are represented by formula (4), formula (5), formula (6), formula (7), formula (8), formula (9), formula (10), formula (11), formula (12), formula (13), formula (14), formula (15) or formula (38); of said groups A, groups A are represented by groups A are represented by formula (4), formula (5), formula (8) and formula (38) are further preferred.

Most preferred groups A are represented by formula (4), formula (5), formula (8) and formula (38), wherein X is NR⁷.

Further even more preferred groups A are: 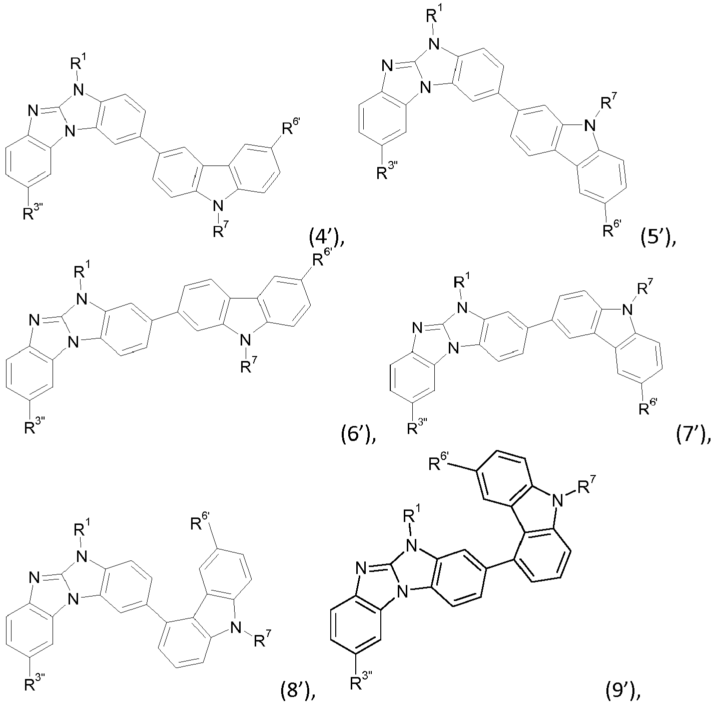 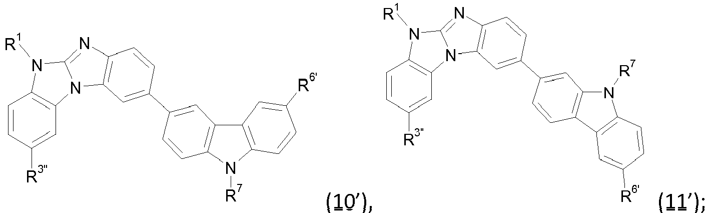 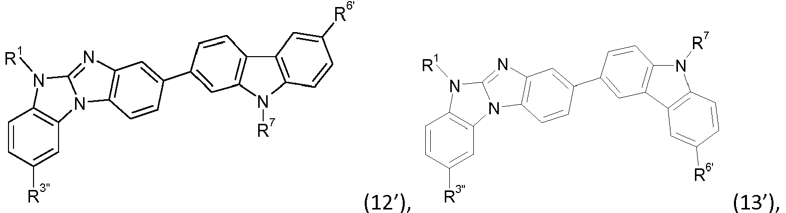 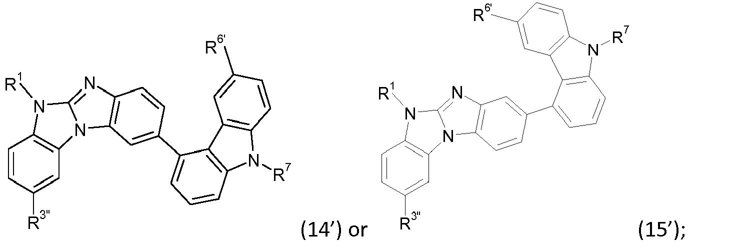 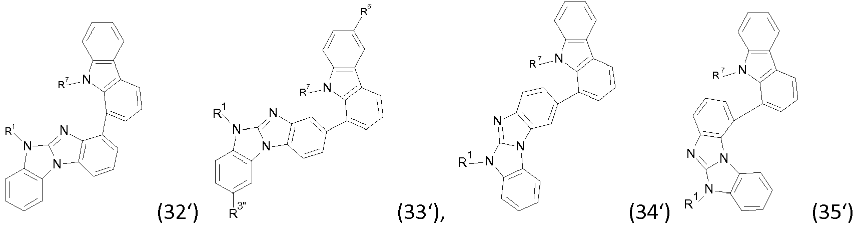 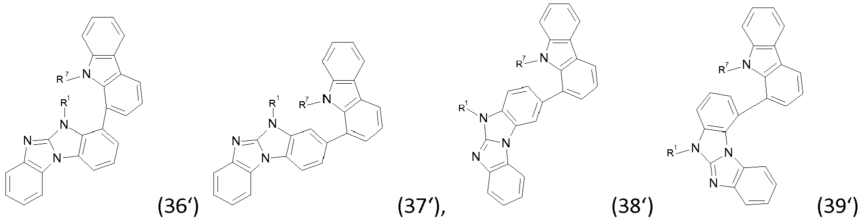 of said groups A, groups A are represented by formula (4'), formula (5'), formula (8') and formula (38') are further preferred.

Preferred residues R¹, R^{3"}, R^{6'} of the compounds of formula (4'), formula (5'), formula (6'), formula (7'), formula (8'), formula (9'), formula (10'), formula (11'), formula (12'), formula (13'), formula (14'), formula (15'), formula (32'), formula (33'), formula (34'), formula (35'), formula (36'), formula (37'), formula (38') and formula (39') are the residues mentioned before; wherein one and/or two, preferably one, of R¹, R^{3"}, R^{6'} and R⁷ is/are replaced by -(B₁)ₒ-(B₂)ₚ-(B₃)_{q}-(B₄)ᵣ-Z.

More preferably, R^{3"} and R^{6'} in the compounds of formula (4'), formula (5'), formula (6'), formula (7'), formula (8'), formula (9'), formula (10'), formula (11'), formula (12'), formula (13'), formula (14') and formula (15') are H and one and/or two, preferably one, of R¹ and R⁷ is/are replaced by -(B₁)ₒ-(B₂)ₚ-(B₃)_{q}-(B₄)ᵣ-Z and the other one of R¹ and R⁷ - in the case that only one of R¹ and R⁷ is -(B₁)ₒ-(B₂)ₚ-(B₃)_{q}-(B₄)ᵣ-Z - is phenyl.

Most preferably, R^{3"} and R^{6'} in the compounds of formula (4'), formula (5'), formula (6'), formula (7'), formula (8'), formula (9'), formula (10'), formula (11'), formula (12'), formula (13'), formula (14') and formula (15') are H, R¹ is phenyl, biphenyl, triphenylyl and R⁷ is replaced by -(B₁)ₒ-(B₂)ₚ-(B₃)_{q}-(B₄)ᵣ-Z. R⁷ is replaced by -(B₁)ₒ-(B₂)ₚ-(B₃)_{q}-(B₄)ᵣ-Z.

### Group -(B₁)ₒ-(B₂)ₚ-(B₃)_{q}-(B₄)ᵣ

B₁, B₂, B₃ and B₄ in group -(B₁)ₒ-(B₂)ₚ-(B₃)_{q}-(B₄)ᵣ-
are independently of each other a C₆-C₂₄arylene group, which can optionally be substituted by G, or a 6 membered heteroarylene group, which can optionally be substituted by G.

Preferred C₆-C₂₄arylene groups are phenylene, naphthylene, especially 1-naphthylene, or 2-naphthylene, biphenylylene, triphenylylene, terphenylylene, pyrenylene, 2- or 9-fluorenylene, phenanthrylene or anthrylene, which are unsubstituted or can optionally be substituted by G. Most preferred C₆-C₂₄arylene groups are: 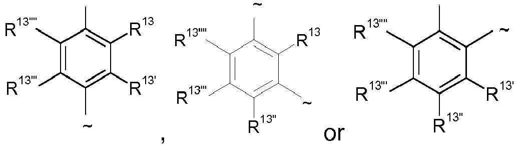 wherein
R¹³, R^{13'}, R^{13"}, R^{13"'} and R^{13""} are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
∼ are bonding sites to the neighboring groups.

Suitable and preferred groups R⁶³, R⁶⁴, R⁶⁵, R⁶⁶, R⁶⁷, R⁶⁸, R⁶⁹, R⁷⁰, R⁷¹, R⁷² are mentioned above. Preferred alkyl groups, aryl groups, heteroaryl groups, and preferred groups D, E and G are mentioned above.

Preferred 6 membered heteroarylene groups are pyridinediyl, pyrimidinediyl, pyridazinediyl and pyrazinediyl, which are unsubstituted or can optionally be substituted by G.

Most preferred 6 membered heteroarylene groups are: 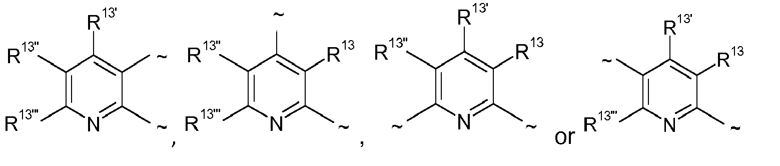 wherein
R¹³, R^{13'}, R^{13"} and R^{13"'} are defined above, and
∼ are bonding sites to the neighboring groups.

Preferably, B₁, B₂, B₃ and B₄ are:
independently of each other a C₆-C₂₄arylene group, which can optionally be substituted by G, preferably phenylene, biphenylylene, triphenylylene, naphthylene, especially 1-naphthylene, or 2-naphthylene, terphenylylene, pyrenylene, 2- or 9-fluorenylene, phenanthrylene or anthrylene, which are unsubstituted or can optionally be substituted by G;
more preferably 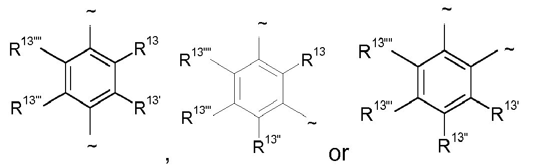 wherein
R¹³, R^{13'}, R^{13"}, R^{13"} and R^{13""} are defined above, and
∼ are bonding sites to the neighboring groups.

o is 0 or 1, p is 0 or 1, q is 0 or 1, r is 0 or 1, wherein at least one of o, p, q and r is 1; preferably, o is 1, p is 0 or 1 and q and r are 0.

Examples for suitable groups -(B₁)ₒ-(B₂)ₚ-(B₃)_{q}-(B₄)ᵣ- are: 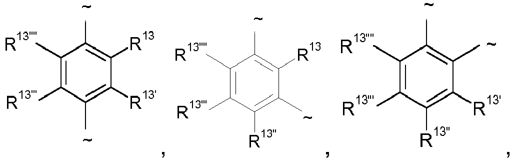 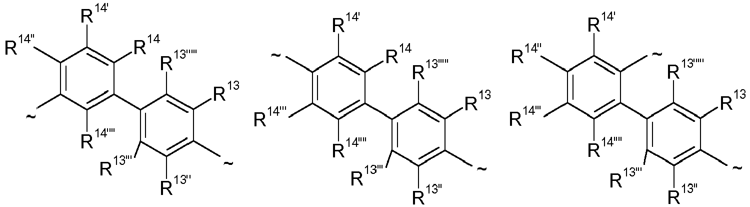 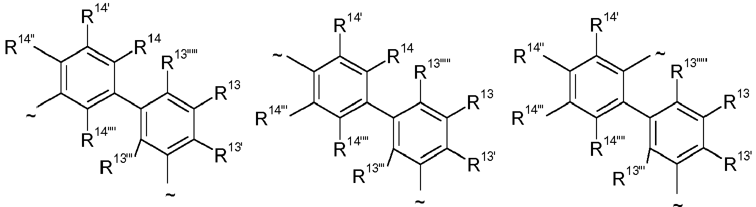 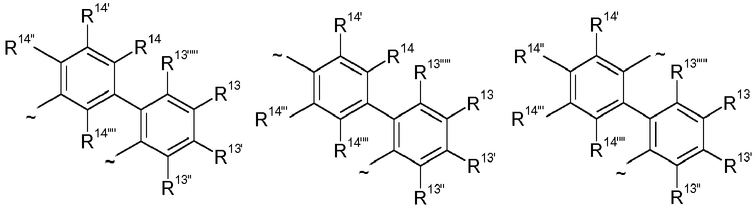 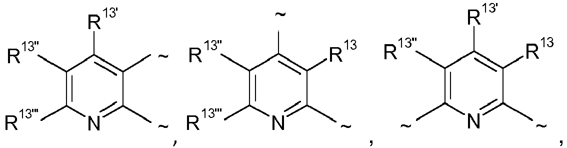 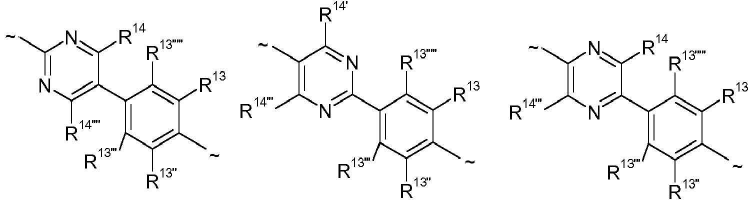 wherein
R¹³, R^{13'}, R^{13"}, R^{13"'}, R^{13""}, R¹⁴, R^{14'}, R^{14"'}, R^{14'"} and R^{14""}
are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C;
E is -OR69, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
∼ are bonding sites to the neighboring groups.

Suitable and preferred groups R⁶³, R⁶⁴, R⁶⁵, R⁶⁶, R⁶⁷, R⁶⁸, R⁶⁹, R⁷⁰, R⁷¹, R⁷² are mentioned above. Preferred alkyl groups, aryl groups, heteroaryl groups, and preferred groups D, E and G are mentioned above.

Preferably, R¹³, R^{13'}, R^{13"}, R^{13"'}, R^{13""}, R¹⁴, R^{14'}, R^{14"'}, R^{14"'} and R^{14""} are H; and
∼ are bonding sites to the neighboring groups.

### Group Z

Z represents a dibenzofuran group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G; or a dibenzothiophene group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
and/or
two adjacent substituents of the dibenzofuran group or of the dibenzothiophene group may form together with the atoms to which they are bonded a ring structure, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴- or -C≡C;
E is -OR69, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O.

Suitable and preferred groups R⁶³, R⁶⁴, R⁶⁵, R⁶⁶, R⁶⁷, R⁶⁸, R⁶⁹, R⁷⁰, R⁷¹, R⁷² are mentioned above. Preferred alkyl groups, aryl groups, heteroaryl groups, and preferred groups D, E and G are mentioned above.

Preferably, Z is represented by one of the following formulae (16), (17), (18) or (19), preferably by one of formulae (16) or (18) 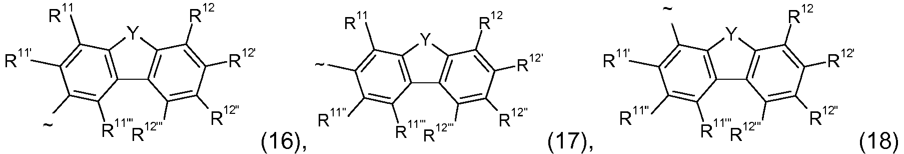 or 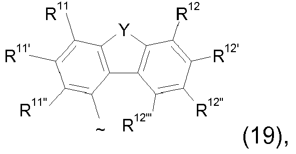 wherein
Y is O or S;
R¹¹, R^{11'}, R^{11"}, R^{11"'}, R¹², R^{12"} and R^{12'"} are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
or
two adjacent groups R¹¹, R^{11'}, R^{11"}, R^{11"'}, R¹², R^{12'}, R^{12"} and R^{12'"} may form together with the atoms to which they are bonded a ring structure;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
∼ is the bonding site to the group A-(B₁)ₒ-(B₂)ₚ-(B₃)_{q}-(B₄)ᵣ-.

Suitable and preferred groups R⁶³, R⁶⁴, R⁶⁵, R⁶⁶, R⁶⁷, R⁶⁸, R⁶⁹, R⁷⁰, R⁷¹, R⁷² are mentioned above. Preferred alkyl groups, aryl groups, heteroaryl groups, and preferred groups D, E and G are mentioned above.

Preferably, R¹¹, R^{11'}, R^{11"}, R^{11"'}, R¹², R^{12'}, R^{12'"} and R^{12'"} are H or CN, most preferably H; and ∼ are bonding sites to the neighboring groups.

Most preferred groups Z are therefore: 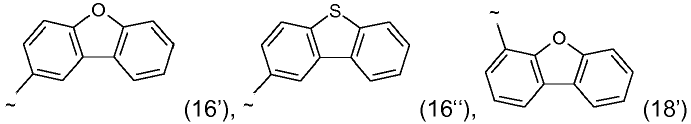 and

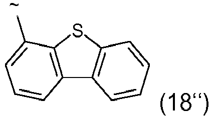

### Heterocyclic derivative of formula (1)

The heterocyclic derivatives according to the present invention are represented by formula (1):

**A-[(B₁)ₒ-(B₂)ₚ-(B₃)_{q}-(B₄)ᵣ-Z]_{z}** (1)

wherein
z is 1 1; and
wherein R⁷ is/are replaced by -(B₁)ₒ-(B₂)ₚ-(B₃)_{q}-(B₄)ᵣ-Z.

The groups A, -(B₁)ₒ-(B₂)ₚ-(B₃)_{q}-(B₄)ᵣ- and Z have been defined before.

Preferred heterocyclic derivatives of formula (1) are therefore heterocyclic derivatives of formula (1'):

**A-(B₁)ₒ-(B₂)ₚ-(B₃)_{q}-(B₄)ᵣ-Z** **(1')**

wherein
the groups A, -(B₁)ₒ-(B₂)ₚ-(B₃)_{q}-(B₄)ᵣ- and Z have been defined before.

Specific examples of the compounds represented by the formula (1) are given below. The compounds represented by the formula (1) are not limited to the following specific examples.

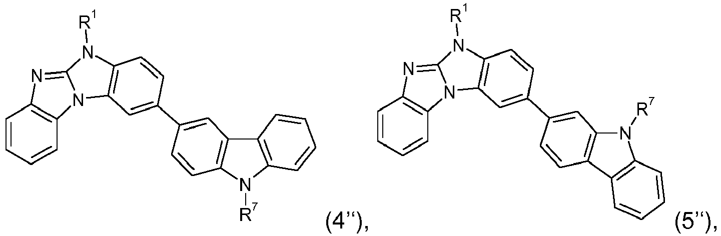

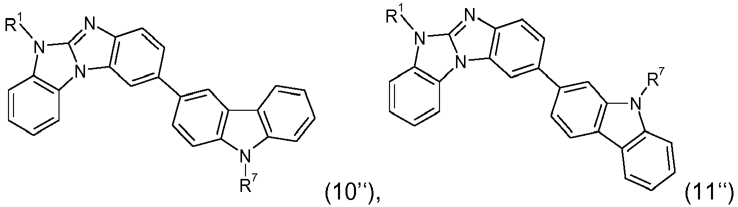

| Nr. | R¹ | R⁷ |
|---|---|---|
| 4"-1, 5"-1, 10"-1, 11"-1 | Ph | 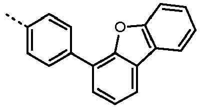 |
| 4"-2, 5"-2, 10"-2, 11"-2 | Ph | 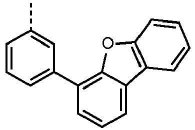 |
| 4"-3, 5"-3, 10"-3, 11 "-3 | Ph | 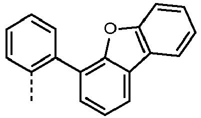 |
| 4"-4, 5"-4, 10"-4, 11 "-4 | Ph | 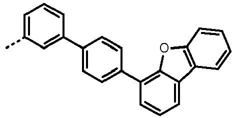 |
| 4"-5, 5"-5, 10"-5, 11 "-5 | Ph | 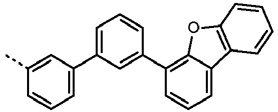 |
| 4"-6, 5"-6, 10"-6, 11"-6 | Ph | 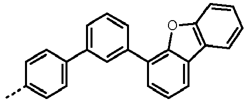 |
| 4"-7, 5"-7, 10"-7, 11 "-7 | Ph | 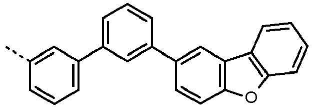 |
| 4"-8, 5"-8, 10"-8, 11"-8 | Ph | 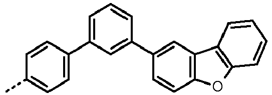 |
| 4"-9, 5"-9, 10"-9, 11"-9 | Ph | 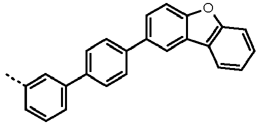 |
| 4"-10, 5"-10, 10"-10, 11"-10 | Ph | 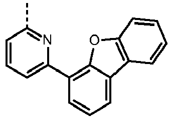 |
| 4"-11, 5"-11, 10"-11, 11"-11 | Ph | 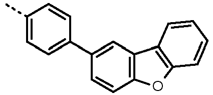 |
| 4"-12, 5"-12, 10"-12, 11"-12 | Ph | 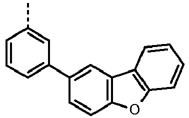 |
| 4"-13, 5"-13, 10"-13, 11"-13 | Ph | 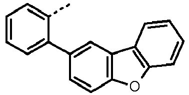 |
| 4"-14, 5"-14, 10"-14, 11"-14 | Ph | 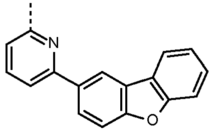 |
| 4"-15, 5"-15, 10"-15, 11"-15 | Ph | 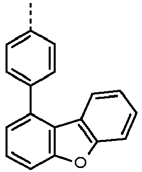 |
| 4"-16, 5"-16, 10"-16, 11"-16 | Ph | 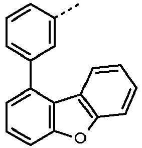 |
| 4"-17, 5"-17, 10"-17, 11"-17 | Ph | 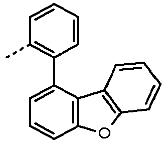 |
| 4"-18, 5"-18, 10"-18, 11"-18 | Ph | 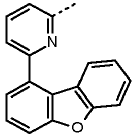 |
| 4"-19, 5"-19, 10"-19, 11"-19 | Ph | 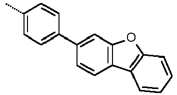 |
| 4"-20, 5"-20, 10"-20, 11"-20 | Ph | 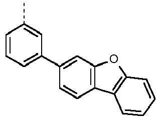 |
| 4"-21, 5"-21, 10"-21, 11"-21 | Ph | 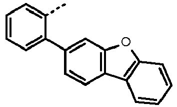 |
| 4"-22, 5"-22, 10"-22, 11"-22 | Ph | 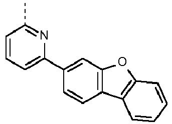 |
| 4"-23, 5"-23, 10"-23, 11"-23 | Ph | 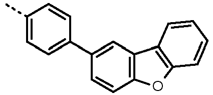 |
| 4"-24, 5"-24, 10"-24, 11"-24 | Ph | 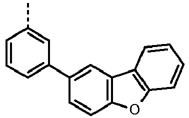 |
| 4"-25, 5"-25, 10"-25, 11"-25 | Ph | 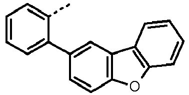 |
| 4"-26, 5"-26, 10"-26, 11"-26 | Ph | 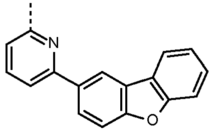 |
| 4"-27, 5"-27, 10"-27, 11"-27 | 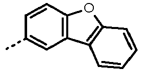 | 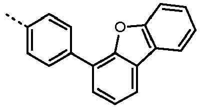 |
| 4"-28, 5"-28, 10"-28, 11"-28 | 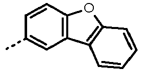 | 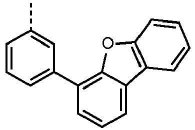 |
| 4"-29, 5"-29, 10"-29, 11"-29 | 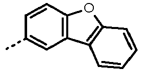 | 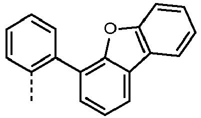 |
| 4"-30, 5"-30, 10"-30, 11"-30 | 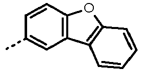 | 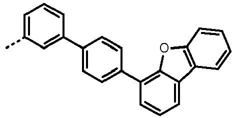 |
| 4"-31, 5"-31, 10"-31, 11"-31 | 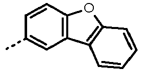 | 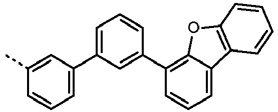 |
| 4"-32, 5"-32, 10"-32, 11"-32 | 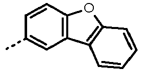 | 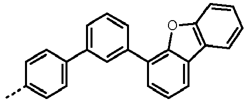 |
| 4"-33, 5"-33, 10"-33, 11"-33 | 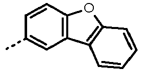 | 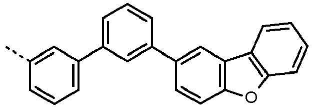 |
| 4"-34, 5"-34, 10"-34, 11"-34 | 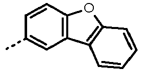 | 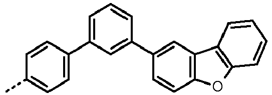 |
| 4"-35, 5"-35, 10"-35, 11"-35 | 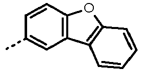 | 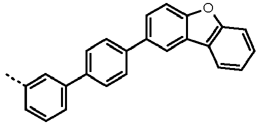 |
| 4"-36, 5"-36, 10"-36, 11"-36 | 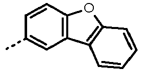 | 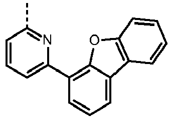 |
| 4"-37, 5"-37, 10"-37, 11"-37 | 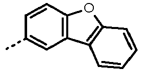 | 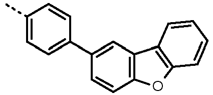 |
| 4"-38, 5"-38, 10"-38, 11"-38 | 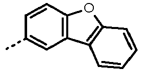 | 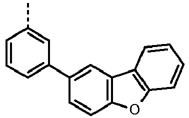 |
| 4"-39, 5"-39, 10"-39, 11"-39 | 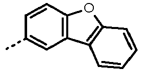 | 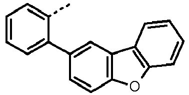 |
| 4"-40, 5"-40, 10"-40, 11"-40 | 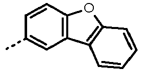 | 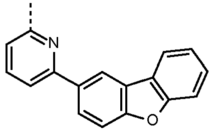 |
| 4"-41, 5"-41, 10"-41, 11"-41 | 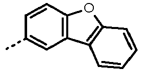 | 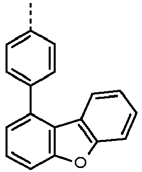 |
| 4"-42, 5"-42, 10"-42, 11"-42 | 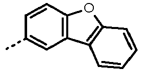 | 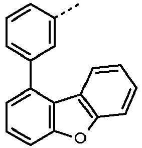 |
| 4"-43, 5"-43, 10"-43, 11"-43 | 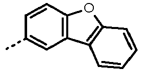 | 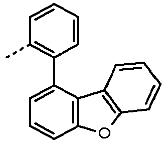 |
| 4"-44, 5"-44, 10"-44, 11"-44 | 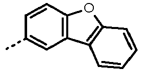 | 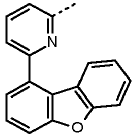 |
| 4"-45, 5"-45, 10"-45, 11"-45 | 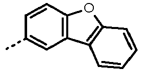 | 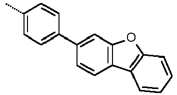 |
| 4"-46, 5"-46, 10"-46, 11"-46 | 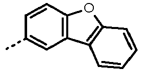 | 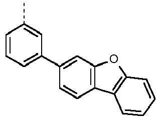 |
| 4"-47, 5"-47, 10"-47, 11"-47 | 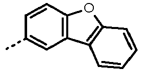 | 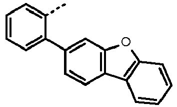 |
| 4"-48, 5"-48, 10"-48, 11"-48 | 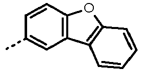 | 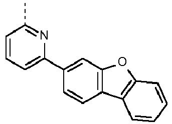 |
| 4"-49, 5"-49, 10"-49, 11"-49 | 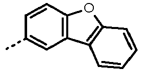 | 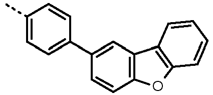 |
| 4"-50, 5"-50, 10"-50, 11"-50 | 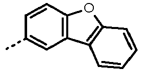 | 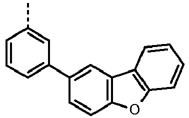 |
| 4"-51, 5"-51, 10"-51, 11"-51 | 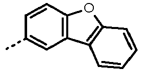 | 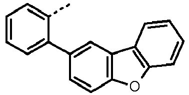 |
| 4"-52, 5"-52, 10"-52, 11"-52 | 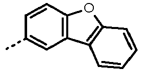 | 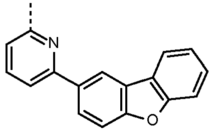 |
| 4"-53, 5"-53, 10"-53, 11"-53 | 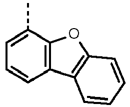 | 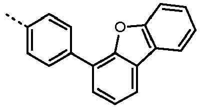 |
| 4"-54, 5"-54, 10"-54, 11"-54 | 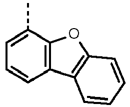 | 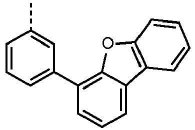 |
| 4"-55, 5"-55, 10"-55, 11"-55 | 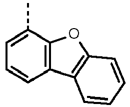 | 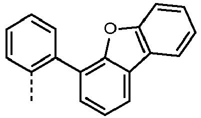 |
| 4"-56, 5"-56, 10"-56, 11"-56 | 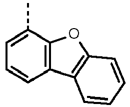 | 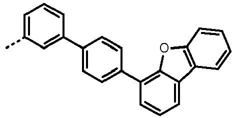 |
| 4"-57, 5"-57, 10"-57, 11"-57 | 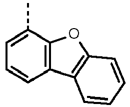 | 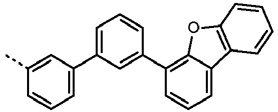 |
| 4"-58, 5"-58, 10"-58, 11"-58 | 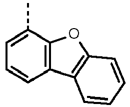 | 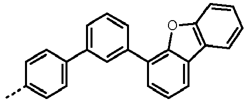 |
| 4"-59, 5"-59, 10"-59, 11"-59 | 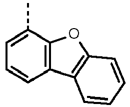 | 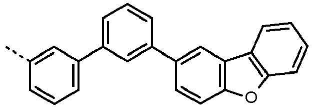 |
| 4"-60, 5"-60, 10"-60, 11"-60 | 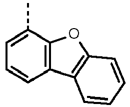 | 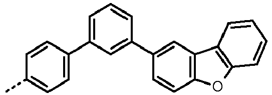 |
| 4"-61, 5"-61, 10"-61, 11"-61 | 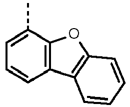 | 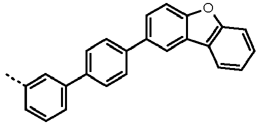 |
| 4"-62, 5"-62, 10"-62, 11"-62 | 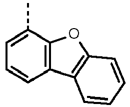 | 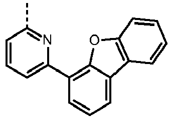 |
| 4"-63, 5"-63, 10"-63, 11"-63 | 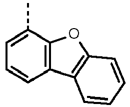 | 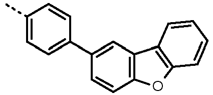 |
| 4"-64, 5"-64, 10"-64, 11"-64 | 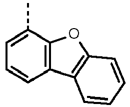 | 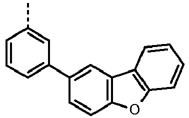 |
| 4"-65, 5"-65, 10"-65, 11"-65 | 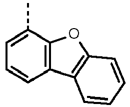 | 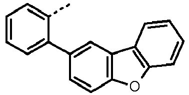 |
| 4"-66, 5"-66, 10"-66, 11"-66 | 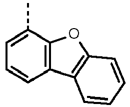 | 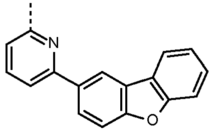 |
| 4"-67, 5"-67, 10"-67, 11"-67 | 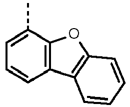 | 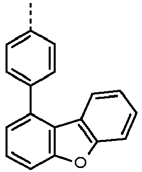 |
| 4"-68, 5"-68, 10"-68, 11"-68 | 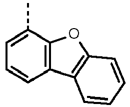 | 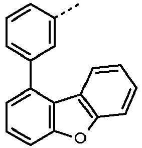 |
| 4"-69, 5"-69, 10"-69, 11"-69 | 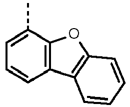 | 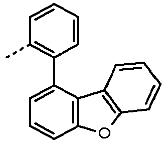 |
| 4"-70, 5"-70, 10"-70, 11"-70 | 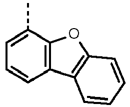 | 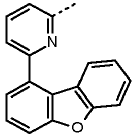 |
| 4"-71, 5"-71, 10"-71, 11"-71 | 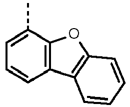 | 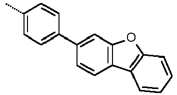 |
| 4"-72, 5"-72, 10"-72, 11"-72 | 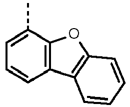 | 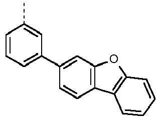 |
| 4"-73, 5"-73, 10"-73, 11"-73 | 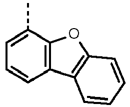 | 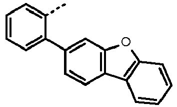 |
| 4"-74, 5"-74, 10"-74, 11"-74 | 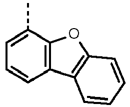 | 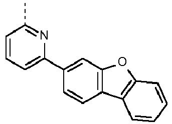 |
| 4"-75, 5"-75, 10"-75, 11"-75 | 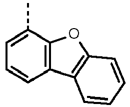 | 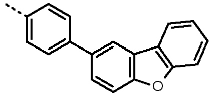 |
| 4"-76, 5"-76, 10"-76, 11"-76 | 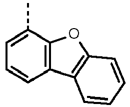 | 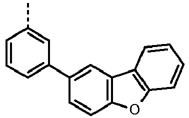 |
| 4"-77, 5"-77, 10"-77, 11"-77 | 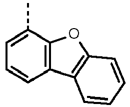 | 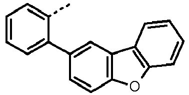 |
| 4"-78, 5"-78, 10"-78, 11"-78 | 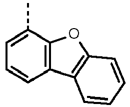 | 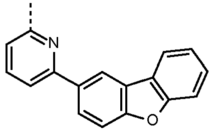 |
| 4"-79, 5"-79, 10"-79, 11"-79 | 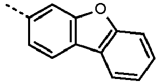 | 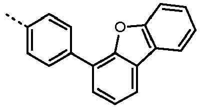 |
| 4"-80, 5"-80, 10"-80, 11"-80 | 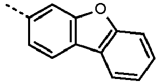 | 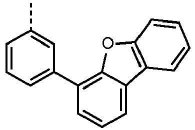 |
| 4"-81, 5"-81, 10"-81, 11"-81 | 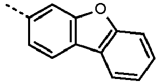 | 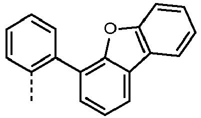 |
| 4"-82, 5"-82, 10"-82, 11"-82 | 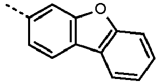 | 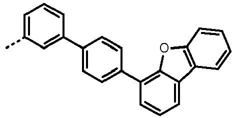 |
| 4"-83, 5"-83, 10"-83, 11"-83 | 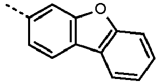 | 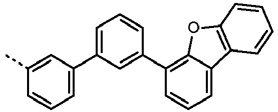 |
| 4"-84, 5"-84, 10"-84, 11"-84 | 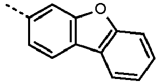 | 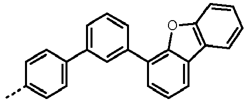 |
| 4"-85, 5"-85, 10"-85, 11"-85 | 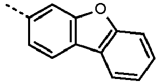 | 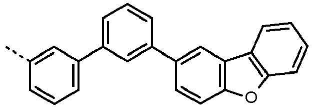 |
| 4"-86, 5"-86, 10"-86, 11"-86 | 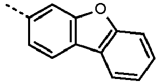 | 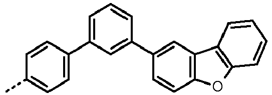 |
| 4"-87, 5"-87, 10"-87, 11"-87 | 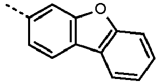 | 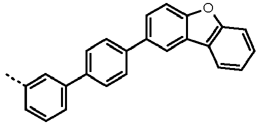 |
| 4"-88, 5"-88, 10"-88, 11"-88 | 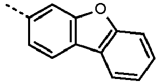 | 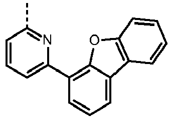 |
| 4"-89, 5"-89, 10"-89, 11"-89 | 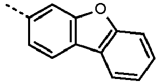 | 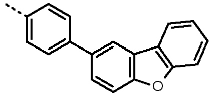 |
| 4"-90, 5"-90, 10"-90, 11"-90 | 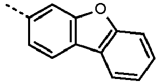 | 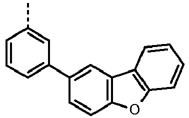 |
| 4"-91, 5"-91, 10"-91, 11"-91 | 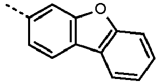 | 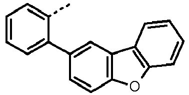 |
| 4"-92, 5"-92, 10"-92, 11"-92 | 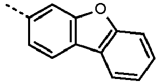 | 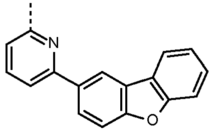 |
| 4"-93, 5"-93, 10"-93, 11"-93 | 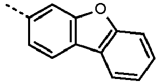 | 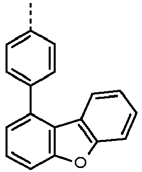 |
| 4"-94, 5"-94, 10"-94, 11"-94 | 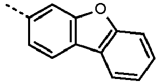 | 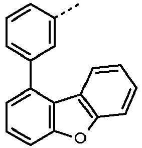 |
| 4"-95, 5"-95, 10"-95, 11"-95 | 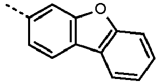 | 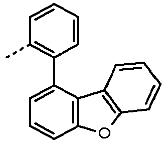 |
| 4"-96, 5"-96, 10"-96, 11"-96 | 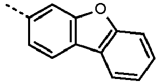 | 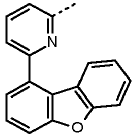 |
| 4"-97, 5"-97, 10"-97, 11"-97 | 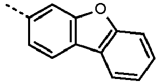 | 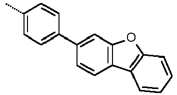 |
| 4"-98, 5"-98, 10"-98, 11"-98 | 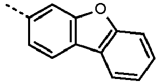 | 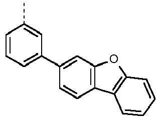 |
| 4"-99, 5"-99, 10"-99, 11"-99 | 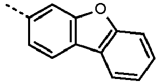 | 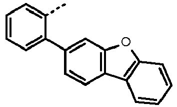 |
| 4"-100, 5"-100, 10"-100, 11"-100 | 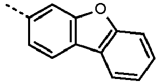 | 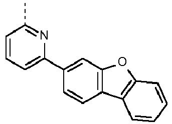 |
| 4"-101, 5"-101, 10"-101, 11"-101 | 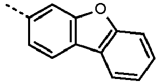 | 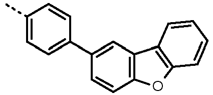 |
| 4"-102, 5"-102, 10"-102, 11"-102 | 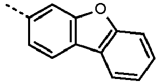 | 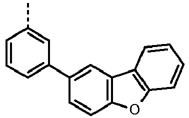 |
| 4"-103, 5"-103, 10"-103, 11"-103 | 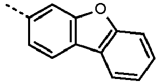 | 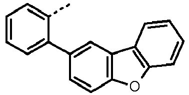 |
| 4"-104, 5"-104, 10"-104, 11"-104 | 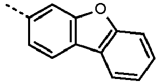 | 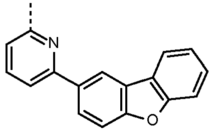 |
| 4"-105, 5"-105, 10"-105, 11"-105 | 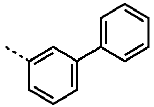 | 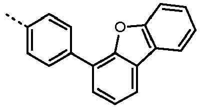 |
| 4"-106, 5"-106, 10"-106, 11"-106 | 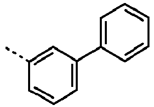 | 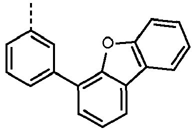 |
| 4"-107, 5"-107, 10"-107, 11"-107 | 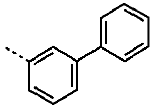 | 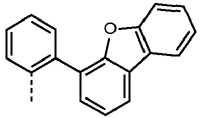 |
| 4"-108, 5"-108, 10"-108, 11"-108 | 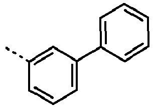 | 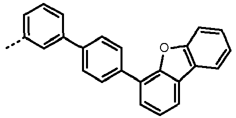 |
| 4"-109, 5"-109, 10"-109, 11"-109 | 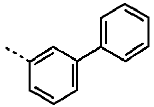 | 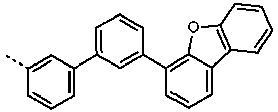 |
| 4"-110, 5"-110, 10"-110, 11"-110 | 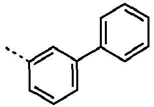 | 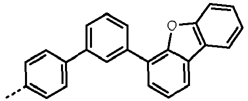 |
| 4"-111, 5"-111, 10"-111, 11"-111 | 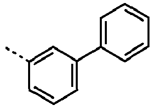 | 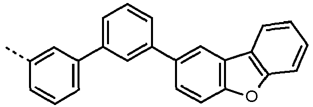 |
| 4"-112, 5"-112, 10"-112, 11"-112 | 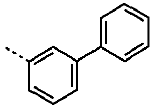 | 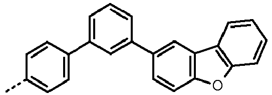 |
| 4"-113, 5"-113, 10"-113, 11"-113 | 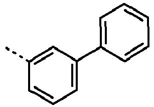 | 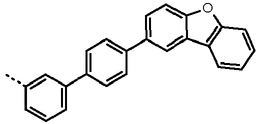 |
| 4"-114, 5"-114, 10"-114, 11"-114 | 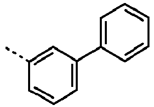 | 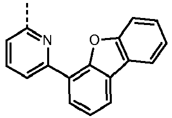 |
| 4"-115, 5"-115, 10"-115, 11"-115 | 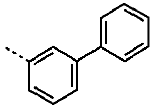 | 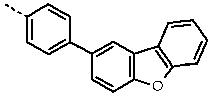 |
| 4"-116, 5"-116, 10"-116, 11"-116 | 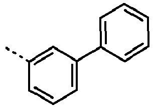 | 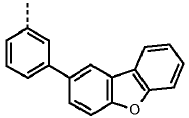 |
| 4"-117, 5"-117, 10"-117, 11"-117 | 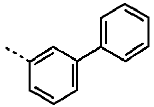 | 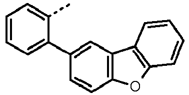 |
| 4"-118, 5"-118, 10"-118, 11"-118 | 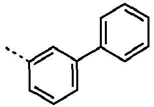 | 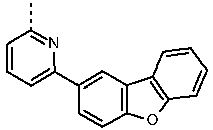 |
| 4"-119, 5"-119, 10"-119, 11"-119 | 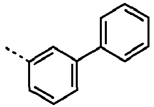 | 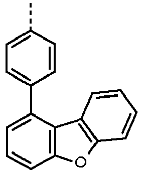 |
| 4"-120, 5"-120 10"-120, 11"-120 | 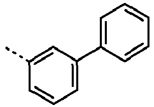 | 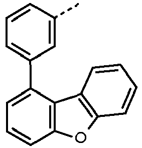 |
| 4"-121, 5"-121, 10"-121, 11"-121 | 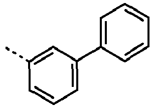 | 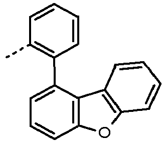 |
| 4"-122, 5"-122, 10"-122, 11"-122 | 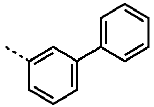 | 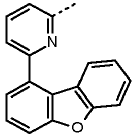 |
| 4"-123, 5"-123, 10"-123, 11"-123 | 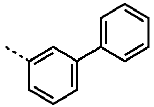 | 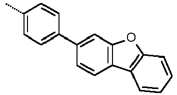 |
| 4"-124, 5"-124, 10"-124, 11"-124 | 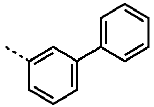 | 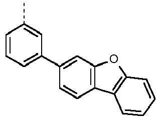 |
| 4"-125, 5"-125, 10"-125, 11"-125 | 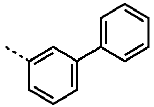 | 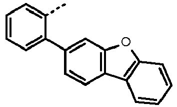 |
| 4"-126, 5"-126, 10"-126, 11"-126 | 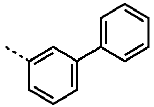 | 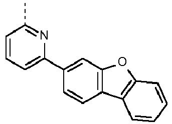 |
| 4"-127, 5"-127, 10"-127, 11"-127 | 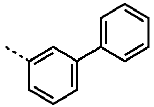 | 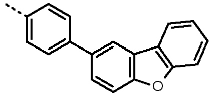 |
| 4"-128, 5"-128, 10"-128, 11"-128 | 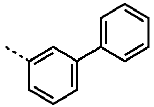 | 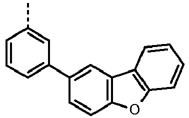 |
| 4"-129, 5"-129, 10"-129, 11"-129 | 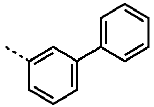 | 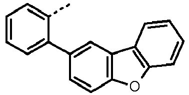 |
| 4"-130, 5"-130, 10"-130, 11"-130 | 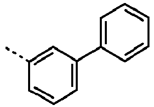 | 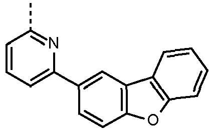 |
| 4"-131, 5"-131, 10"-131, 11"-131 | 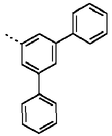 | 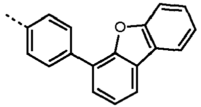 |
| 4"-132, 5"-132, 10"-132, 11"-132 | 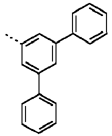 | 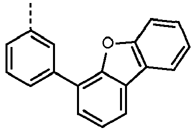 |
| 4"-133, 5"-133, 10"-133, 11"-133 | 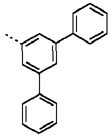 | 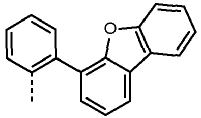 |
| 4"-134, 5"-134, 10"-134, 11"-134 | 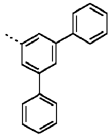 | 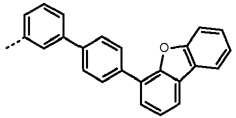 |
| 4"-135, 5"-135, 10"-135, 11"-135 | 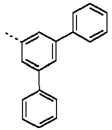 | 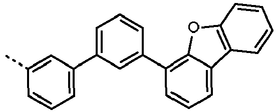 |
| 4"-136, 5"-136, 10"-136, 11"-136 | 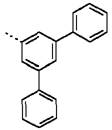 | 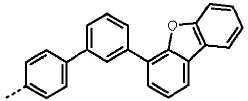 |
| 4"-137, 5"-137, 10"-137, 11"-137 | 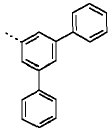 | 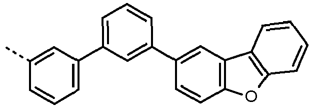 |
| 4"-138, 5"-138, 10"-138, 11"-138 | 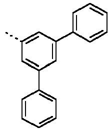 | 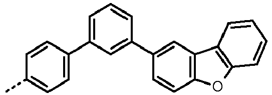 |
| 4"-139, 5"-139, 10"-139, 11"-139 | 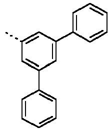 | 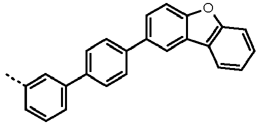 |
| 4"-140, 5"-140, 10"-140, 11"-140 | 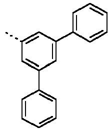 | 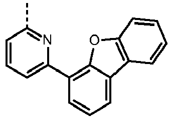 |
| 4"-141, 5"-141, 10"-141, 11"-141 | 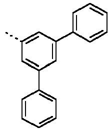 | 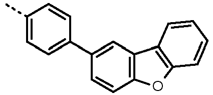 |
| 4"-142, 5"-142, 10"-142, 11"-142 | 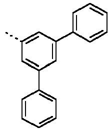 | 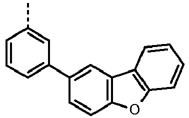 |
| 4"-143, 5"-143, 10"-143, 11"-143 | 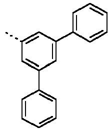 | 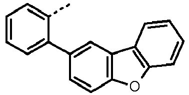 |
| 4"-144, 5"-144, 10"-144, 11"-144 | 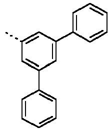 | 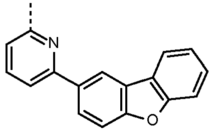 |
| 4"-145, 5"-145, 10"-145, 11"-145 | 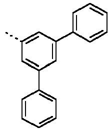 | 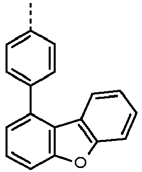 |
| 4"-146, 5"-146, 10"-146, 11"-146 | 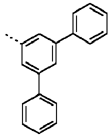 | 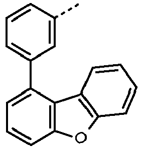 |
| 4"-147, 5"-147, 10"-147, 11"-147 | 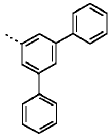 | 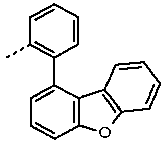 |
| 4"-148, 5"-148, 10"-148, 11"-148 | 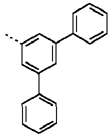 | 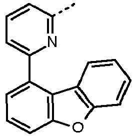 |
| 4"-149, 5"-149, 10"-149, 11"-149 | 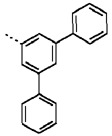 | 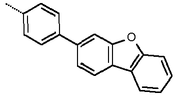 |
| 4"-150, 5"-150, 10"-150, 11"-150 | 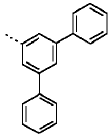 | 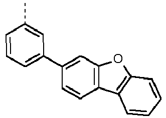 |
| 4"-151, 5"-151, 10"-151, 11"-151 | 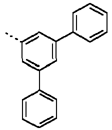 | 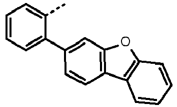 |
| 4"-152, 5"-152, 10"-152, 11"-152 | 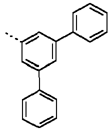 | 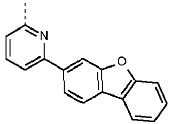 |
| 4"-153, 5"-153, 10"-153, 11"-153 | 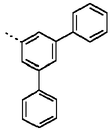 | 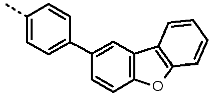 |
| 4"-154, 5"-154, 10"-154, 11"-154 | 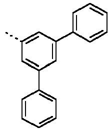 | 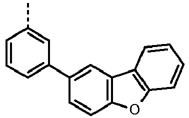 |
| 4"-155, 5"-155, 10"-155, 11"-155 | 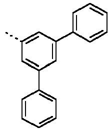 | 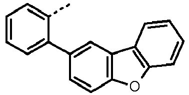 |
| 4"-156, 5"-156, 10"-156, 11"-156 | 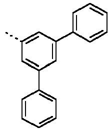 | 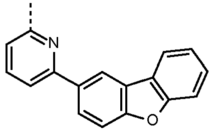 |

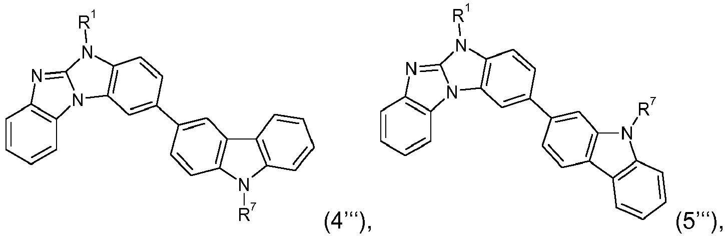

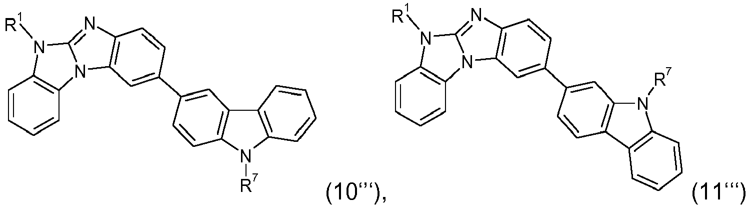

| Nr. | R⁷ | R¹ |
|---|---|---|
| 4"'-1, 5"'-1, 10"'-1, 11"'-1 | Ph | 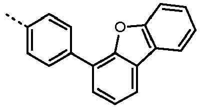 |
| 4"'-2, 5"'-2, 10"'-2, 11"'-2 | Ph | 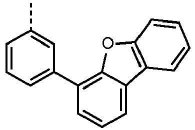 |
| 4"'-3, 5"'-3, 10"'-3, 11"'-3 | Ph | 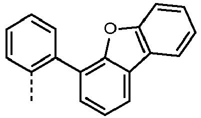 |
| 4"'-4, 5"'-4, 10"'-4, 11"'-4 | Ph | 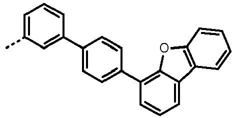 |
| 4"'-5, 5"'-5, 10"'-5, 11"'-5 | Ph | 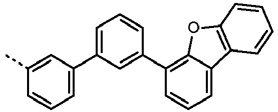 |
| 4"'-6, 5"'-6, 10"'-6, 11"'-6 | Ph | 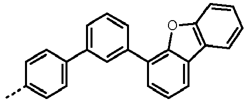 |
| 4"'-7, 5"'-7, 10"'-7, 11"'-7 | Ph | 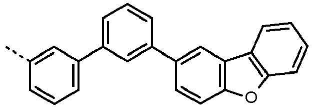 |
| 4"'-8, 5"'-8, 10"'-8, 11"'-8 | Ph | 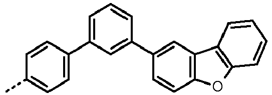 |
| 4"'-9, 5"'-9, 10"'-9, 11"'-9 | Ph | 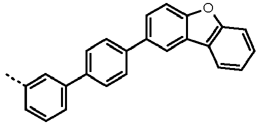 |
| 4"'-10, 5"'-10, 10"'-10, 11"'-10 | Ph | 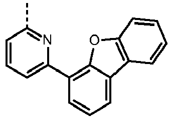 |
| 4"'-11, 5"'-11, 10"'-11, 11"'-11 | Ph | 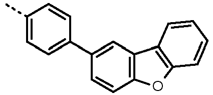 |
| 4"'-12, 5"'-12, 10"'-12, 11"'-12 | Ph | 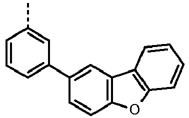 |
| 4"'-13, 5"'-13, 10"'-13, 11"'-13 | Ph | 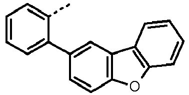 |
| 4"'-14, 5"'-14, 10"'-14, 11"'-14 | Ph | 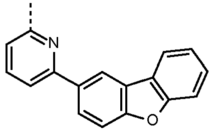 |
| 4"'-15, 5"'-15, 10"'-15, 11"'-15 | Ph | 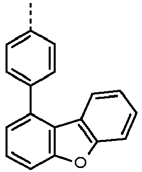 |
| 4"'-16, 5"'-16, 10"'-16, 11"'-16 | Ph | 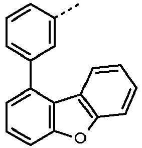 |
| 4"'-17, 5"'-17, 10"'-17, 11"'-17 | Ph | 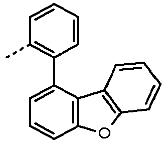 |
| 4"'-18, 5"'-18, 10"'-18, 11"'-18 | Ph | 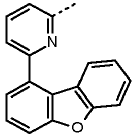 |
| 4"'-19, 5"'-19, 10"'-19, 11"'-19 | Ph | 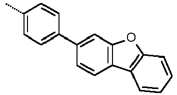 |
| 4"'-20, 5"'-20, 10"'-20, 11"'-20 | Ph | 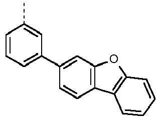 |
| 4"'-21, 5"'-21, 10"'-21, 11"'-21 | Ph | 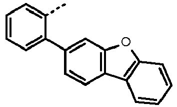 |
| 4"'-22, 5"'-22, 10"'-22, 11"'-22 | Ph | 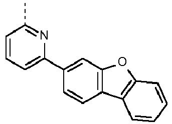 |
| 4"'-23, 5"'-23, 10"'-23, 11"'-23 | Ph | 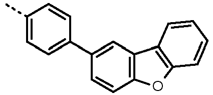 |
| 4"'-24, 5"'-24, 10"'-24, 11"'-24 | Ph | 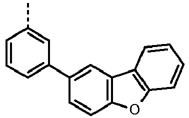 |
| 4"'-25, 5"'-25, 10"'-25, 11"'-25 | Ph | 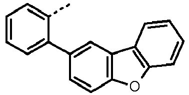 |
| 4"'-26, 5"'-26, 10"'-26, 11"'-26 | Ph | 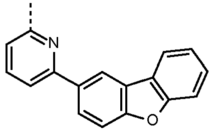 |
| 4"'-27, 5"'-27, 10"'-27, 11"'-27 | 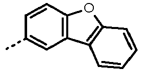 | 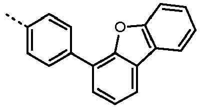 |
| 4"'-28, 5"'-28, 10"'-28, 11"'-28 | 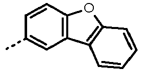 | 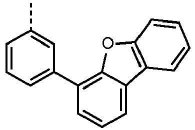 |
| 4"'-29, 5"'-29, 10"'-29, 11"'-29 | 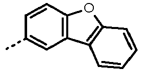 | 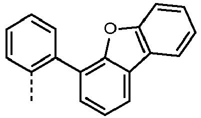 |
| 4"'-30, 5"'-30, 10"'-30, 11"'-30 | 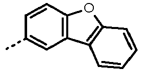 | 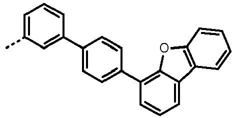 |
| 4"'-31, 5"'-31, 10"'-31, 11"'-31 | 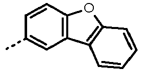 | 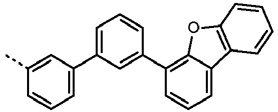 |
| 4"'-32, 5"'-32, 10"'-32, 11"'-32 | 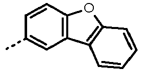 | 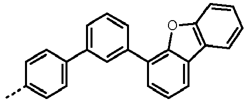 |
| 4"'-33, 5"'-33, 10"'-33, 11"'-33 | 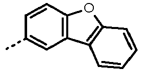 | 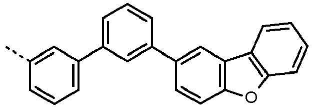 |
| 4"'-34, 5"'-34, 10"'-34, 11"'-34 | 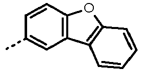 | 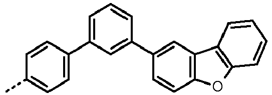 |
| 4"'-35, 5"'-35, 10"'-35, 11"'-35 | 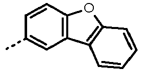 | 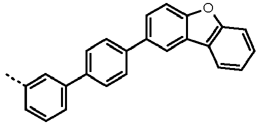 |
| 4"'-36, 5"'-36, 10"'-36, 11"'-36 | 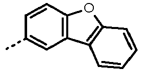 | 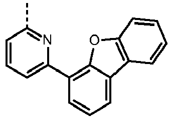 |
| 4"'-37, 5"'-37, 10"'-37, 11"'-37 | 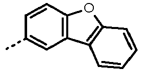 | 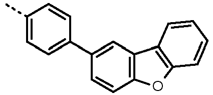 |
| 4"'-38, 5"'-38, 10"'-38, 11"'-38 | 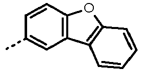 | 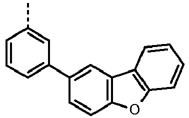 |
| 4"'-39, 5"'-39, 10"'-39, 11"'-39 | 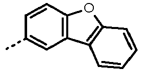 | 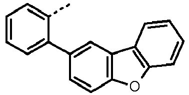 |
| 4"'-40, 5"'-40, 10"'-40, 11"'-40 | 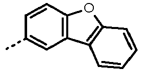 | 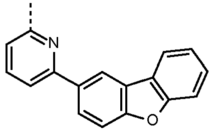 |
| 4"'-41, 5"'-41, 10"'-41, 11"'-41 | 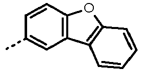 | 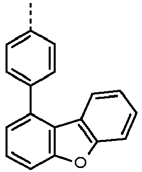 |
| 4"'-42, 5"'-42, 10"'-42, 11"'-42 | 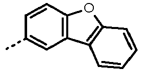 | 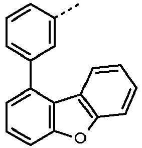 |
| 4"'-43, 5"'-43, 10"'-43, 11"'-43 | 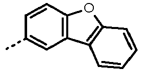 | 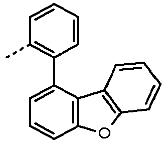 |
| 4"'-44, 5"'-44, 10"'-44, 11"'-44 | 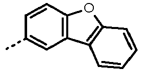 | 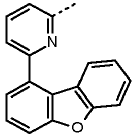 |
| 4"'-45, 5"'-45, 10"'-45, 11"'-45 | 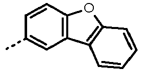 | 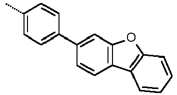 |
| 4"'-46, 5"'-46, 10"'-46, 11"'-46 | 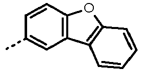 | 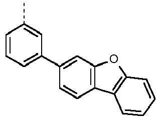 |
| 4"'-47, 5"'-47, 10"'-47, 11"'-47 | 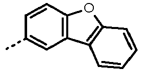 | 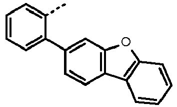 |
| 4"'-48, 5"'-48, 10"'-48, 11"'-48 | 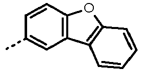 | 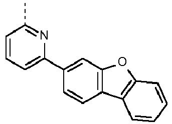 |
| 4"'-49, 5"'-49, 10"'-49, 11"'-49 | 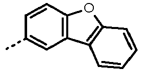 | 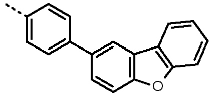 |
| 4"'-50, 5"'-50, 10"'-50, 11"'-50 | 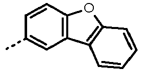 | 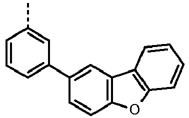 |
| 4"'-51, 5"'-51, 10"'-51, 11"'-51 | 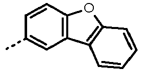 | 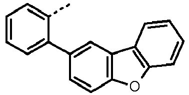 |
| 4"'-52, 5"'-52, 10"'-52, 11"'-52 | 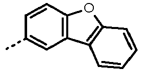 | 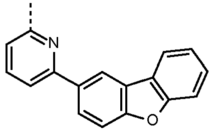 |
| 4"'-53, 5"'-53, 10"'-53, 11"'-53 | 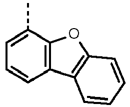 | 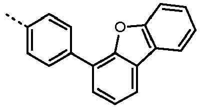 |
| 4"'-54, 5"'-54, 10"'-54, 11"'-54 | 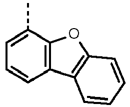 | 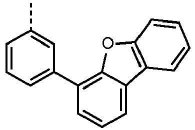 |
| 4"'-55, 5"'-55, 10"'-55, 11"'-55 | 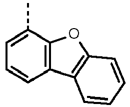 | 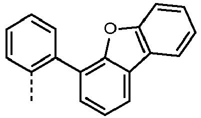 |
| 4"'-56, 5"'-56, 10"'-56, 11"'-56 | 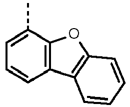 | 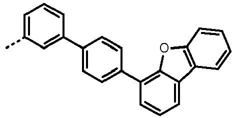 |
| 4"'-57, 5"'-57, 10"'-57, 11"'-57 | 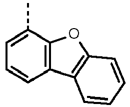 | 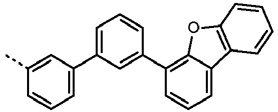 |
| 4"'-58, 5"'-58, 10"'-58, 11"'-58 | 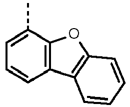 | 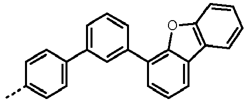 |
| 4"'-59, 5"'-59, 10"'-59, 11"'-59 | 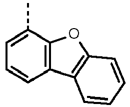 | 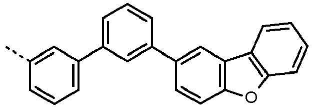 |
| 4"'-60, 5"'-60, 10"'-60, 11"'-60 | 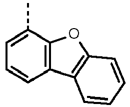 | 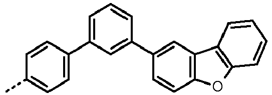 |
| 4"'-61, 5"'-61, 10"'-61, 11"'-61 | 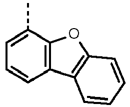 | 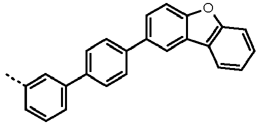 |
| 4"'-62, 5"'-62, 10"'-62, 11"'-62 | 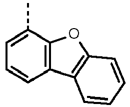 | 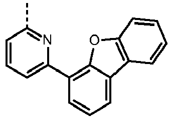 |
| 4"'-63, 5"'-63, 10"'-63, 11"'-63 | 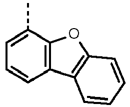 | 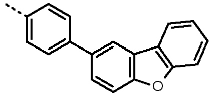 |
| 4"'-64, 5"'-64, 10"'-64, 11"'-64 | 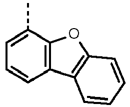 | 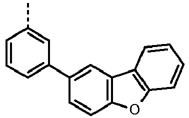 |
| 4"'-65, 5"'-65, 10"'-65, 11"'-65 | 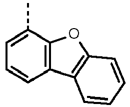 | 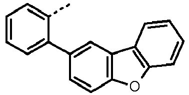 |
| 4"'-66, 5"'-66, 10"'-66, 11"'-66 | 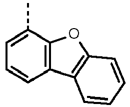 | 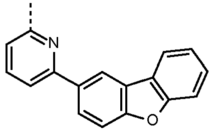 |
| 4"'-67, 5"'-67, 10"'-67, 11"'-67 | 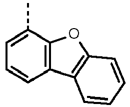 | 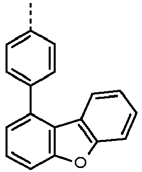 |
| 4"'-68, 5"'-68, 10"'-68, 11"'-68 | 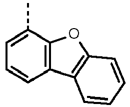 | 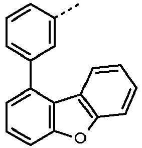 |
| 4"'-69, 5"'-69, 10"'-69, 11"'-69 | 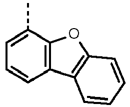 | 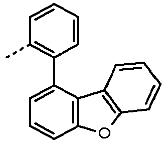 |
| 4"'-70, 5"'-70, 10"'-70, 11"'-70 | 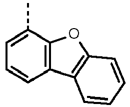 | 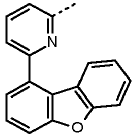 |
| 4"'-71, 5"'-71, 10"'-71, 11"'-71 | 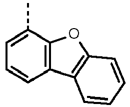 | 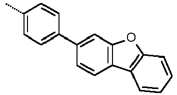 |
| 4"'-72, 5"'-72, 10"'-72, 11"'-72 | 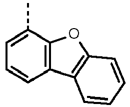 | 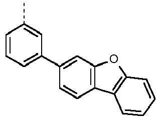 |
| 4"'-73, 5"'-73, 10"'-73, 11'"-73 | 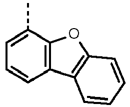 | 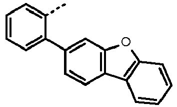 |
| 4"'-74, 5"'-74, 10"'-74, 11"'-74 | 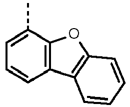 | 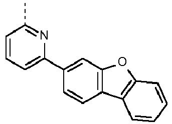 |
| 4"'-75, 5"'-75, 10"'-75, 11"'-75 | 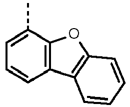 | 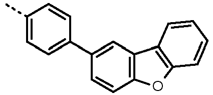 |
| 4"'-76, 5"'-76, 10"'-76, 11"'-76 | 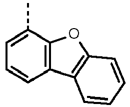 | 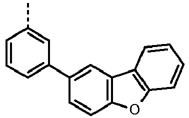 |
| 4"'-77, 5"'-77, 10"'-77, 11"'-77 | 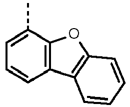 | 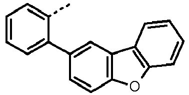 |
| 4"'-78, 5"'-78, 10"'-78, 11"'-78 | 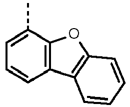 | 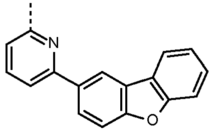 |
| 4"'-79, 5"'-79, 10"'-79, 11'"-79 | 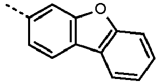 | 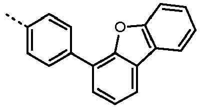 |
| 4"'-80, 5"'-80, 10"'-80, 11"'-80 | 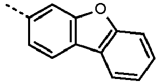 | 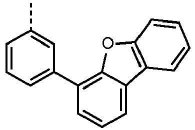 |
| 4"'-81, 5"'-81, 10"'-81, 11"'-81 | 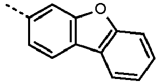 | 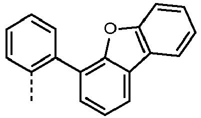 |
| 4"'-82, 5"'-82, 10"'-82, 11"'-82 | 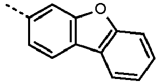 | 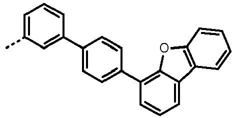 |
| 4"'-83, 5"'-83, 10"'-83, 11"'-83 | 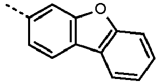 | 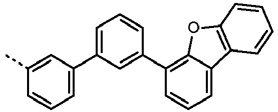 |
| 4"'-84, 5"'-84, 10"'-84, 11"'-84 | 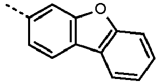 | 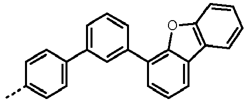 |
| 4"'-85, 5"'-85, 10"'-85, 11"'-85 | 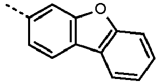 | 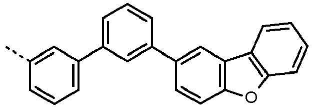 |
| 4"'-86, 5"'-86, 10"'-86, 11"'-86 | 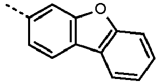 | 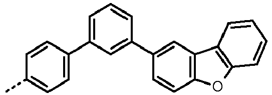 |
| 4"'-87, 5"'-87, 10"'-87, 11"'-87 | 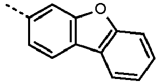 | 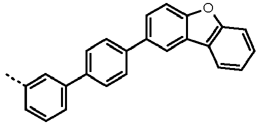 |
| 4"'-88, 5"'-88, 10"'-88, 11"'-88 | 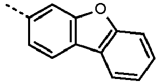 | 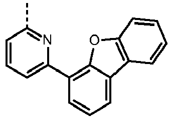 |
| 4"'-89, 5"'-89, 10"'-89, 11"'-89 | 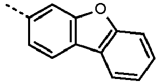 | 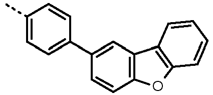 |
| 4"'-90, 5"'-90, 10"'-90, 11"'-90 | 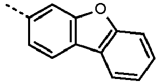 | 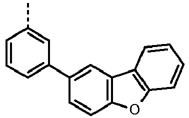 |
| 4"'-91, 5"'-91, 10"'-91, 11"'-91 | 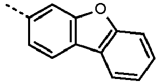 | 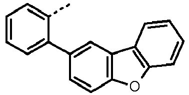 |
| 4"'-92, 5"'-92, 10"'-92, 11"'-92 | 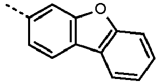 | 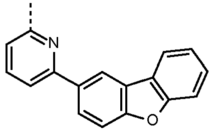 |
| 4"'-93, 5"'-93, 10"'-93, 11"'-93 | 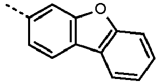 | 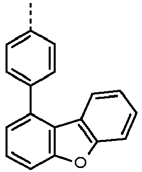 |
| 4"'-94, 5"'-94, 10"'-94, 11"'-94 | 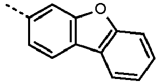 | 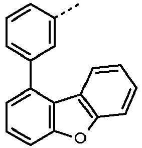 |
| 4"'-95, 5"'-95, 10"'-95, 11"'-95 | 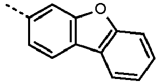 | 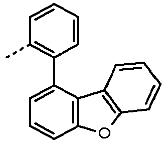 |
| 4"'-96, 5"'-96, 10"'-96, 11"'-96 | 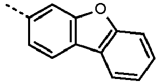 | 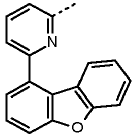 |
| 4"'-97, 5"'-97, 10"'-97, 11"'-97 | 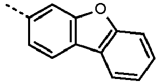 | 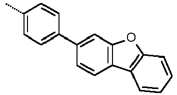 |
| 4"'-98, 5"'-98, 10"'-98, 11"'-98 | 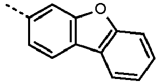 | 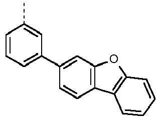 |
| 4"'-99, 5"'-99, 10"'-99, 11"'-99 | 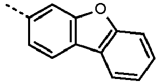 | 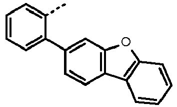 |
| 4"'-100, 5"'-100, 10"'-100, 11"'-100 | 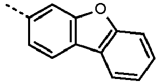 | 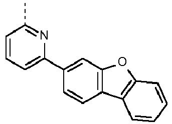 |
| 4"'-101, 5"'-101, 10"'-101, 11"'-101 | 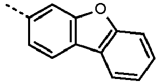 | 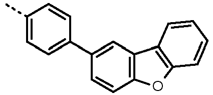 |
| 4"'-102, 5"'-102, 10"'-102, 11'"-102 | 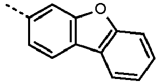 | 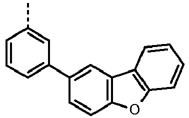 |
| 4"'-103, 5"'-103, 10"'-103, 11'"-103 | 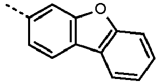 | 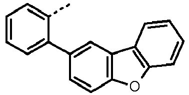 |
| 4"'-104, 5"'-104, 10"'-104, 11'"-104 | 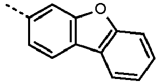 | 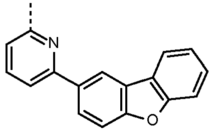 |
| 4"'-105, 5"'-105, 10"'-105, 11'"-105 | 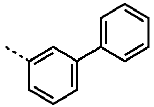 | 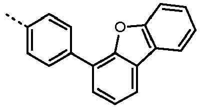 |
| 4"'-106, 5"'-106, 10"'-106, 11'"-106 | 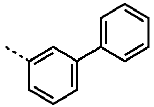 | 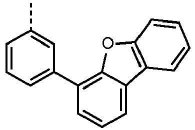 |
| 4"'-107, 5"'-107, 10"'-107, 11'"-107 | 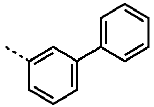 | 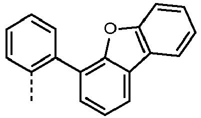 |
| 4"'-108, 5"'-108, 10"'-108, 11"'-108 | 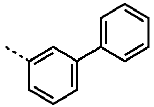 | 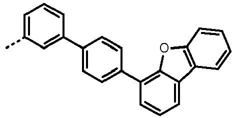 |
| 4"'-109, 5"'-109, 10"'-109, 11'"-109 | 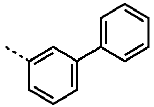 | 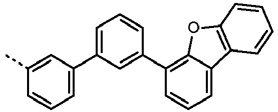 |
| 4"'-110, 5"'-110, 10"'-110, 11"'-110 | 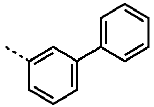 | 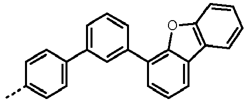 |
| 4"'-111, 5"'-111, 10"'-111, 11"'-111 | 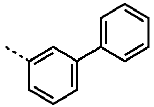 | 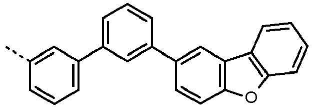 |
| 4"'-112, 5"'-112, 10"'-112, 11"'-112 | 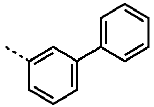 | 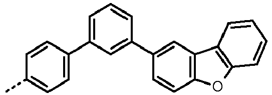 |
| 4"'-113, 5"'-113, 10"'-113, 11"'-113 | 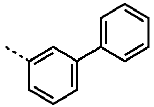 | 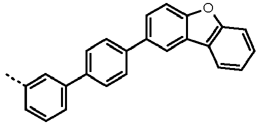 |
| 4"'-114, 5"'-114, 10"'-114, 11"'-114 | 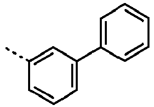 | 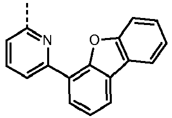 |
| 4"'-115, 5"'-115, 10"'-115, 11"'-115 | 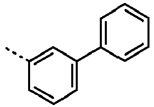 | 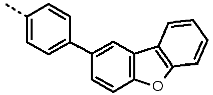 |
| 4"'-116, 5"'-116, 10"'-116, 11"'-116 | 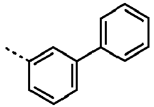 | 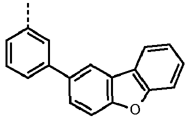 |
| 4"'-117, 5"'-117, 10"'-117, 11'"-117 | 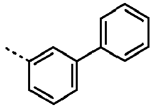 | 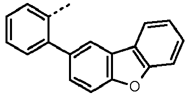 |
| 4"'-118, 5"'-118, 10"'-118, 11'"-118 | 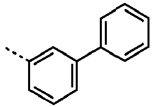 | 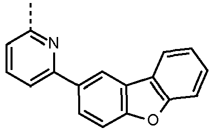 |
| 4"'-119, 5"'-119, 10"'-119, 11'"-119 | 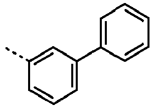 | 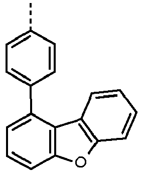 |
| 4"'-120, 5"'-120 10"'-120, 11'"-120 | 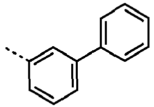 | 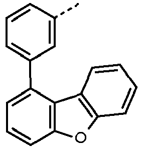 |
| 4"'-121, 5"'-121, 10"'-121, 11"'-121 | 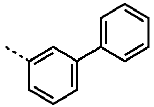 | 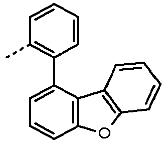 |
| 4"'-122, 5"'-122, 10"'-122, 11'"-122 | 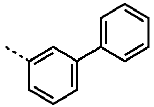 | 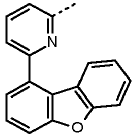 |
| 4"'-123, 5"'-123, 10"'-123, 11"'-123 | 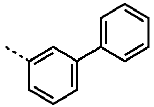 | 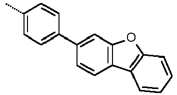 |
| 4"'-124, 5"'-124, 10"'-124, 11'"-124 | 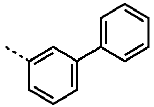 | 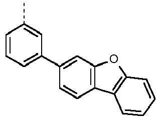 |
| 4"'-125, 5"'-125, 10"'-125, 11"'-125 | 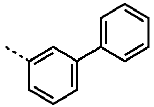 | 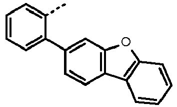 |
| 4"'-126, 5"'-126, 10"'-126, 11"'-126 | 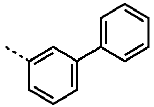 | 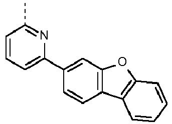 |
| 4"'-127, 5"'-127, 10"'-127, 11"'-127 | 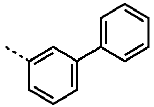 | 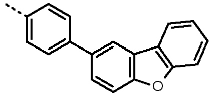 |
| 4"'-128, 5"'-128, 10"'-128, 11"'-128 | 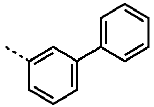 | 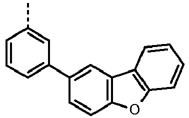 |
| 4"'-129, 5"'-129, 10"'-129, 11'"-129 | 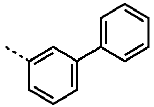 | 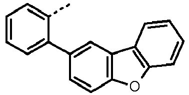 |
| 4"'-130, 5"'-130, 10"'-130, 11'"-130 | 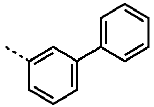 | 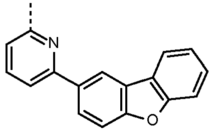 |
| 4"'-131, 5"'-131, 10"'-131, 11'"-131 | 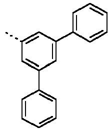 | 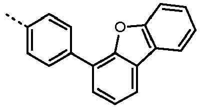 |
| 4"'-132, 5"'-132, 10"'-132, 11"'-132 | 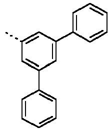 | 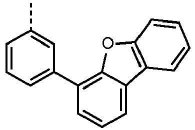 |
| 4"'-133, 5"'-133, 10"'-133, 11'"-133 | 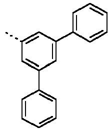 | 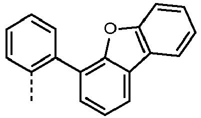 |
| 4"'-134, 5"'-134, 10"'-134, 11'"-134 | 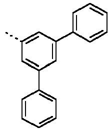 | 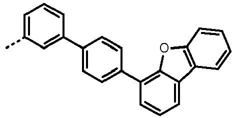 |
| 4"'-135, 5"'-135, 10"'-135, 11'"-135 | 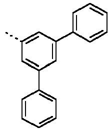 | 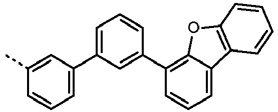 |
| 4"'-136, 5"'-136, 10"'-136, 11'"-136 | 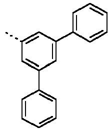 | 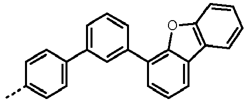 |
| 4"'-137, 5"'-137, 10"'-137, 11'"-137 | 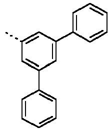 | 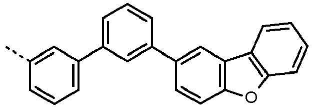 |
| 4"'-138, 5"'-138, 10"'-138, 11'"-138 | 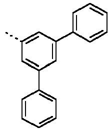 | 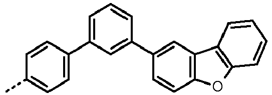 |
| 4"'-139, 5"'-139, 10"'-139, 11'"-139 | 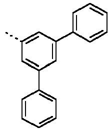 | 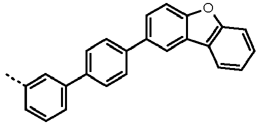 |
| 4"'-140, 5"'-140, 10"'-140, 11"'-140 | 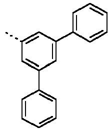 | 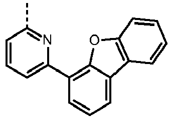 |
| 4"'-141, 5"'-141, 10"'-141, 11"'-141 | 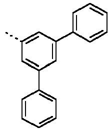 | 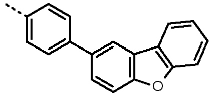 |
| 4"'-142, 5"'-142, 10"'-142, 11"'-142 | 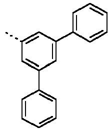 | 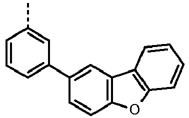 |
| 4"'-143, 5"'-143, 10"'-143, 11'"-143 | 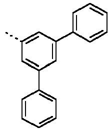 | 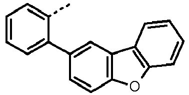 |
| 4"'-144, 5"'-144, 10"'-144, 11'"-144 | 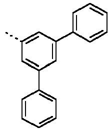 | 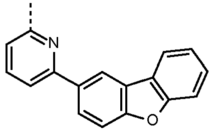 |
| 4"'-145, 5"'-145, 10"'-145, 11'"-145 | 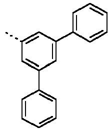 | 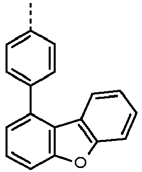 |
| 4"'-146, 5"'-146, 10"'-146, 11"'-146 | 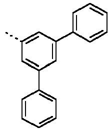 | 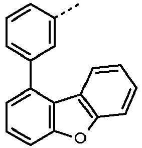 |
| 4"'-147, 5"'-147, 10"'-147, 11'"-147 | 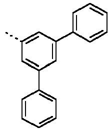 | 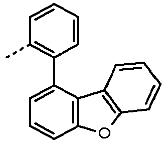 |
| 4"'-148, 5"'-148, 10"'-148, 11'"-148 | 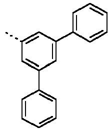 | 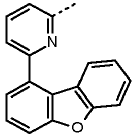 |
| 4"'-149, 5"'-149, 10"'-149, 11"'-149 | 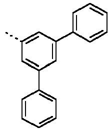 | 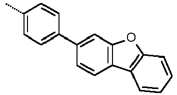 |
| 4"'-150, 5"'-150, 10"'-150, 11"'-150 | 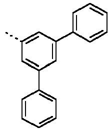 | 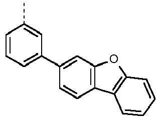 |
| 4"'-151, 5"'-151, 10"'-151, 11'"-151 | 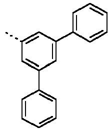 | 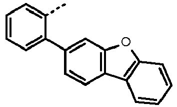 |
| 4"'-152, 5"'-152, 10"'-152, 11'"-152 | 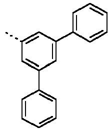 | 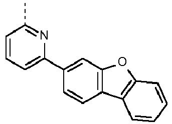 |
| 4"'-153, 5"'-153, 10"'-153, 11"'-153 | 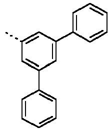 | 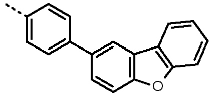 |
| 4"'-154, 5"'-154, 10"'-154, 11'"-154 | 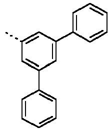 | 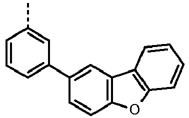 |
| 4"'-155, 5"'-155, 10"'-155, 11"'-155 | 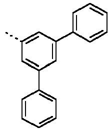 | 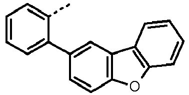 |
| 4"'-156, 5"'-156, 10"'-156, 11"'-156 | 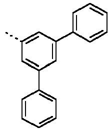 | 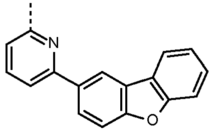 |

The specific heterocyclic derivatives of formula (**1**) of the present invention are found to be suitable for use in organo-electroluminescent devices. In particular, the heterocyclic derivatives of formula (**1**) are suitable host materials, especially host materials for phosphorescent emitters, hole transport materials and/or electron/exciton blocker materials with good efficiency and durability.

The combination of the linking group B₁, B₂, B₃ and/or B₄ with the group Z gives rise to materials that are highly suitable in devices that emit green, red or yellow light, preferably green or red light, more preferably green light, as well as to more balanced charge transport in devices resulting in low voltages and high external quantum efficiencies (EQE's) and/or long lifetimes.

One key finding of the inventors of the present invention is the relevance of the linking group B₁, B₂, B₃ and/or B₄ to achieve better lifetimes in organic electronic devices compared with organic electronic devices comprising compounds of the prior art.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices (EL devices), such as, for example, organic light-emitting diodes (OLEDs). Preferably, the compounds of the present invention are used in electroluminescent devices, especially in OLEDs.

Accordingly, a further subject of the present invention is directed to an electronic device, comprising a compound according to the present invention. The electronic device is preferably an electroluminescent device (EL-device), especially an OLED.

The compounds of formula (**1**) can in principal be used in any layer of an EL device, but are preferably used as host, hole transport and/or electron/exciton blocking material. Particularly, the compounds of formula (1) are used as host material for green, red or yellow, preferably green or red, more preferably green light emitting emitters, which are preferably phosphorescent emitters.

Hence, a further subject of the present invention is directed to a hole transport layer comprising a compound of formula (**1**) according to the present invention.

A further subject of the present invention is directed to an emitting layer, comprising a compound of formula (**1**) according to the present invention. In said embodiment a compound of formula (**1**) is preferably used as host material in combination with an emitter, which is preferably a phosphorescent emitter. The compound of formula (**1**) is useful as a single host material in the light-emitting layer or as co-host together with one or more, preferably one further host material. Suitable further host materials which are useful with the compound of formula (**1**) as co-host are mentioned below.

A further subject of the present invention is directed to a electron/exciton blocking layer, comprising a compound of formula (**1**) according to the present invention.

### Synthesis oft the compounds of formula (1)

### Synthesis of the compounds of formula (1)

Generally, the heterocyclic derivatives of formula (1) are prepared in analogy to the preparation processes described in the prior art, e.g. in WO2012/130709, WO2014/009317, WO2014/044722, European patent application no. 13191100.0, WO2015/014791, European patent application no. EP14197947.9 and European patent application no. EP14197952.6.

The present invention further relates to a process for the preparation of the heterocyclic derivatives of formula (**1**) comprising:
a) Coupling of a group 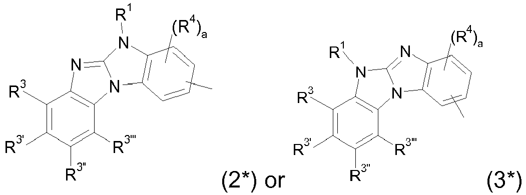 with a group 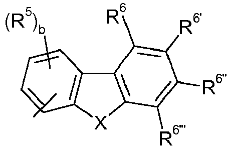 via a group L¹,
   whereby a heterocyclic group A of formula (2) or formula (3) is obtained 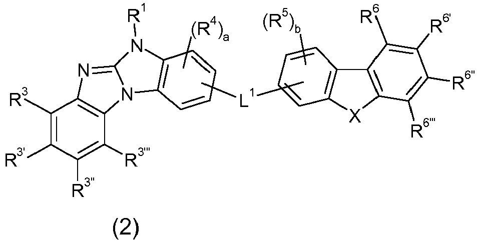 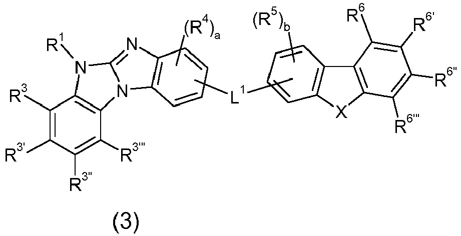 wherein the residues and groups R¹, R³, R^{3'}, R^{3"}, R^{3"'}, R⁴, R⁵, R⁶, R^{6'}, R^{6"}, R^{6"'},X and L¹ and the indices a and b have been described above; and
b) Introduction of one groups -(B₁)ₒ-(B₂)ₚ-(B₃)_{q}-(B₄)ᵣ-Z into the heterocyclic group A of formula (2) or formula (3),
wherein the groups B₁, B₂, B₃, B₄ and Z and the indices o, p, q and r have been described above;
whereby a heterocyclic derivative of formula (**1**)

A-[(B₁)ₒ-(B₂)ₚ-(B₃)_{q}-(B₄)ᵣ-Z]_{z} (**1**)

is obtained.

Preferred heterocyclic derivatives of formula (**1**) are mentioned above.

Specific reaction conditions of the steps a) and b) of the process according to the present invention are described below as well as in the example part of the present application.

The preparation of the group -(B₁)ₒ-(B₂)ₚ-(B₃)_{q}-(B₄)ᵣ-Z is known by a person skilled in the art. Specific reaction conditions for a functionalization of the group Z are additionally mentioned below.

In the following, two examples for preparation processes for compounds of formula (1) are shown, wherein X is NR⁷ and one of R¹ and R⁷ or both, R¹ and R⁷, is/are -(B₁)ₒ-(B₂)ₚ-(B₃)_{q}-(B₄)ᵣ-Z, and the other group R¹ respectively R⁷ is as defined above, preferably phenyl:

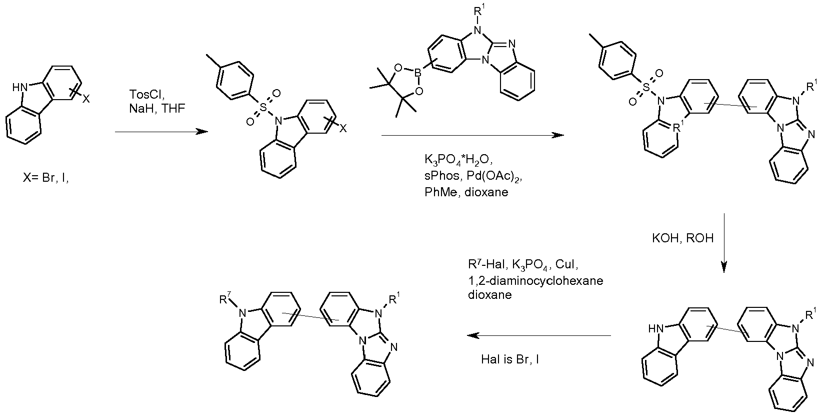

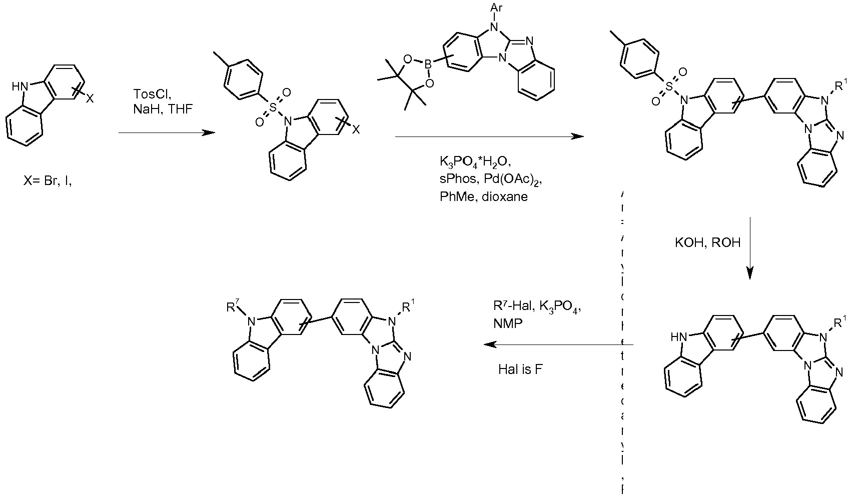

### Base skeleton

The synthesis of the compounds of formula (**1**) can be carried out in analogy to the synthesis of benzimidazolo[1,2-a]benzimidazoles mentioned in the prior art.

The synthesis of 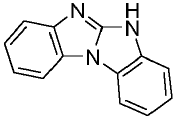 is described, for example, in Achour, Reddouane; Zniber, Rachid, Bulletin des Societes Chimiques Belges 96 (1987) 787-92, WO12130709, Org. Lett. 2012,14, 02, 452, Eur. J. Org. Chem. 2014, 5986-5997, and RSC Advances 2014, 4, 21904-21908

### N-arylation

The introduction of the group -R¹ (N-arylation) is generally carried out by reacting the base skeleton 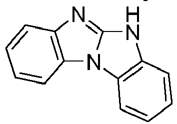 with a group Hal-R¹, wherein Hal is F, Cl, Br or I, preferably F, Br or I. Suitable groups R¹ are mentioned before.

The nucleophilic aromatic substitution (N-arylation) of 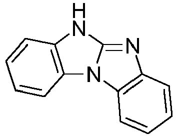 with F-R¹ is generally performed in the presence of a base (Angew. Chem. 2012, 124, 8136 -8140, Angew. Chem. Int. Ed. 2008, 47, 8104 -8107). Suitable bases are known to those skilled in the art and are preferably selected from the group consisting of alkali metal alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, alkaline metal phosphates such as K₃PO₄ alkaline metal fluorides such as KF, CsF and alkali metal alkoxides such as NaOMe, NaOEt. In addition, mixtures of the aforementioned bases are suitable. K₂CO₃ or Cs₂CO₃, K₃PO₄ are preferred.

The nucleophilic aromatic substitution (N-arylation) can be performed in solvent or in a melt. Preferably, the reaction is carried out in a solvent. Suitable solvents are, for example, (polar) aprotic solvents such as dimethyl sulfoxide (DMSO), dimethylformamide (DMF), N-methyl-2-pyrrolidone (NMP) or dimethylacetamide (DMA).

The reaction temperature is strongly dependent on the reactivity of the aryl fluoride. The reaction (N-arylation) is preferably carried out at a temperature of -10 to 220 °C, more preferably 60 to 150 °C.

Ullmann reaction (N-arylation) of 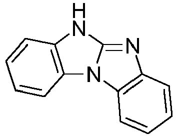 with Y-R¹ (Y is Cl, Br, or I) generally performed in the presence of a base and a catalyst.

Reaction conditions for Ullmann reactions are, for example, described in Angew Chem Int Ed Engl., 48 (2009) 6954-71 WO14009317, WO12130709, J. Am. Chem. Soc. 131 (2009) 2009-2251, J. Org. Chem, 70 (2005) 5165.

Typically the Ullmann coupling of the compound of formula 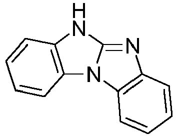 with a compound of formula Y-R¹ (Y is Cl, Br, or I, especially Br, I very especially I) is done in the presence of copper, or a copper salt, such as, for example, Cul, CuBr, Cu₂O, or CuO, and a ligand, such as, for example, L-proline, trans-cyclohexane-1,2-diamine (DACH), 1,10-phenanthroline in a solvent, such as, for example, dimethylsulfoxide (DMSO), dimethylformamide (DMF), dimethylacetamide (DMA), N-methylpyrrolidone (NMP) and dioxane, or a solvent mixture. The reaction temperature is dependent on the reactivity of the starting materials, but is generally in the range of 25 to 200 °C. If copper salt are used without a ligand the reaction temperatures are higher.

The N-arylation is, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151.

Suitable base skeletons of the formula 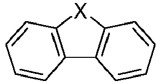 are either commercially available (especially in the cases when X is S, O, NH), or can be obtained by processes known to those skilled in the art. Reference is made to WO2010079051 and EP1885818.

The halogenation of said base skeletons 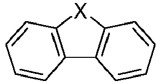 (carbazole, dibenzofuran or dibezothiophene, which is unsubstituted or substituted) can be performed by methods known to those skilled in the art. Preference is given to brominating or iodinating in the 3 and 6 positions (dibromination, diiodation or mixed bromination/iodation) or in the 3 or 6 positions (monobromination, monoiodation) of the base skeleton in the case of carbazole, respectively in the 2 and 8 positions (dibromination, diiodation) or in the 2 or 8 positions (monobromination, monoiodation) of the base skeleton in the case of dibenzofuran and dibenzothiophene.

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

For the nucleophilic substitution, Cl- or F-substituted dibenzofurans, dibenzothiophenes and carbazoles are preferred. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 (2002) 953-957.

*Introduction of the* 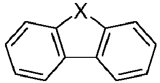 *skeleton*

The introduction of the 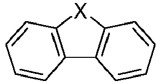 skeleton, can be affected, for example, by copper-catalyzed coupling (Ullmann reaction). Suitable reaction components and reaction conditions for carrying out the Ullmann reaction are mentioned above.

Alternatively, the introduction of the 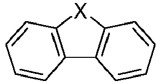 skeleton, especially in cases, wherein the 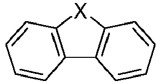 skeleton is substituted, e.g. by a group 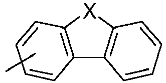 or 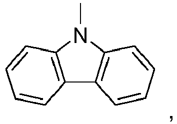 can be affected, for example, by Pd catalyzed coupling of diboronic acid or diboronate group containing dibenzofurans, dibenzothiophenes or carbazoles with halogenated aromatic groups, wherein the halogen is preferably I (Suzuki coupling).

An Example for a Suzuki coupling is shown in the example part of the present application:

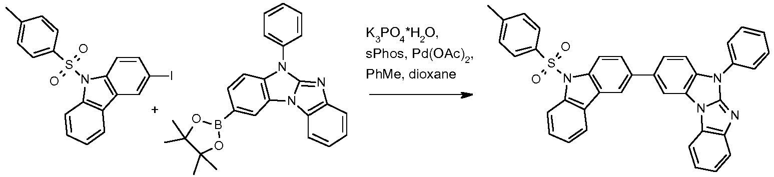

Diboronic acid or diboronate group containing dibenzofurans, dibenzothiophenes and carbazoles can be readily prepared by an increasing number of routes. An overview of the synthetic routes is, for example, given in Angew. Chem. Int. Ed. 48 (2009) 9240 - 9261.

By one common route diboronic acid or diboronate group containing dibenzofurans, dibenzothiophenes, and carbazoles can be obtained by reacting halogenated dibenzofurans, dibenzothiophenes and carbazoles with (Y¹O)₂B-B(OY¹)₂, 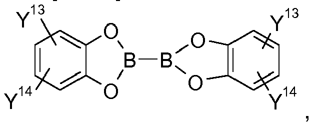 or 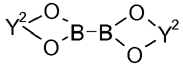 in the presence of a catalyst, such as, for example, [1,1'-bis(diphenylphosphino)ferrocene]dichloropalladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204), wherein Y¹ is independently in each occurrence a C₁-C₁₈alkylgroup and Y² is independently in each occurrence a C₂-C₁₀alkylene group, such as -CY³Y⁴-CY⁵Y⁶-, or-CY⁷Y⁸-CY⁹Y¹⁰-CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, - C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup.

Diboronic acid or diboronate group containing dibenzofurans, dibenzothiophenes and carbazoles can also be prepared by reacting halogenated dibenzofurans, dibenzothiophenes and carbazoles with alkyl lithium reagents, such as, for example, n-butyl lithium, or t-buthyl lithium, followed by reaction with boronic esters, such as, for example, B(isopropoxy)₃, B(methoxy)₃, or 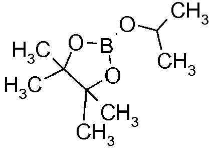 (cf. Synthesis (2000) 442-446). Diboronic acid or diboronate group containing dibenzofurans, dibenzothiophenes and carbazoles can also be prepared by reacting dibenzofurans, dibenzothiophenes and carbazoles with lithium amides such as, for example, lithium diisopropylamide (LDA) followed by reaction with boronic esters such as, for example, B(isopropoxy)₃, B(methoxy)₃, or 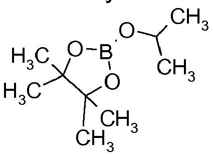 (J. Org. Chem. 73 (2008) 2176-2181).

### Compounds of formula (1) in organic electronics applications

It has been found that the compounds of the formula (1) are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs).

The *organic transistor* generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor. The layers with charge transport capacity may comprise the compounds of formula (**1**).

The *organic solar cell* (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compounds of formula (**1**).

The compounds of the formula (**1**) being particularly suitable in *OLEDs* for use as matrix material in a light-emitting layer and/or as electron and/or exciton blocker material and/or hole transport materials, especially in combination with a phosphorescence emitter.

In the case of use of the inventive compounds of the formula (**1**) in OLEDs, OLEDs which have good efficiencies and a long lifetime and which can be operated especially at a low use and operating voltage are obtained. The inventive compounds of the formula (**1**) are suitable especially for use as matrix and/or hole transport and/or electron/exciton blocker materials for green, red and yellow, preferably green and red, more preferably green emitters. Furthermore, the compounds of the formula (**1**) can be used as conductor/complementary materials in organic electronics applications selected from switching elements and organic solar cells. (In the sense of the present application, the terms matrix and host are used interchangeable).

In the emission layer or one of the emission layers of an OLED, it is also possible to combine an emitter material with at least one matrix material of the compound of the formula (**1**) and one or more, preferably one, further matrix materials (co-host). This may achieve a high quantum efficiency, low driving voltage and/or long lifetime of this devices.

It is likewise possible that the compounds of the formula (**1**) are present in two or three of the following layers: in the light-emitting layer (preferably as matrix material), in the blocking layer (as electron/exciton blockers) and/or in the transport layer (as hole transport material).

When a compound of the formula the compound of the formula (**1**) is used as matrix (host) material in an emission layer and additionally as electron/exciton blocker material and/or as hole transport material, owing to the chemical identity or similarity of the materials, an improved interface between the emission layer and the adjacent material, which can lead to a decrease in the voltage with equal luminance and to an extension of the lifetime of the OLED. Moreover, the use of the same material as hole transport material and/or as electron/exciton blocker material and as matrix of an emission layer allows the production process of an OLED to be simplified, since the same source can be used for the vapor deposition process of the material of one of the compounds of the formula the compound of the formula (1).

Suitable structures of organic electronic devices, especially organic light-emitting diodes (OLED), are known to those skilled in the art and are specified below.

The present invention further provides an organic light-emitting diode (OLED) comprising an anode (a) and a cathode (i) and a light-emitting layer (e) arranged between the anode (a) and the cathode (i), and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula (**1**) is present in the light-emitting layer (e) and/or in at least one of the further layers. The at least one compound of the formula the compound of the formula (**1**) is preferably present in the light-emitting layer and/or the electron/exciton blocking layer and/or the hole transport layer.

In a preferred embodiment of the present invention, at least one compound of the formula (**1**) is used as hole transport material. Examples of preferred compounds of the formula (**1**) are shown above.

In another preferred embodiment of the present invention, at least one compound of the formula (**1**) is used as electron/exciton blocker material. Examples of preferred compounds of the formula (**1**) are shown above.

The present application further relates to a light-emitting layer comprising at least one compound of the formula (**1**), preferably as host material or co-host material. Examples of preferred compounds of formula the compound of the formula (**1**) are shown above.

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

An example for a suitable hole injection material is: 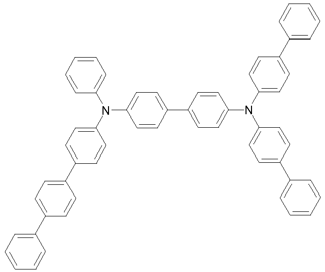 (see also hole-transporting molecules).

### Hole transport layer (c):

The compound of the formula (**1**) is suitable as hole transport material, either alone or in combination with one or more of the hole transport materials mentioned below.

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 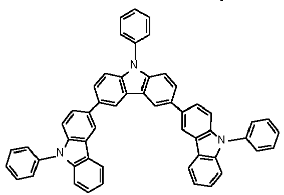 (HTL1-1) and 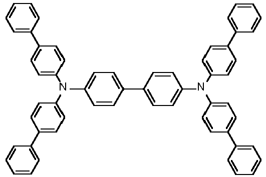 (HTL2-1) constitute the hole transport layer.

Customarily used hole-transporting molecules are selected from the group consisting of 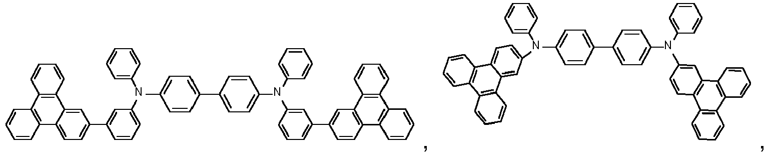 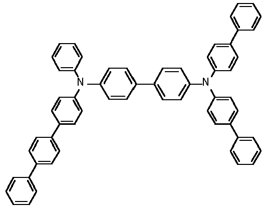 (4-phenyl-N-(4-phenylphenyl)-N-[4-[4-(N-[4-(4-phenyl-phenyl)phenyl]anilino)phenyl]phenyl]aniline), 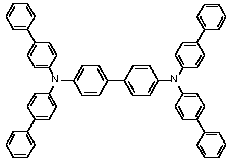 (4-phenyl-N-(4-phenylphenyl)-N-[4-[4-(4-phenyl-N-(4-phenylphenyl)anilino)phenyl]phenyl]aniline), 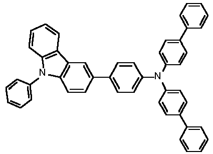 (4-phenyl-N-[4-(9-phenylcarbazol-3-yl)phenyl]-N-(4-phenylphenyl)aniline), 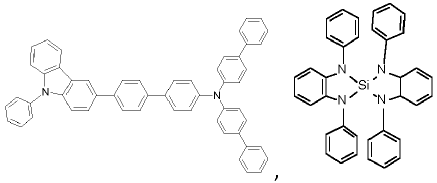 (1,1',3,3'-tetraphenylspiro[1,3,2-benzodiazasilole-2,2'-3a,7a-dihydro-1,3,2-benzodiazasilole]), 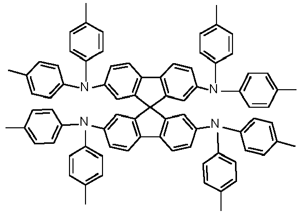 (N2,N2,N2',N2',N7,N7,N7',N7'-octakis(p-tolyl)-9,9'-spirobi[fluorene]-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetra-cyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254.

### Electron/exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

The compound of the formula (**1**) is suitable as electron/exciton blocker material, either alone or in combination with one or more of the electron/exciton blocker materials mentioned below.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application.

### Emitting layer (e)

The light emitting layer is an organic layer having a light emitting function and is formed from one or more layers, wherein one of the layers comprises the host material and the light emitting material as described below.

Preferably, the light emitting layer of the inventive OLED comprises at least one compound of formula (1) as host material.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are combinedly used and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) comprises at least one emitter material. In principle, it may be a fluorescence or phosphorescence emitter, suitable emitter materials being known to those skilled in the art. The at least one emitter material is preferably a phosphorescence emitter.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 nm to 570 nm).

The phosphorescent dopant (phosphorescent emitter material) is a compound which emits light by releasing the energy of excited triplet state and preferably a organometallic complex comprising at least one metal selected from Ir, Pt, Pd, Os, Au, Cu, Re, Rh and Ru and a ligand, although not particularly limited thereto as long as emitting light by releasing the energy of excited triplet state. A ligand having an ortho metal bond is preferred. In view of obtaining a high phosphorescent quantum yield and further improving the external quantum efficiency of electroluminescence device, a metal complex comprising a metal selected from Ir, Os, and Pt is preferred, with iridium complex, osmium complex, and platinum, particularly an ortho metallated complex thereof being more preferred, iridium complex and platinum complex being still more preferred, and an ortho metallated iridium complex being particularly preferred.

The compounds of the formula (**1**) can be used as the matrix in the light-emitting layer.

Suitable metal complexes for use in the inventive OLEDs, preferably as emitter material, are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoylmethane)]mono-(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Particularly suitable metal complexes are described in US2012223295, US2014367667, US2013234119, US2014001446, US2014231794, US2014008633, WO2012108388 and WO2012108389. The emitters mentioned in US2013234119, paragraph [0222], are exemplified. Selected emitters, especially red emitters, of said emitters mentioned in US2013234119, paragraph [0222], are:

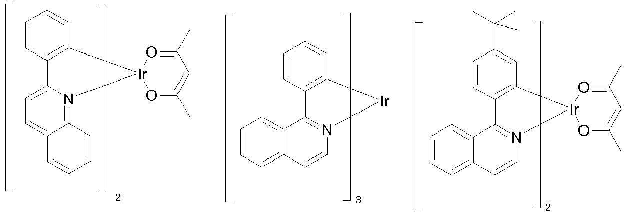

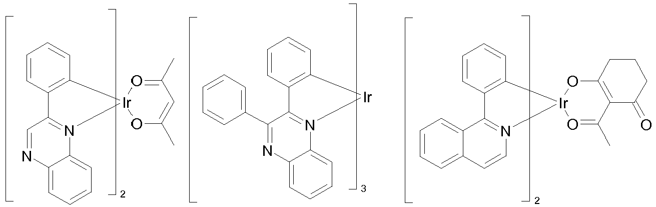

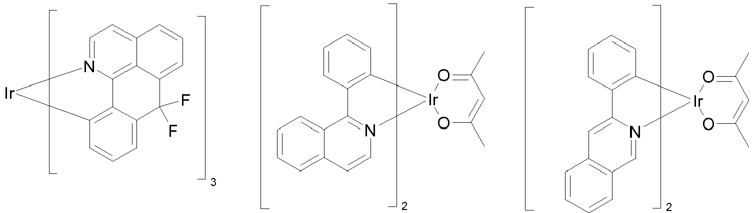

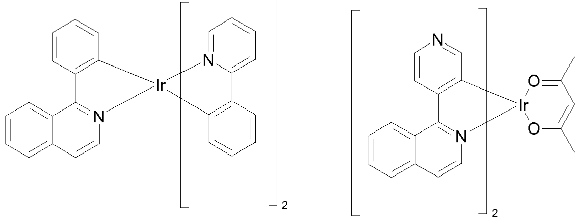

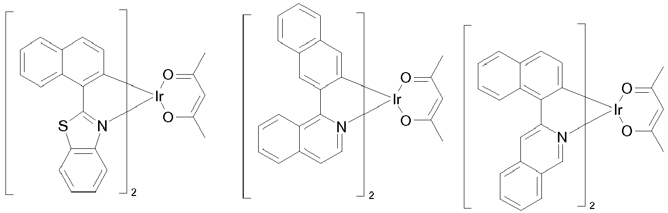

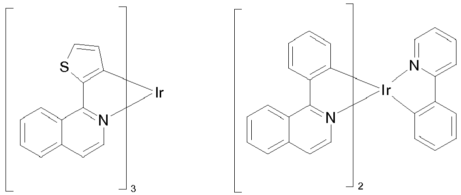

Further suitable Emitters are mentioned in: Mrs Bulletin, 2007, 32, 694:

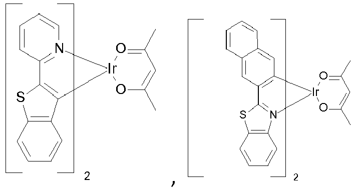

Further suitable Emitters are mentioned in:WO2009100991:

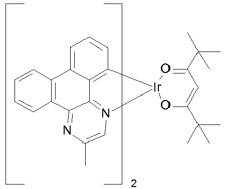

Further suitable Emitters are mentioned in: WO2008101842:

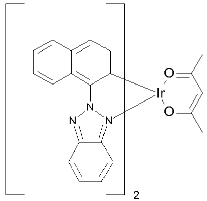

Further suitable Emitters are mentioned in: US 20140048784, especially in paragraph [0159]:

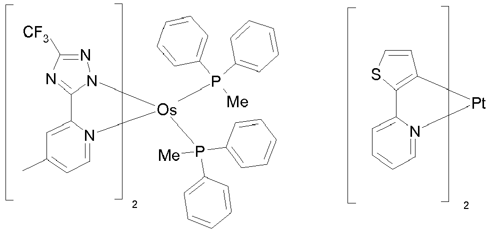

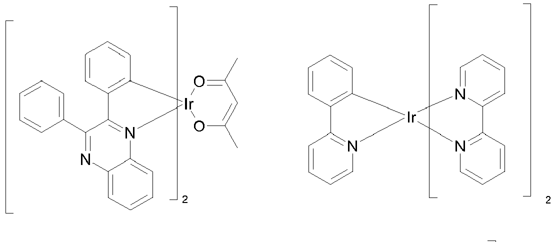

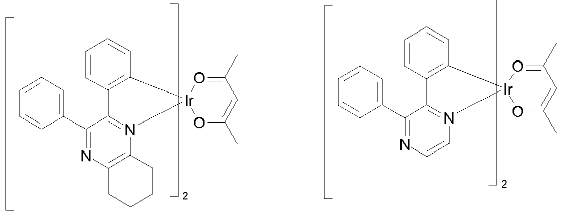

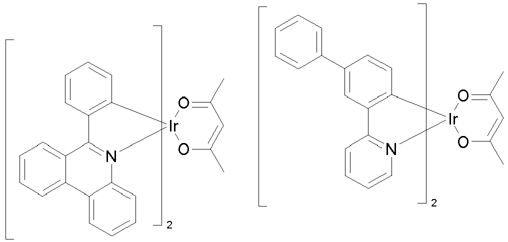

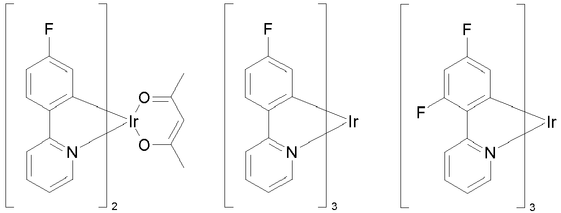

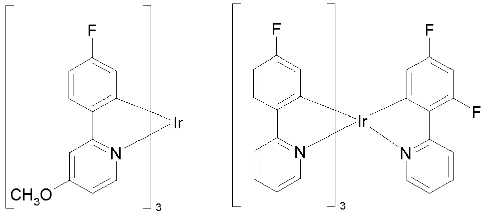

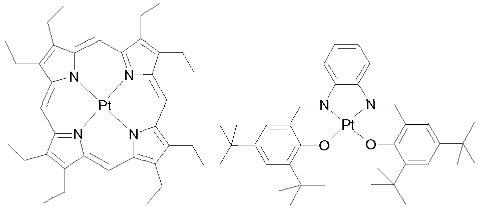

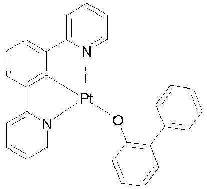

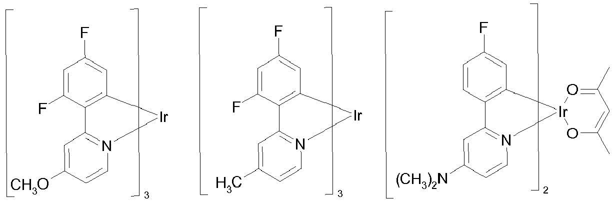

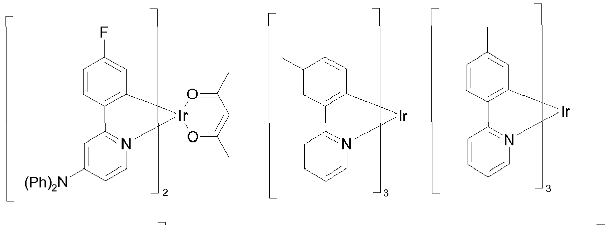

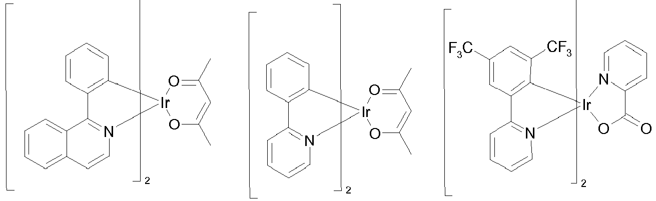

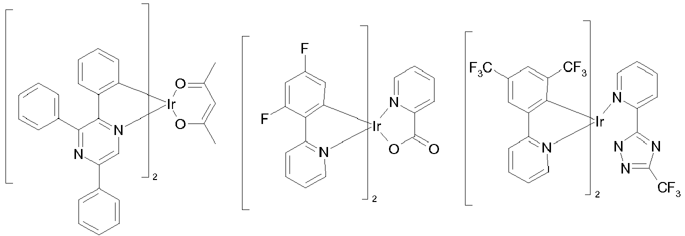

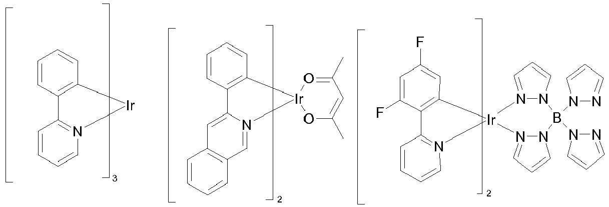

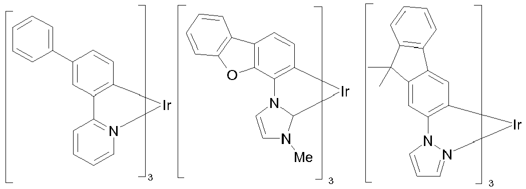

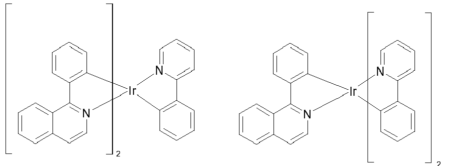

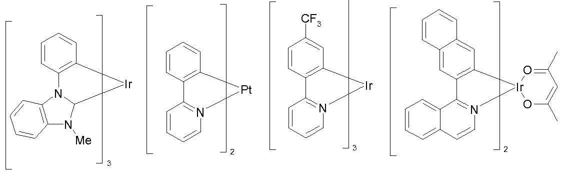

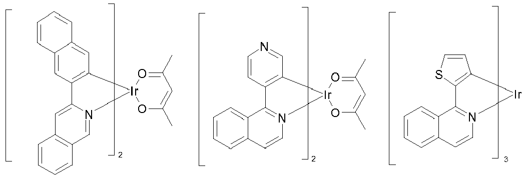

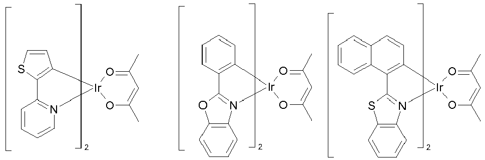

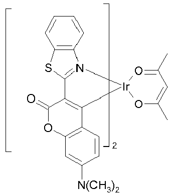

Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, PCT/EP2014/064054 and PCT/EP2014/066272.

The light emitting layer (e) comprises for example at least one carbene complex as phosphorescence emitter. Suitable carbene complexes are, for example, compounds of the formula 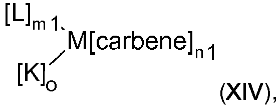 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand, preferably selected from the group consisting of phosphines; phosphonates and derivatives thereof, arsenates and derivatives thereof; phosphites; CO; pyridines; nitriles and conjugated dienes which form a π complex with M¹;
n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 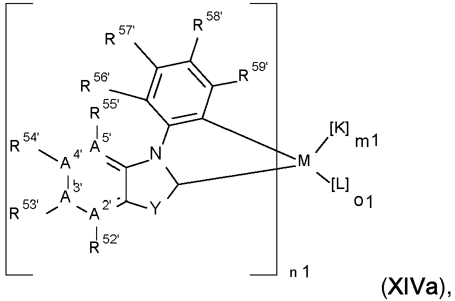 which are described in WO2011/073149,
where M is Ir, or Pt,
n1 is an integer selected from 1, 2 and 3,
Y is NR^{51'}, O, S or C(R^{25'})₂,
A^{2'}, A^{3'}, A^{4'}, and A^{5'}are each independently N or C, where 2 A' = nitrogen atoms and at least one carbon atom is present between two nitrogen atoms in the ring,
R^{51'} is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R^{52'}, R^{53'}, R^{54'} and R^{55'}are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R^{53'} and R^{54'} together with A^{3'}and A^{4'} form an optionally substituted, unsaturated ring optionally interrupted by at least one further heteroatom and having a total of 5 to 18 carbon atoms and/or heteroatoms,
R^{56'}, R^{57'}, R^{58'} and R^{59'} are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R^{56'} and R^{57'}, R^{57'} and R^{58'} or R^{58'} and R^{59'}, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R^{55'} and R^{56'} together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R^{25'} is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula XIV is preferably a compound of the formula:

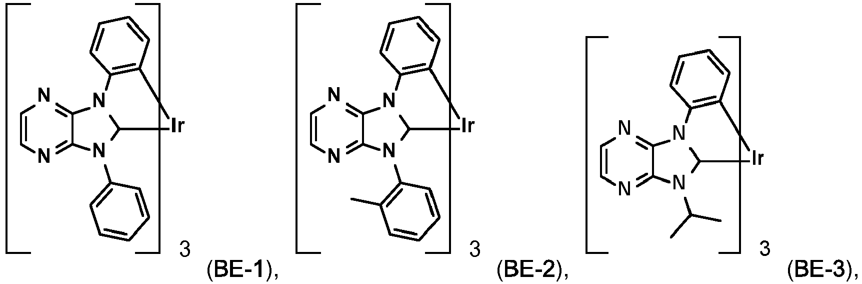

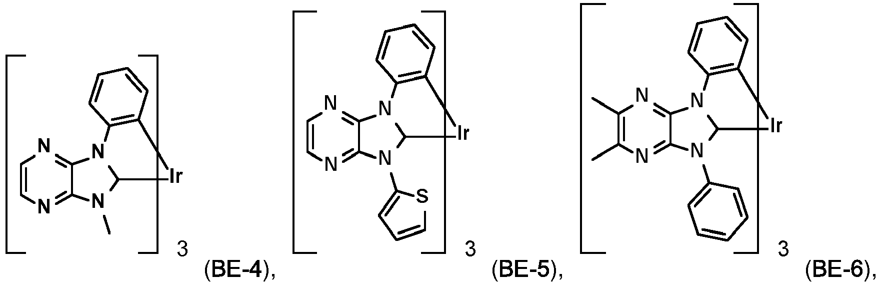

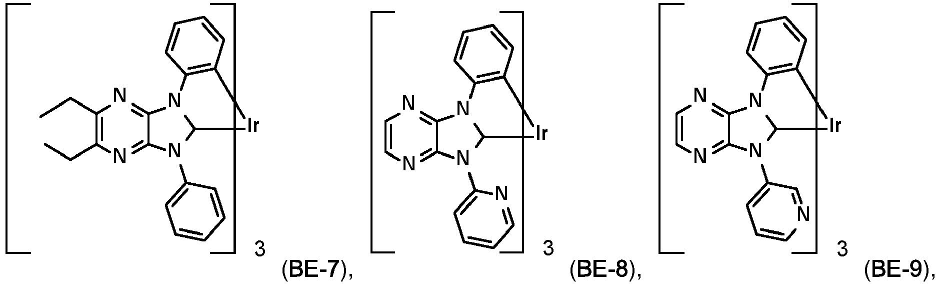

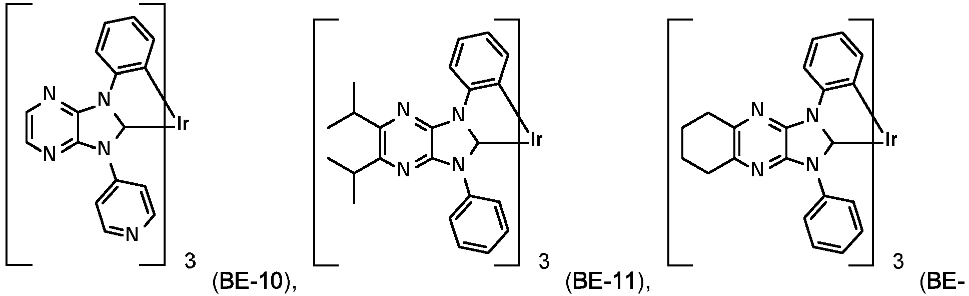

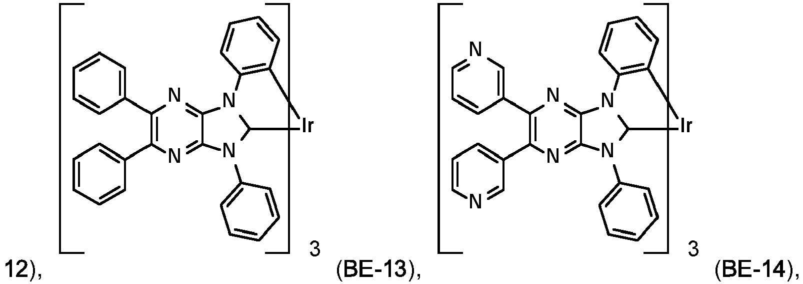

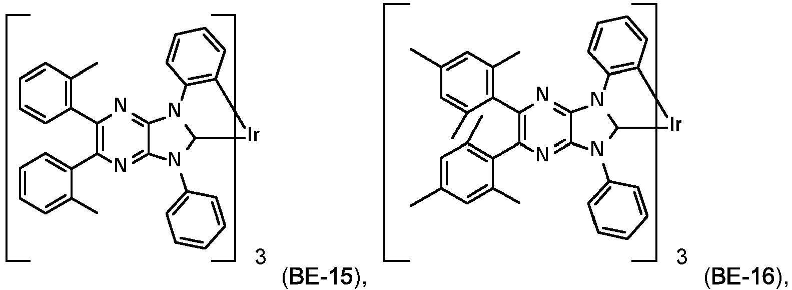

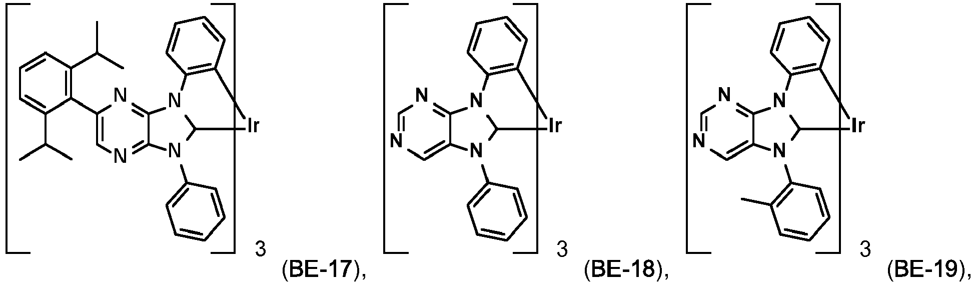

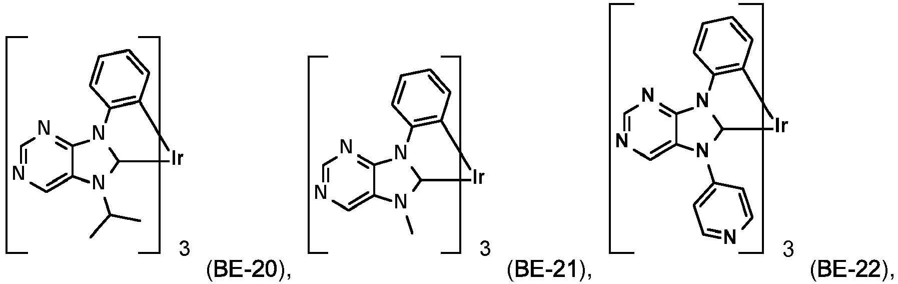

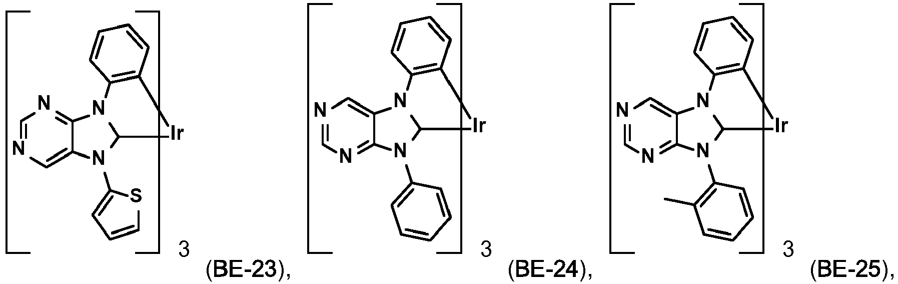

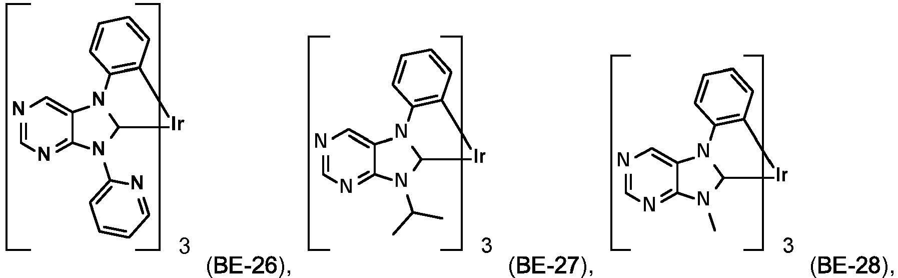

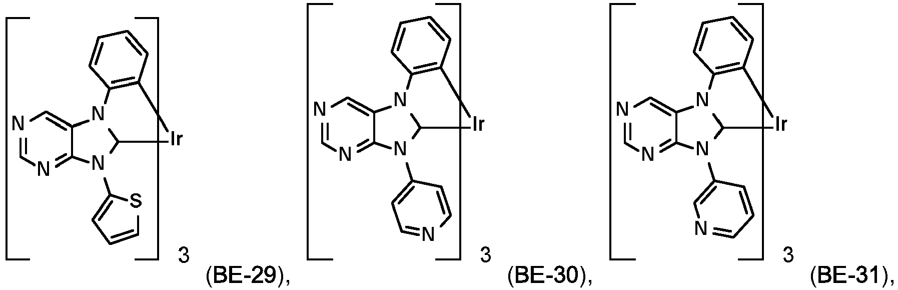

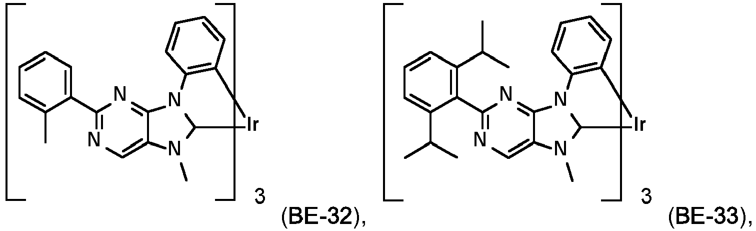

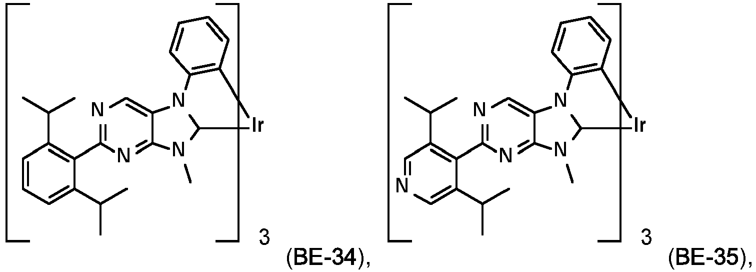

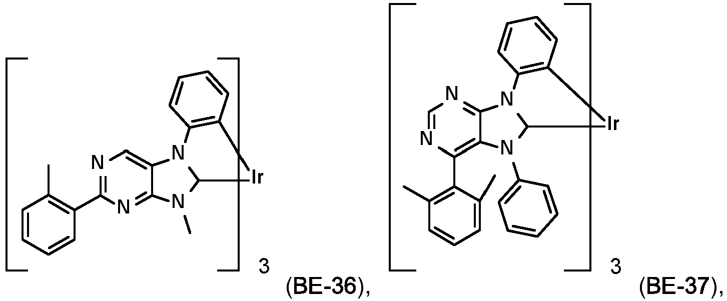

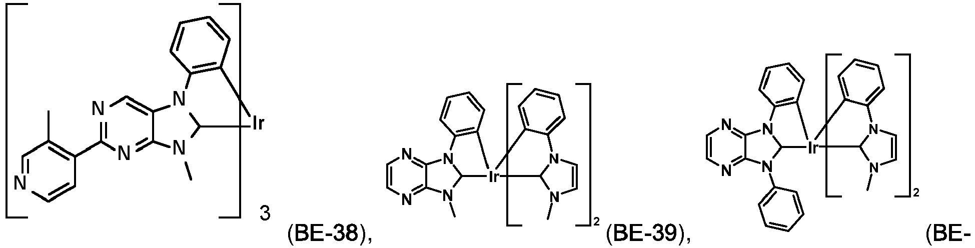

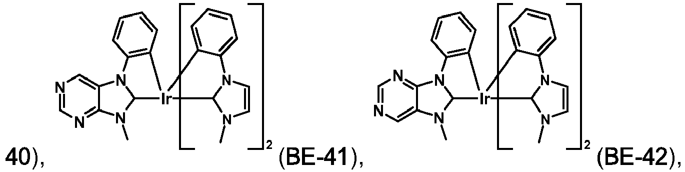

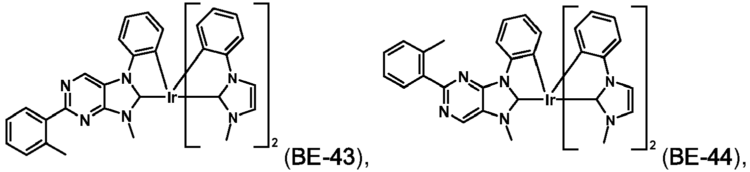

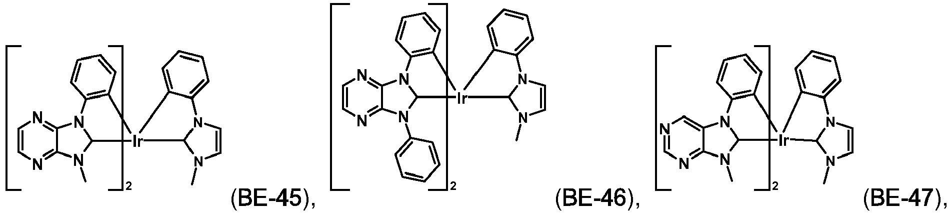

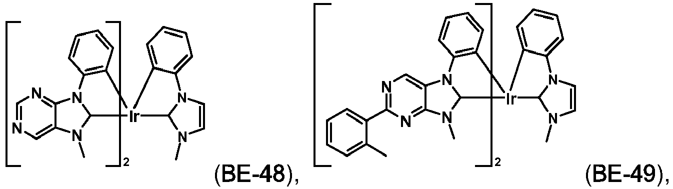

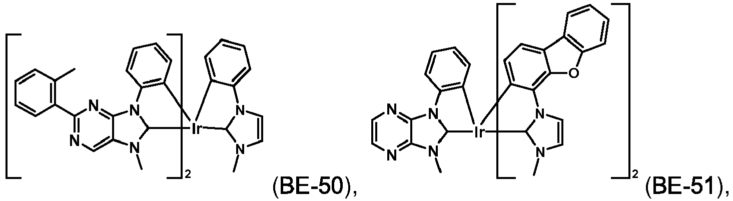

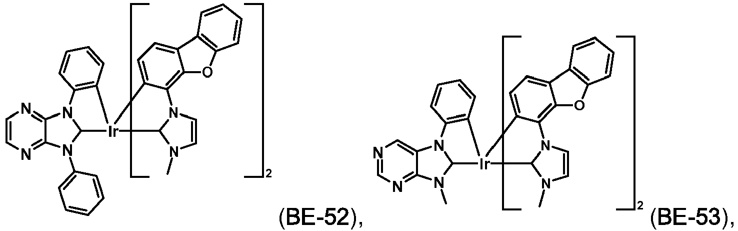

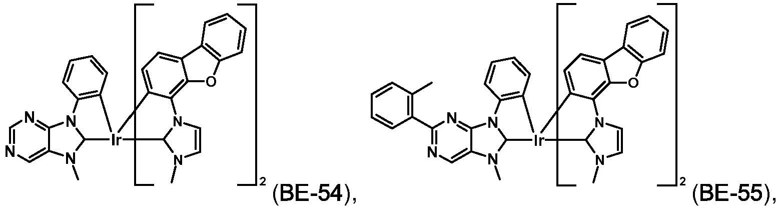

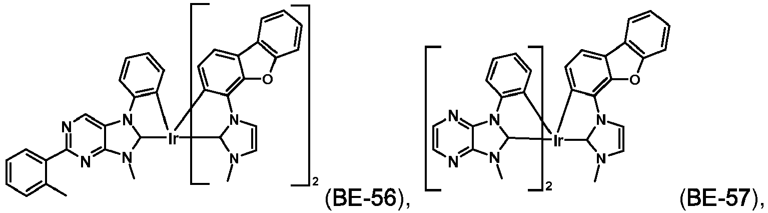

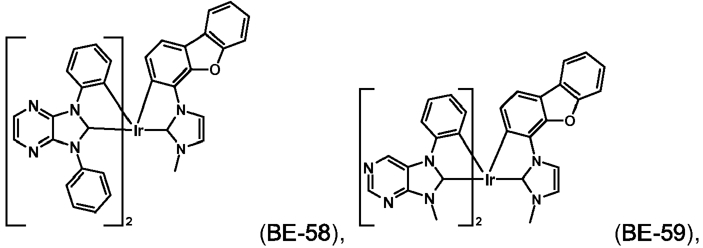

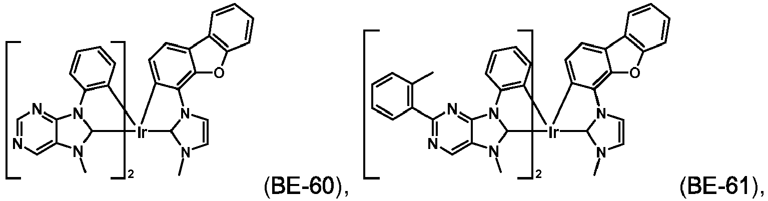

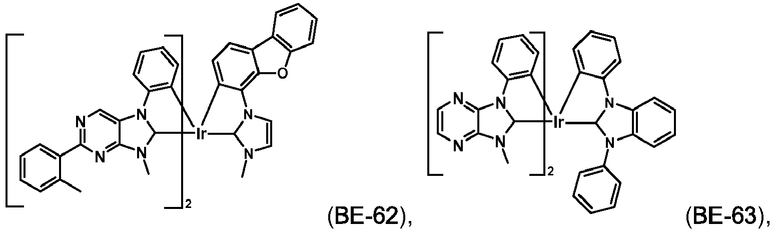

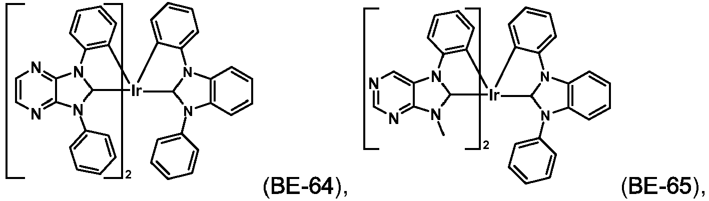

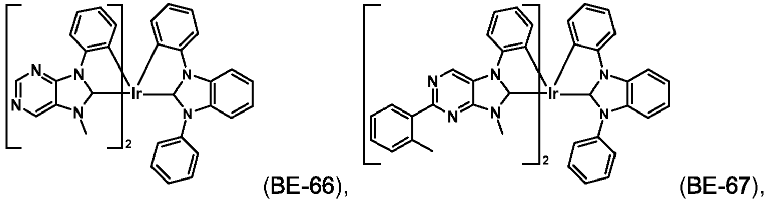

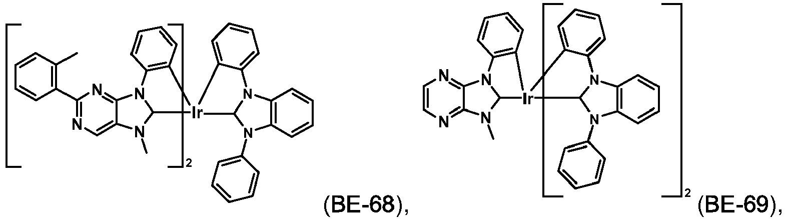

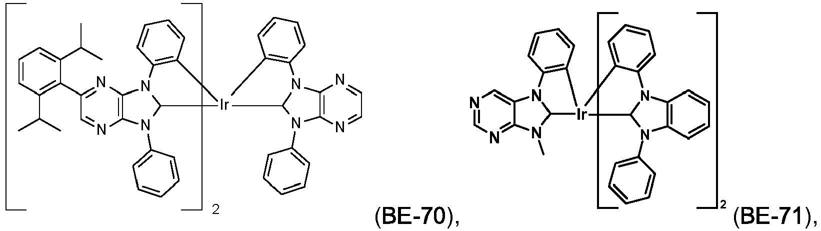

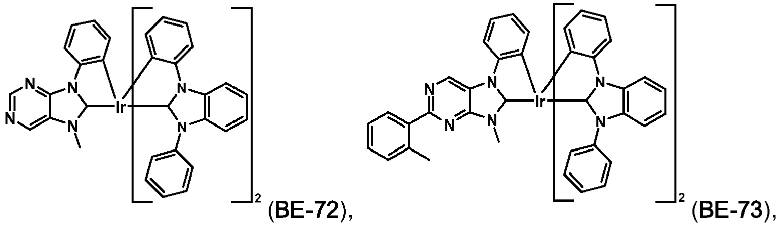

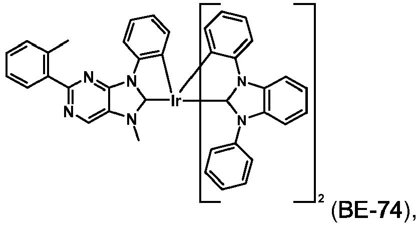

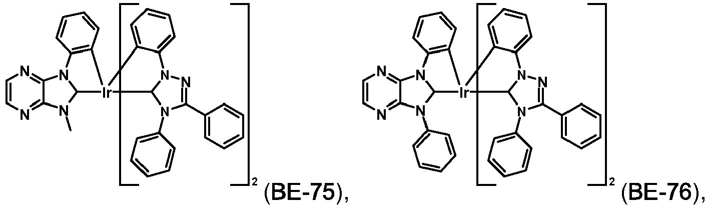

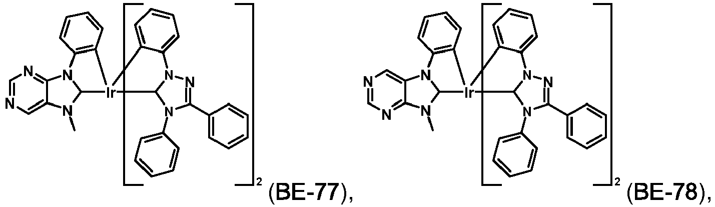

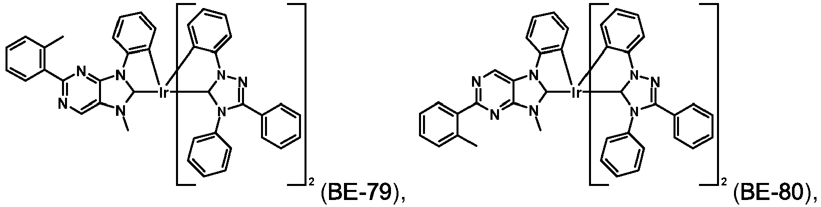

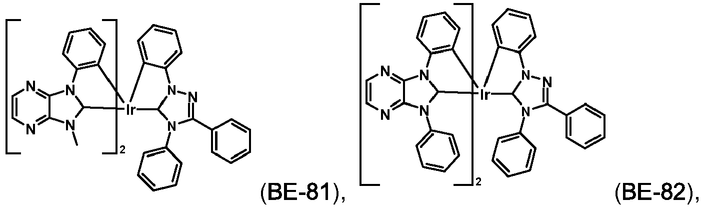

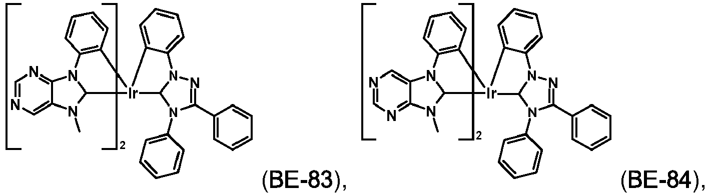

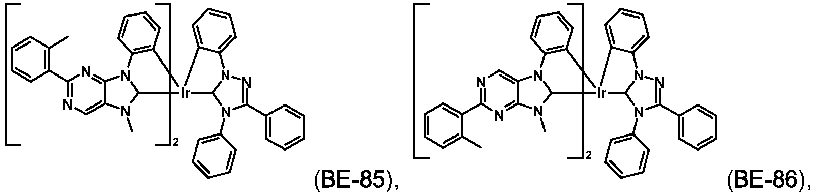

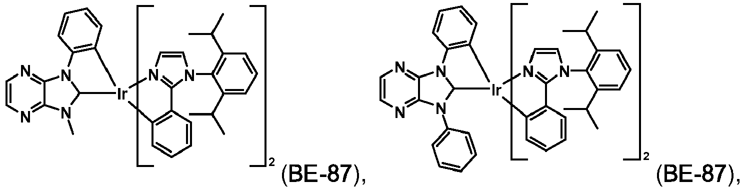

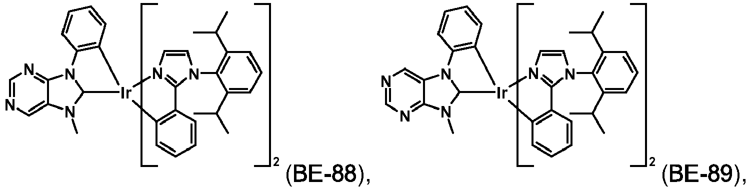

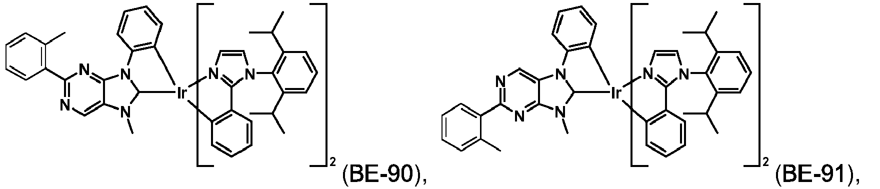

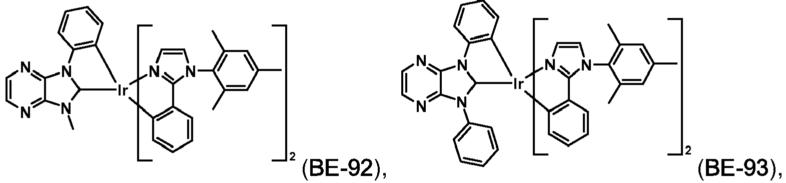

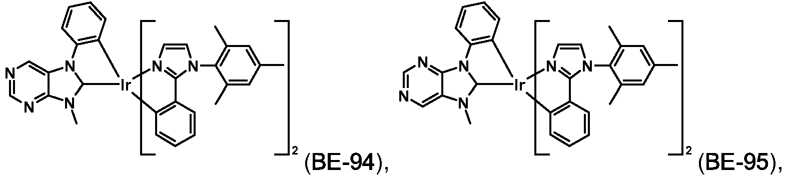

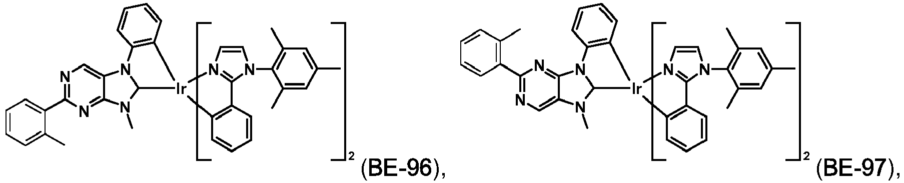

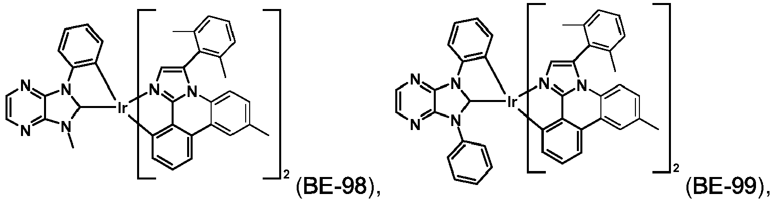

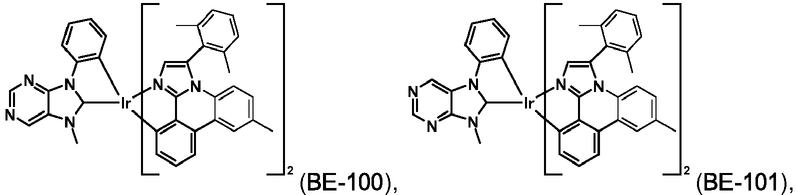

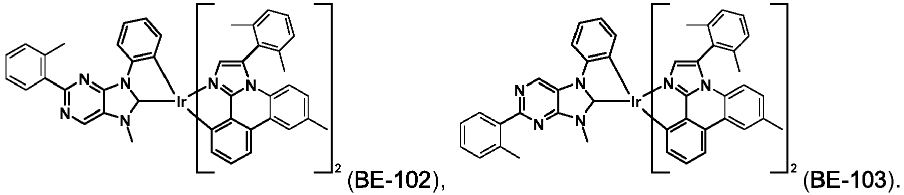

Further suitable non-carbene emitter materials are mentioned below:

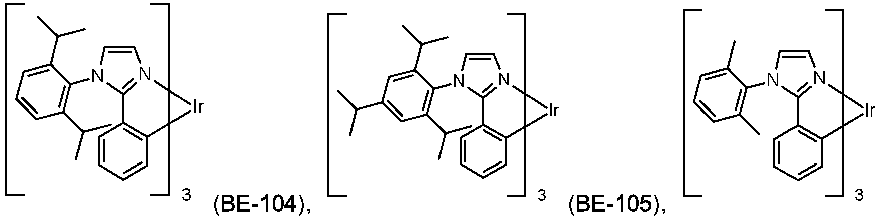

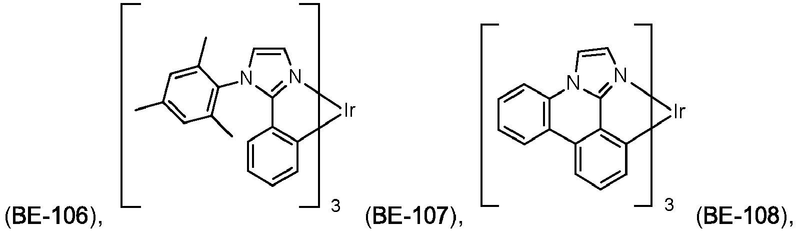

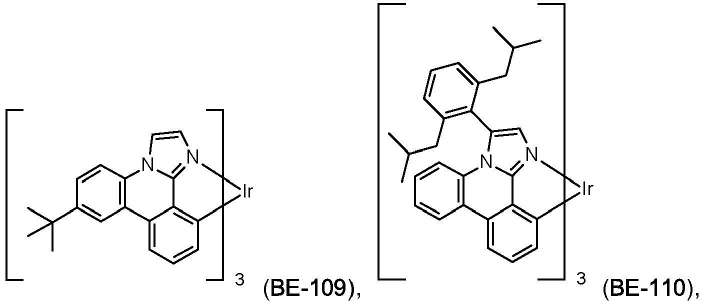

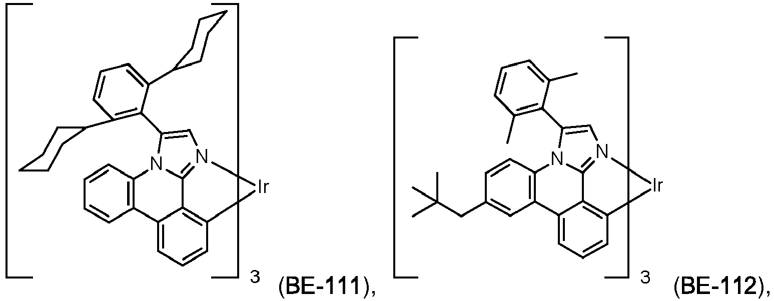

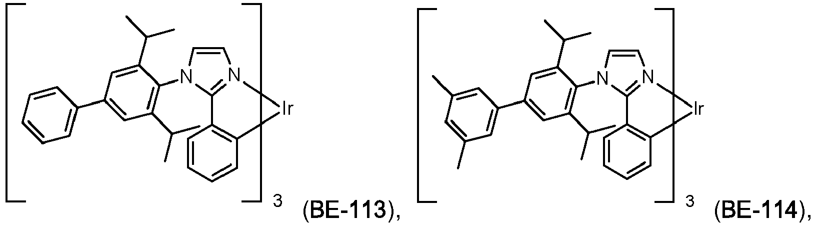

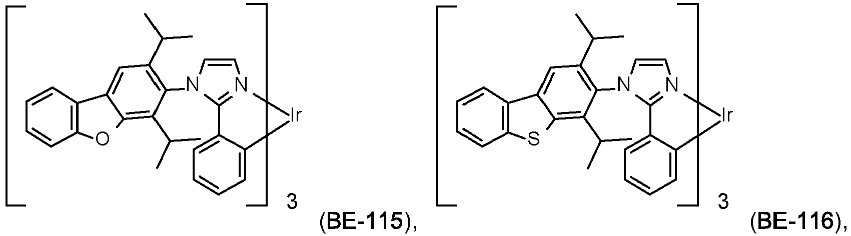

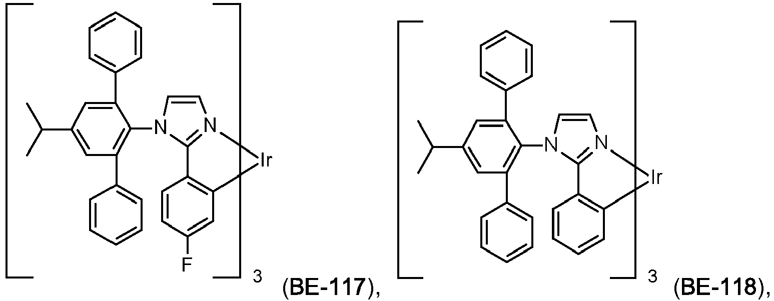

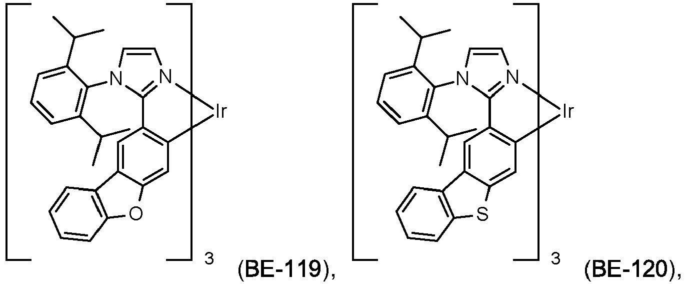

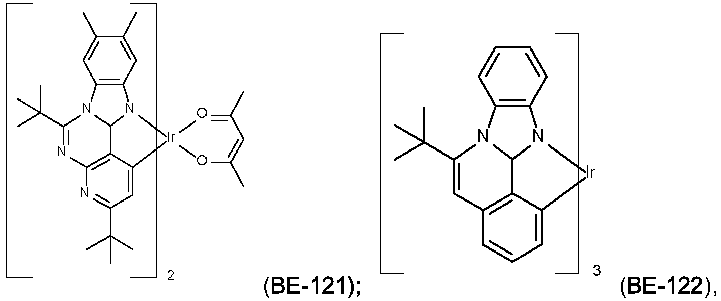

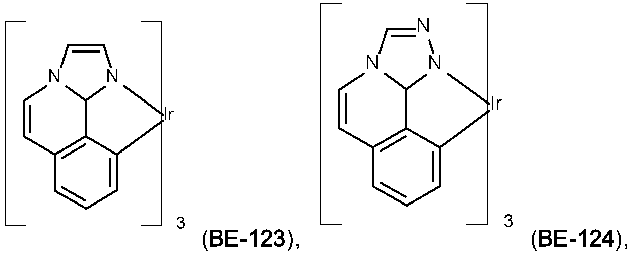

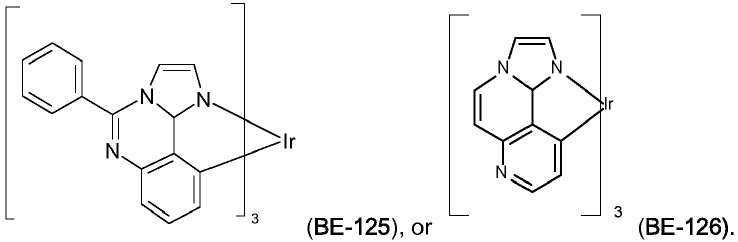

The compound of formula **XIV** is more preferably a compound (**BE-1**), (**BE-2**), (**BE**-**7**), (**BE**-**12**), (**BE**-**16**), (**BE**-**64**), or (**BE**-**70**). The most preferred phosphorescent blue emitters are compounds (**BE-1**) and (**BE**-**12**).

The homoleptic metal-carbene complexes may be present in the form of facial or meridional isomers or mixtures thereof, preference being given to the facial isomers.

Suitable carbene complexes of formula (**XIV**) and their preparation process are, for example, described in WO2011/073149.

The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO2012053627; US6921915, US20090039776; JP2007123392 and European patent application no. 14180422.9.

Examples of suitable phosphorescent green emitters are shown below:

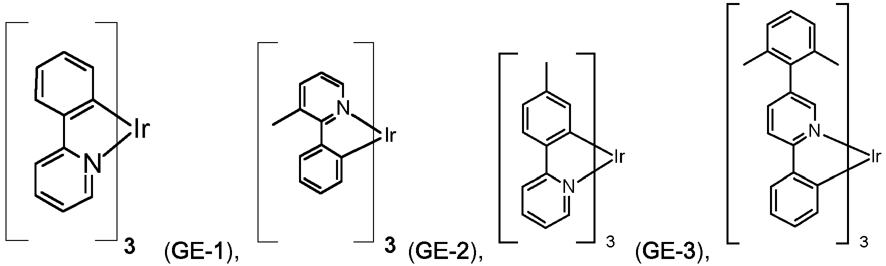

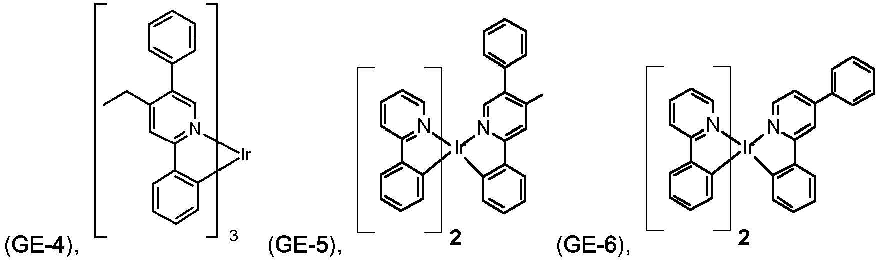

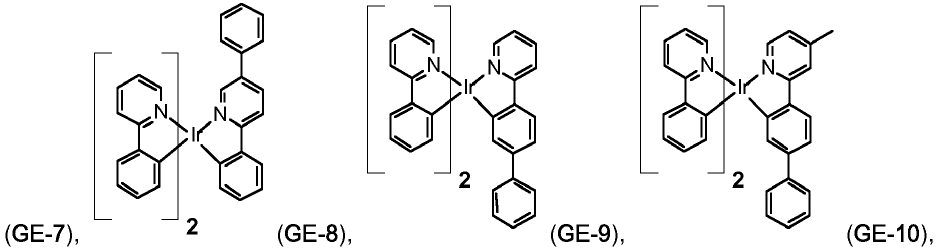

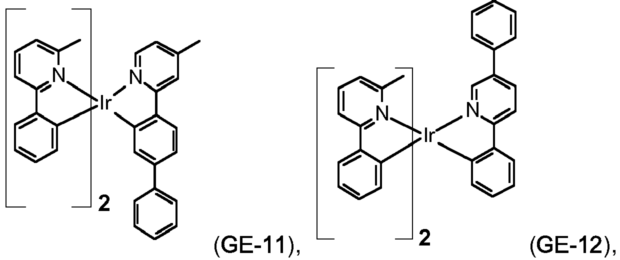

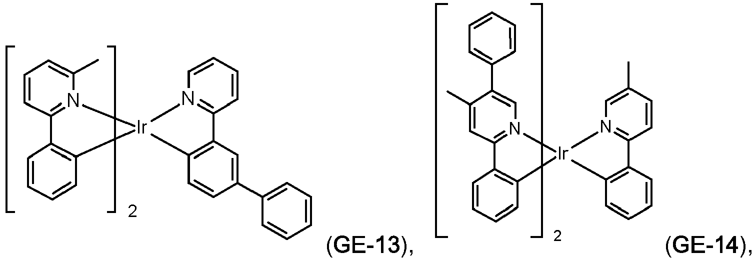

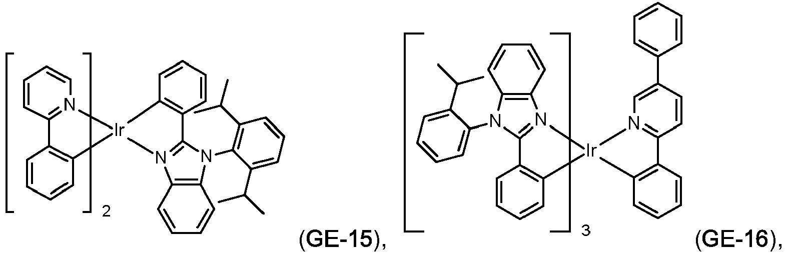

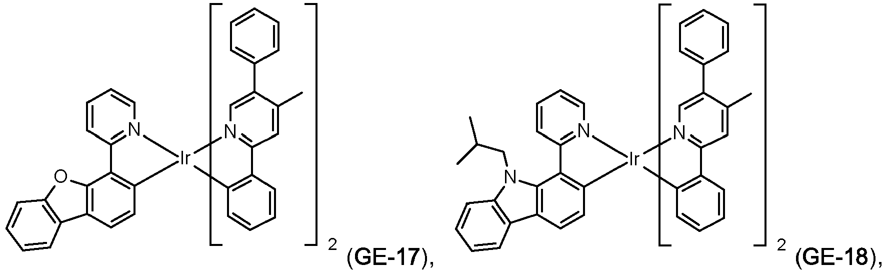

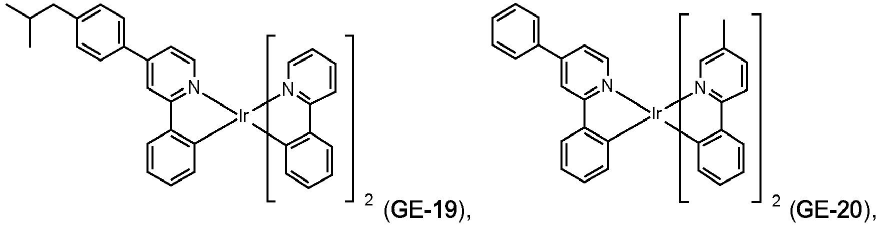

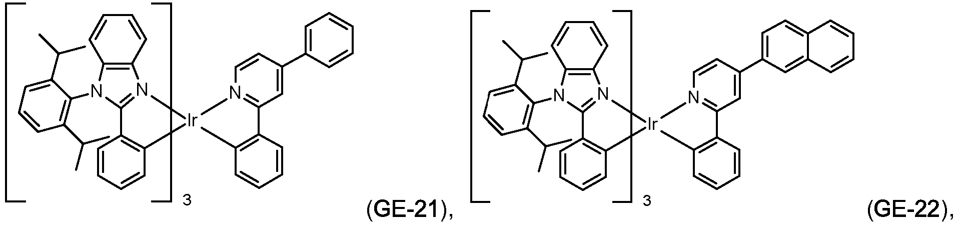

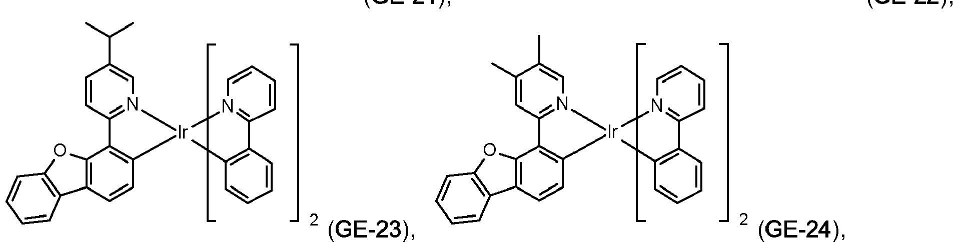

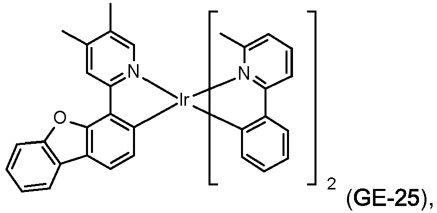

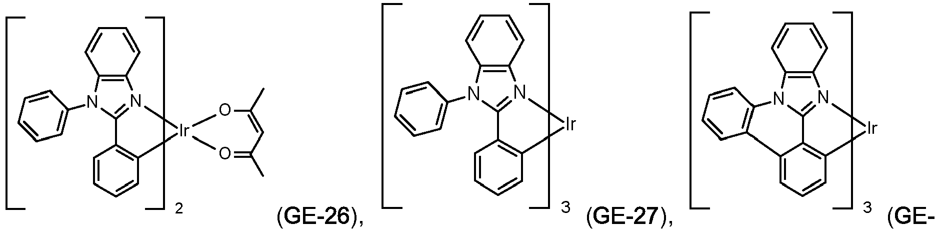

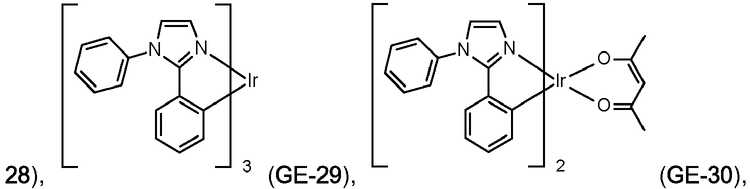

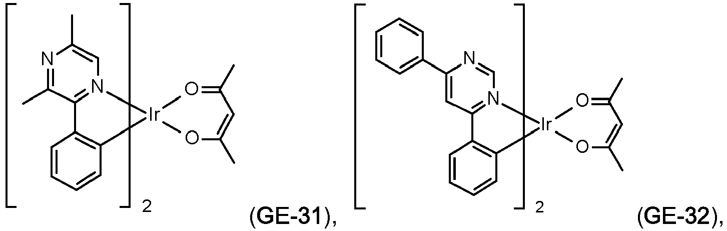

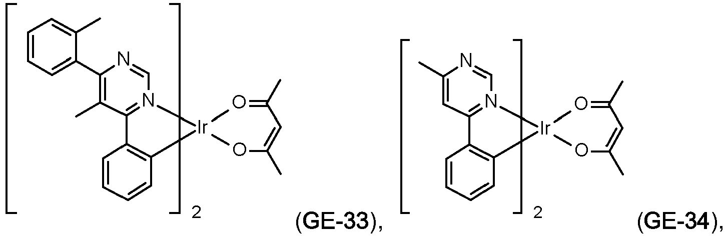

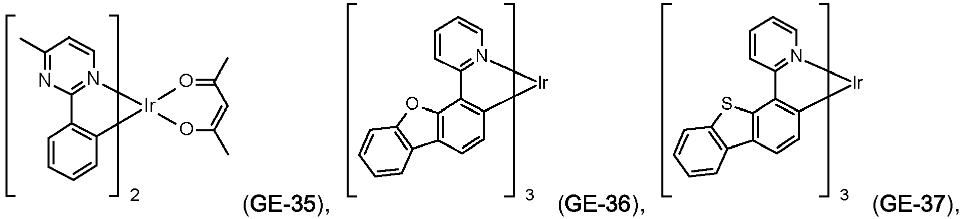

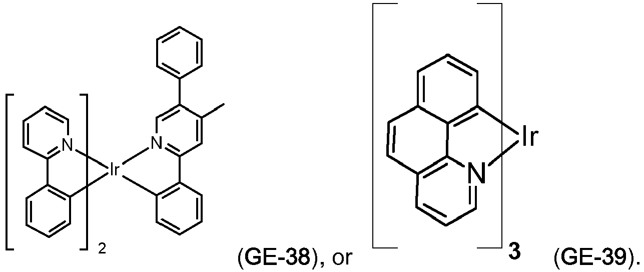

The emitter materials (dopants), preferably the phosphorescent emitter materials, may be used alone or in combination of two or more.

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass.

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In the case that one or more phosphorescent emitter materials are used in the light emitting layer, one or more phosphorescent hosts are employed as host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of at least one emitter material and of at least one of the matrix materials mentioned below- in one embodiment at least one compound of the formula (**1**) is used as matrix (host) material. In one embodiment, the light-emitting layer comprises at least one emitter material and at least two matrix materials, wherein one of the matrix materials is a compound of the formula (1) and the other matrix material(s) is/are used as co-host(s). Suitable other host materials than the compound of formula (1) (co-hosts) are mentioned below.

The compounds of the formula (**1**) are suitable as single host material as well as host material, together with one or more further host materials (co-host). Suitable further host materials are mentioned below. "Further host materials" means in the sense of the present application, host materials different from the compounds of formula (**1**). However, it is also possible to use two or more different compounds of formula (**1**) as host material in the light-emitting layer in an OLED of the present application.

In another preferred embodiment of the present invention, at least one compound of the formula (**1**) is used as host material. Examples of preferred compounds of formula (**1**) useful as host material are shown above.

In a more preferred embodiment, the light-emitting layer is formed from 0.1 to 70% by weight, preferably 1 to 30% by weight, of at least one of the aforementioned emitter materials and 30 to 99.9% by weight, preferably 70 to 99% by weight, of at least one of the matrix materials mentioned in the specification - in one embodiment at least one compound of the formula (1) - where the sum total of the emitter material and of the matrix material adds up to 100% by weight.

In a further more preferred embodiment, the light-emitting layer comprises a compound of formula (1) as matrix material, one further matrix material (co-host) and at least one emitter material. In said embodiment, the light-emitting layer is formed from 0.1 to 70% by weight, preferably 1 to 30% by weight, of the at least one emitter material and 30 to 99.9% by weight, preferably 70 to 99% by weight, of a compound of the formula (**1**) and the further matrix material, where the sum total of the at least one emitter material, the further matrix material and of the compound of formula (**1**) adds up to 100% by weight.

The content ratio of the compound of the formula (**1**) as first host material and the further matrix material as the second host material (co-host) in the light emitting layer is not particularly limited and may be selected accordingly, and the ratio of first host material:second host material is preferably 1:99 to 99:1, more preferably 10:90 to 90:10, each based on mass.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and European patent applications EP12175635.7, EP12185230.5 and EP12191408.9 (in particular page 25 to 29 of EP12191408.9).

The above-mentioned small molecules are more preferred than the above-mentioned (co)polymers of the small molecules.

Further suitable host materials, are described in WO2011137072 (for example, 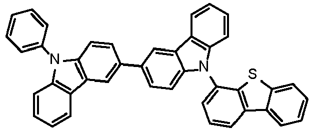 and 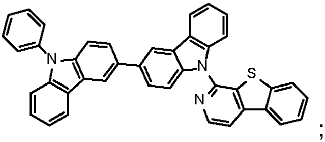 best results are achieved if said compounds are combined with 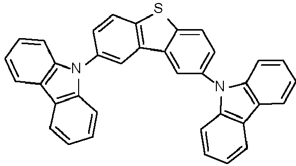 ); WO2012048266 (for example, 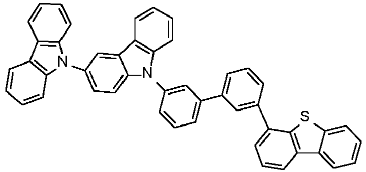 and 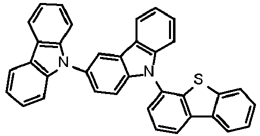 ).

The host materials mentioned above may be used in the OLED of the present invention a alone or in combination with the compound of formula (1) as host material. In this case, the compound of formula (1) is the host and the host materials mentioned above are the co-hosts.

Further examples of the compounds which are suitable as phosphorescent host, alone or in combination with the compound of formula (1) as host material, include a carbazole derivative, a triazole derivative, a oxazole derivative, an oxadiazole derivative, an imidazole derivative, a polyarylalkane derivative, a pyrazoline derivative, a pyrazolone derivative, a phenylenediamine derivative, an arylamine derivative, an amino-substituted chalcone derivative, a styrylanthracene derivative, a fluorenone derivative, a hydrazone derivative, a stilbene derivative, a silazane derivative, an aromatic tertiary amine compound, a styrylamine compound, an aromatic methylidene compound, a porphyrin compound, an anthraquinodimethane derivative, an anthrone derivative, a diphenylquinone derivative, a thiopyran dioxide derivative, a carbodiimide derivative, a fluorenylidenemethane derivative, a distyrylpyrazine derivative, a tetracarboxylic anhydride of fused ring such as naphthalene and perylene, a phthalocyanine derivative, a metal complex of 8-quinolinol derivative, metal phthalocyanine, metal complexes having a ligand such as benzoxazole and benzothiazole, an electroconductive oligomer, such as a polysilane compound, a poly(N-vinylcarbazole) derivative, an aniline copolymer, thiophene oligomer, and a polythiophene, and a polymer such as a polythiophene derivative, a polyphenylene derivative, a polyphenylenevinylene derivative, and a polyfluorene derivative. These phosphorescent hosts may be used alone or in combination of two or more. Specific examples thereof are shown below:

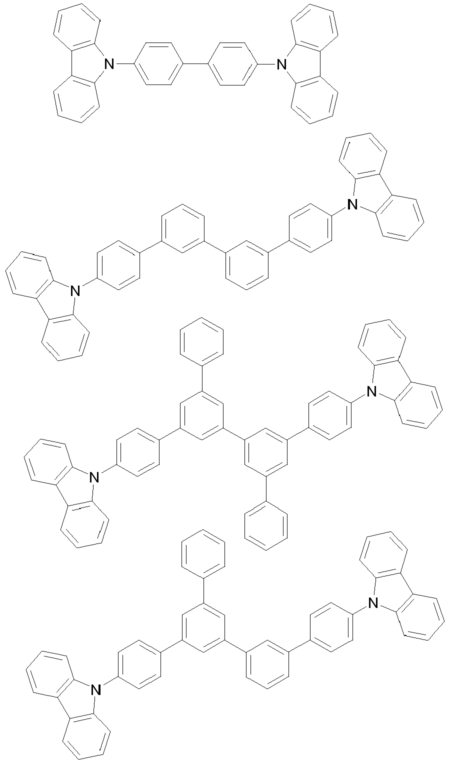

Further suitable hosts, which are especially useful as co-host together with at least one compound of formula (1) are the hosts described in US2012223295, US2014367667, US2013234119, US2014001446, US2014231794, US2014008633, WO2012108388 and WO2012108389, as well as the compounds of formula (1) described in the EP application filed at the same day as the present application, i.e. on October 1, 2015, with the title "Benzimidazolo[1,2-a]benzimidazole carrying triazine groups for Organic Light Emitting Diodes".

Especially preferred are the second host materials mentioned in US2013234119 and the compounds of formula (1) described in the EP application filed at the same day as the present application , i.e. on October 1, 2015, with the title "Benzimidazolo[1,2-a]benzimidazole carrying triazine groups for Organic Light Emitting Diodes"

Examples for suitable host materials used as co-hosts mentioned in US2013234119 WO2012108388 and WO2014009317 are:

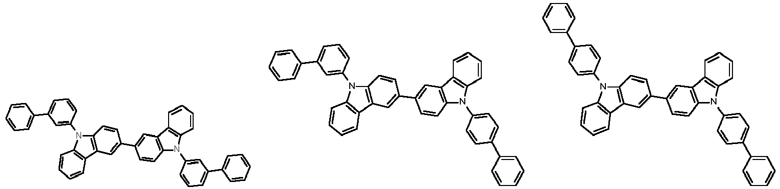

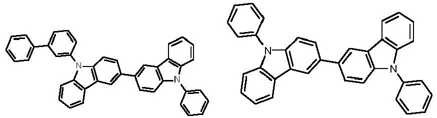

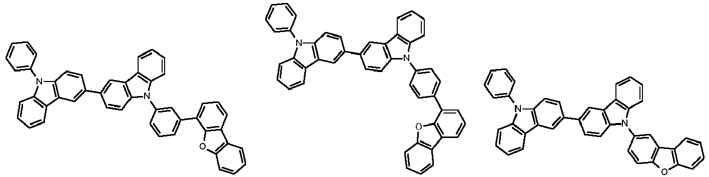

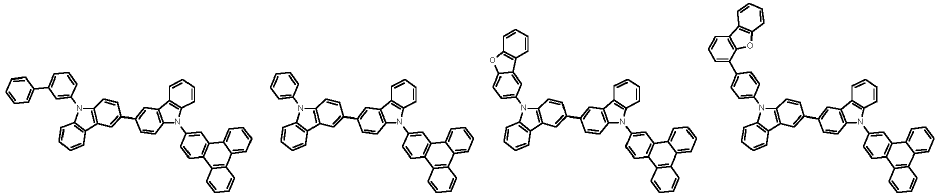

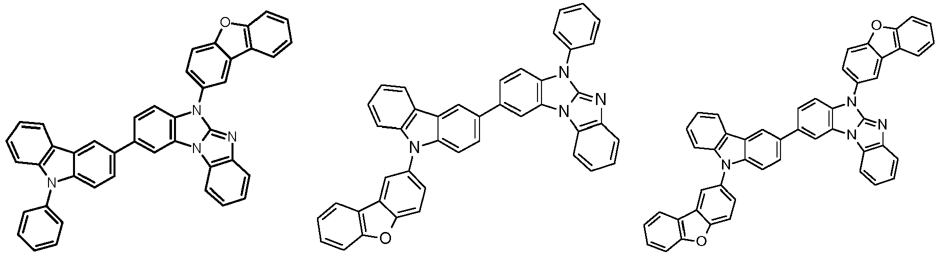

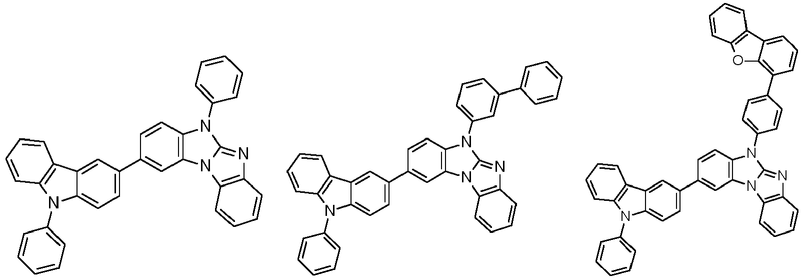

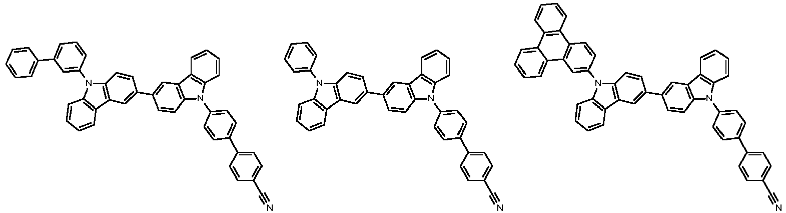

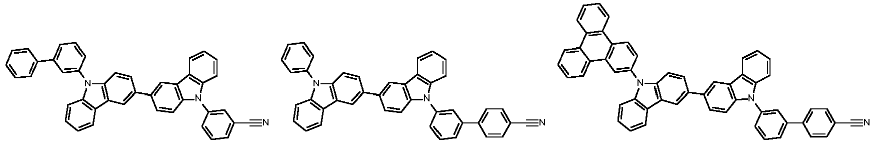

The second host material mentioned in US2013234119 which is preferably used as used co-host together with at least one compound of formula (1) in the light emitting layer of an OLED according to the present invention is represented by formula (KoH1). The lifetime of an OLED is increased by combinedly using as a first host material at least one compound of formula (1) and as co-host the second host material represented by formula (KoH1) in the light emitting layer. 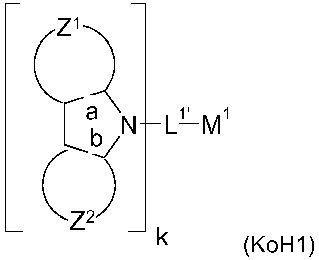
Z¹ represents a ring structure fused to the side a and represented by formula (KoH1-1) or (KoH 1-2), and
Z² represents a ring structure fused to the side b and represented by formula (KoH1-1) or (KoH1-2),
provided that at least one of Z¹ and Z² is represented by formula (KoH1-1);
M¹ represents a nitrogen-containing heteroaryl group having 5 to 30 ring atoms, which may be unsubstituted or substituted for example by G;
L^{1'} represents a single bond, a divalent aromatic hydrocarbon group having 6 to 30 ring carbon atoms which may be unsubstituted or substituted for example by G, a divalent heterocyclic group having 5 to 30 ring atoms which may be unsubstituted or substituted for example by G, a cycloalkylene group having 5 to 30 ring atoms, or a group in which the preceding groups are directly linked to each other; and
k represents 1 or 2.

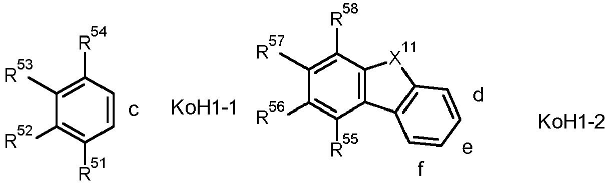

In formula (KoH1-1), a side c is fused to the side a or b of formula (KoH1).

In formula (KoH1-2), any one of sides d, e and f is fused to the side a or b of formula (KoH1).

In formulae (KoH1-1) and (KoH1-2):
X¹¹ represents a sulfur atom, an oxygen atom, NR⁷⁷, or C(R⁷⁸)(R⁷⁹); and
each of R⁵¹ to R⁵⁴ and R⁵⁵ to R⁵⁸ independently represents a hydrogen atom, a heavy hydrogen atom, a halogen atom, a cyano group, an aryl group having 6 to 30 ring carbon atoms, which may be unsubstituted or substituted for example by G, a cycloalkylene group having 5 to 30 ring atoms, a heterocyclic group having 5 to 30 ring atoms, which may be unsubstituted or substituted for example by G, an alkyl group having 1 to 30 carbon atoms, which may be unsubstituted or substituted for example by E, an alkenyl group having 2 to 30 carbon atoms, which may be unsubstituted or substituted for example by E, an alkynyl group having 2 to 30 carbon atoms, which may be unsubstituted or substituted for example by G, an alkylsilyl group having 3 to 30 carbon atoms, which may be unsubstituted or substituted for example by E, an arylsilyl group having 6 to 30 ring carbon atoms, which may be unsubstituted or substituted for example by G, an alkoxy group having 1 to 30 carbon atoms, which may be unsubstituted or substituted for example by E, an aralkyl group having 6 to 30 ring carbon atoms, which may be unsubstituted or substituted for example by G, or an aryloxy group having 6 to 30 ring carbon atoms, which may be unsubstituted or substituted for example by G, provided that adjacent groups of R⁵¹ to R⁵⁴ and R⁵⁵ to R⁵⁸ may be bonded to each other to form a ring;
R⁷⁷ is a C₆-C₂₄aryl group, which can optionally be substituted by G, or a C₁-C₂₄heteroaryl group, which can optionally be substituted by G;
R⁷⁸, R⁷⁹ is a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen. E is preferably -OR⁶⁹; -SR⁶⁹; -NR⁶⁵R⁶⁶; -COR⁶⁸; -COOR⁶⁷; -CON⁶⁵R⁶⁶; or -CN; wherein R⁶⁵, R⁶⁶, R⁶⁷, R⁶⁸ and R⁶⁹ are preferably independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, triphenylyl or biphenylyl;
G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆oheteroaryl group, or a C₂-C₆₀heteroaryl group, which is substituted by F, C₁-C₁₈alkyl, or C₁-C₁₈alkyl which is interrupted by O. G is preferably - OR69, -SR⁶⁹, -NR⁶⁵R⁶⁶; a C₁-C₁₈alkyl group, a C₆-C₁₈aryl group, a C₆-C₁₈aryl group, which is substituted by F, or C₁-C₁₈alkyl; a C₂-C₂₄heteroaryl group, or a C₂-C₂₄heteroaryl group, which is substituted by F, or C₁-C₁₈alkyl; wherein R⁶⁵, R⁶⁶ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, G is a C₆-C₁₈aryl group like phenyl, tolyl, triphenylyl or biphenylyl, or a C₆-C₂₄heteroaryl group like dibenzothiophenylyl, dibenzofuranyl, pyridyl, triazinyl, pyrimidinyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl or dibenzo[f,h]quinazolinyl.

The nitrogen-containing heteroaryl group represented by M¹ of formula (KoH1) includes azine rings. Examples of the nitrogen-containing heteroaryl group include pyridine, pyrimidine, pyrazine, triazine, aziridine, azaindolizine, indolizine, imidazole, indole, isoindole, indazole, purine, pteridine, β-carboline, naphthyridine, quinoxaline, terpyridine, bipyridine, acridine, phenanthroline, phenazine, and imidazopyridine, with pyridine, pyrimidine, and triazine being particularly preferred.

The formula (KoH1) is preferably represented by formula (KoH2): 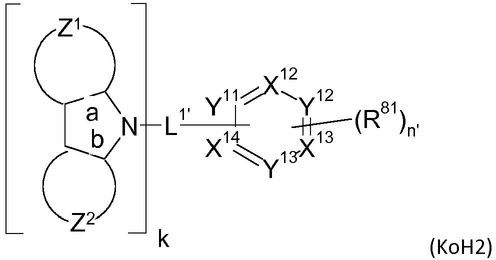 wherein
Z¹ represents a ring structure fused to the side a and represented by formula (KoH1-1) or (KoH1-2), and
Z² represents a ring structure fused to the side b and represented by formula (KoH1-1) or (KoH1-2),
provided that at least one of Z¹ and Z² is represented by formula (KoH1-1);
L^{1'} is as defined in formula (KoH1);
each of X¹² to X¹⁴ independently represents a nitrogen atom, CH, or a carbon atom bonded to R⁸¹ or L^{1'},
provided that at least one of X¹² to X¹⁴ represents a nitrogen atom;
each Y¹¹ to Y¹³ independently represents CH or a carbon atom bonded to R⁸¹ or L¹,
each of R⁸¹ independently represents a halogen atom, a cyano group, an aryl group having 6 to 30 ring carbon atoms, which may be unsubstituted or substituted for example by G, a heterocyclic group having 5 to 30 ring atoms, which may be unsubstituted or substituted for example by G, an alkyl group having 1 to 30 carbon atoms, which may be unsubstituted or substituted for example by E, an alkenyl group having 2 to 30 carbon atoms, which may be unsubstituted or substituted for example by E, an alkynyl group having 2 to 30 carbon atoms, which may be unsubstituted or substituted for example by E, an alkylsilyl group having 3 to 30 carbon atoms, which may be unsubstituted or substituted for example by E, an arylsilyl group having 6 to 30 ring carbon atoms, which may be unsubstituted or substituted for example by G, an alkoxy group having 1 to 30 carbon atoms, which may be unsubstituted or substituted for example by E, an aralkyl group having 6 to 30 ring carbon atoms, which may be unsubstituted or substituted for example by G, or an aryloxy group having 6 to 30 ring carbon atoms, which may be unsubstituted or substituted for example by G;
when two or more R⁸¹ groups exist, the R⁸¹ groups may be the same or different and adjacent R⁸¹ groups may be bonded to each other to form a ring, k represents 1 or 2, and n' represents an integer of 0 to 4;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen. E is preferably -OR⁶⁹; -SR⁶⁹; -NR⁶⁵R⁶⁶; -COR⁶⁸; -COOR⁶⁷; -CON⁶⁵R⁶⁶; or -CN; wherein R⁶⁵, R⁶⁶, R⁶⁷, R⁶⁸ and R⁶⁹ are preferably independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, triphenylyl or biphenylyl;
G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆oheteroaryl group, or a C₂-C₆₀heteroaryl group, which is substituted by F, C₁-C₁₈alkyl, or C₁-C₁₈alkyl which is interrupted by O. G is preferably - OR69, -SR⁶⁹, -NR⁶⁵R⁶⁶; a C₁-C₁₈alkyl group, a C₆-C₁₈aryl group, a C₆-C₁₈aryl group, which is substituted by F, or C₁-C₁₈alkyl; a C₂-C₂₄heteroaryl group, or a C₂-C₂₄heteroaryl group, which is substituted by F, or C₁-C₁₈alkyl; wherein R⁶⁵, R⁶⁶ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, G is a C₆-C₁₈aryl group like phenyl, tolyl, triphenylyl or biphenylyl, or a C₆-C₂₄heteroaryl group like dibenzothiophenylyl, dibenzofuranyl, pyridyl, triazinyl, pyrimidinyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl or dibenzo[f,h]quinazolinyl;
the side c of formula (KoH1-1) is fused to the side a or b of formula (KoH-2); and
any one of sides d, e and f of formula (KoH1-2) is fused to the side a or b of formula (KoH-2).

Examples of the compound wherein the ring represented by formula (KoH1-1) or (KoH1-2) is fused to the side a or b of formula (KoH2) are shown below

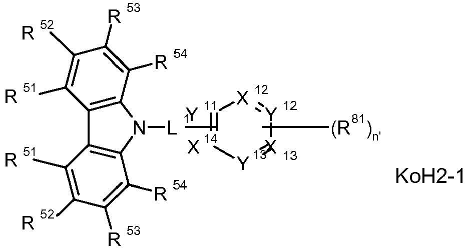

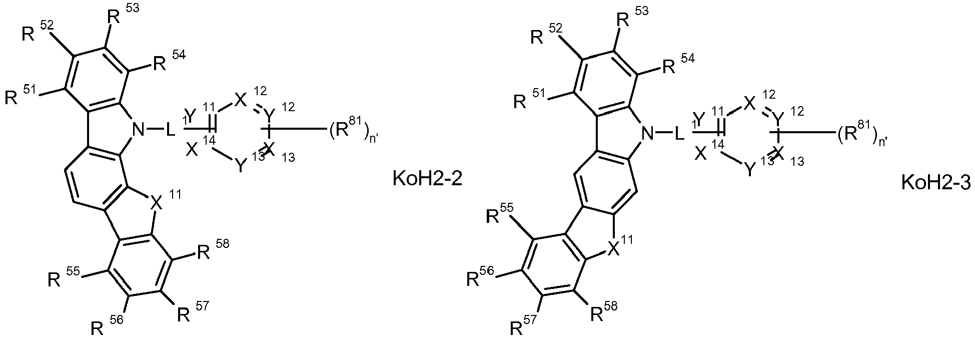

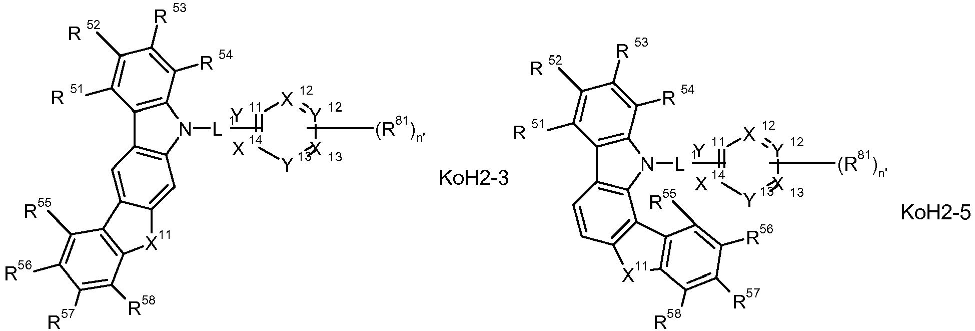

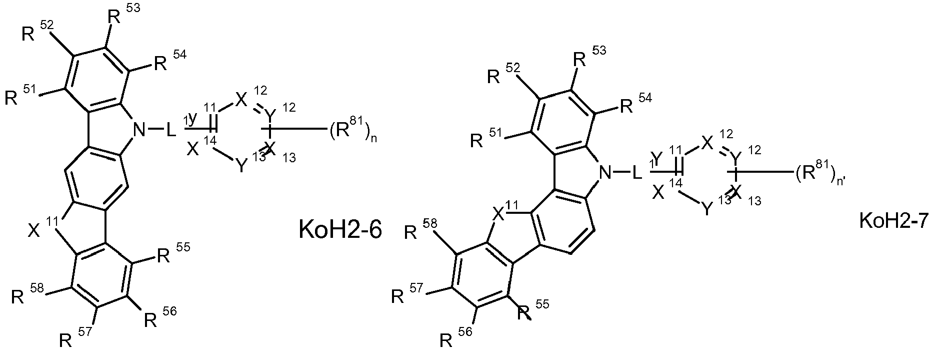

The compound represented by formula (KoH1) or (KoH2) is more preferably represented by formula (KoH3) and particularly preferably represented by formula (KoH4). 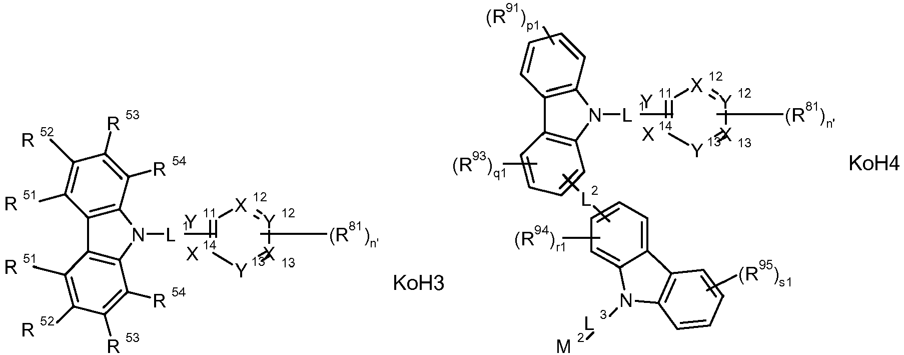 In formula (KoH3),
L^{1'} is as defined in formula (KoH1);
each of X¹² to X¹⁴ independently represents a nitrogen atom, CH, or a carbon atom bonded to R⁸¹ or L^{1'},
provided that at least one of X¹² to X¹⁴ represents a nitrogen atom;
each of Y¹¹ to Y¹³ independently represents CH or a carbon atom bonded to R⁸¹ or L¹,
each of R⁸¹ independently represents a halogen atom, a cyano group, an aryl group having 6 to 30 ring carbon atoms, which may be unsubstituted or substituted for example by G, a heterocyclic group having 5 to 30 ring atoms, which may be unsubstituted or substituted for example by G, an alkyl group having 1 to 30 carbon atoms, which may be unsubstituted or substituted for example by E, an alkenyl group having 2 to 30 carbon atoms, which may be unsubstituted or substituted for example by E, an alkynyl group having 2 to 30 carbon atoms, which may be unsubstituted or substituted for example by E, an alkylsilyl group having 3 to 30 carbon atoms, which may be unsubstituted or substituted for example by E, an arylsilyl group having 6 to 30 ring carbon atoms, which may be unsubstituted or substituted for example by G, an alkoxy group having 1 to 30 carbon atoms, which may be unsubstituted or substituted for example by E, an aralkyl group having 6 to 30 ring carbon atoms, which may be unsubstituted or substituted for example by G, or an aryloxy group having 6 to 30ring carbon atoms, which may be unsubstituted or substituted for example by G;
when two or more R⁸¹ groups exist, the R⁸¹ groups may be the same or different and adjacent R⁸¹ groups may be bonded to each other to form a ring;
n' represents an integer of 0 to 4;
each of R⁵¹ to R⁵⁸ independently represents a hydrogen atom, a heavy hydrogen atom, a halogen atom, a cyano group, an aryl group having 6 to 30 ring carbon atoms, which may be unsubstituted or substituted for example by G, a heterocyclic group having 5 to 30 ring atoms, which may be unsubstituted or substituted for example by G, an alkyl group having 1 to 30 carbon atoms, which may be unsubstituted or substituted for example by E, an alkenyl group having 2 to 30 carbon atoms, which may be unsubstituted or substituted for example by E, an alkynyl group having 2 to 30 carbon atoms, which may be unsubstituted or substituted for example by E, an alkylsilyl group having 3 to 30 carbon atoms, which may be unsubstituted or substituted for example by E, an arylsilyl group having 6 to 30 ring carbon atoms, which may be unsubstituted or substituted for example by G, an alkoxy group having 1 to 30 carbon atoms, which may be unsubstituted or substituted for example by E, an aralkyl group having 6 to 30 ring carbon atoms, which may be unsubstituted or substituted for example by G, or an aryloxy group having 6 to 30 ring carbon atoms, which may be unsubstituted or substituted for example by G;
and adjacent groups of R⁵¹ to R⁵⁸ may be bonded to each other to form a ring;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen. E is preferably -OR⁶⁹; -SR⁶⁹; -NR⁶⁵R⁶⁶; -COR⁶⁸; -COOR⁶⁷; -CON⁶⁵R⁶⁶; or -CN; wherein R⁶⁵, R⁶⁶, R⁶⁷, R⁶⁸ and R⁶⁹ are preferably independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, triphenylyl or biphenylyl;
G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆oheteroaryl group, or a C₂-C₆₀heteroaryl group, which is substituted by F, C₁-C₁₈alkyl, or C₁-C₁₈alkyl which is interrupted by O. G is preferably - OR69, -SR⁶⁹, -NR⁶⁵R⁶⁶; a C₁-C₁₈alkyl group, a C₆-C₁₈aryl group, a C₆-C₁₈aryl group, which is substituted by F, or C₁-C₁₈alkyl; a C₂-C₂₄heteroaryl group, or a C₂-C₂₄heteroaryl group, which is substituted by F, or C₁-C₁₈alkyl; wherein R⁶⁵, R⁶⁶ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, G is a C₆-C₁₈aryl group like phenyl, tolyl, triphenylyl or biphenylyl, or a C₆-C₂₄heteroaryl group like dibenzothiophenylyl, dibenzofuranyl, pyridyl, triazinyl, pyrimidinyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl or dibenzo[f,h]quinazolinyl.

In formula (KoH4),
L^{1'} is as defined in formula (KoH-1);
each of X¹² to X¹⁴ independently represents a nitrogen atom, CH, or a carbon atom bonded to R⁸¹ or L^{1'},
provided that at least one of X¹² to X¹⁴ represents a nitrogen atom;
each of Y¹¹ to Y¹³ independently represents CH or a carbon atom bonded to R⁸¹ or L¹,
each of R⁸¹ independently represents a halogen atom, a cyano group, an aryl group having 6 to 30 ring carbon atoms, which may be unsubstituted or substituted for example by G, a heterocyclic group having 5 to 30 ring atoms, which may be unsubstituted or substituted for example by G, an alkyl group having 1 to 30 carbon atoms, which may be unsubstituted or substituted for example by E, an alkenyl group having 2 to 30 carbon atoms, which may be unsubstituted or substituted for example by E, an alkynyl group having 2 to 30 carbon atoms, which may be unsubstituted or substituted for example by E, an alkylsilyl group having 3 to 30 carbon atoms, which may be unsubstituted or substituted for example by E, an arylsilyl group having 6 to 30 ring carbon atoms, which may be unsubstituted or substituted for example by G, an alkoxy group having 1 to 30 carbon atoms, which may be unsubstituted or substituted for example by E, an aralkyl group having 6 to 30 ring carbon atoms, which may be unsubstituted or substituted for example by G, or an aryloxy group having 6 to 30 ring carbon atoms, which may be unsubstituted or substituted for example by G;
adjacent R⁸¹ groups may be bonded to each other to form a ring;
n' represents an integer of 0 to 4;
each of L^{1'}, L² and L³ independently represents a single bond, a divalent aromatic hydrocarbon group having 6 to 30 ring carbon atoms, which may be unsubstituted or substituted for example by G, a divalent heterocyclic group having 5 to 30 ring atoms, which may be unsubstituted or substituted for example by G, a cycloalkylene group having 5 to 30 ring atoms, or a group in which the preceding groups are directly linked to each other;
each of R⁹¹ to R⁹⁵ independently represents a halogen atom, a cyano group, an aryl group having 6 to 30 ring carbon atoms, which may be unsubstituted or substituted for example by G, a heterocyclic group having 5 to 30 ring atoms, which may be unsubstituted or substituted for example by G, an alkyl group having 1 to 30 carbon atoms, which may be unsubstituted or substituted for example by E, an alkenyl group having 2 to 30 carbon atoms, which may be unsubstituted or substituted for example by E, an alkynyl group having 2 to 30 carbon atoms, which may be unsubstituted or substituted for example by E, an alkylsilyl group having 3 to 30 carbon atoms, which may be unsubstituted or substituted for example by E, an arylsilyl group having 6 to 30 ring carbon atoms, which may be unsubstituted or substituted for example by G, an alkoxy group having 1 to 30 carbon atoms, which may be unsubstituted or substituted for example by E, an aralkyl group having 6 to 30 ring carbon atoms, which may be unsubstituted or substituted for example by G, or an aryloxy group having 6 to 30 ring carbon atoms, which may be unsubstituted or substituted for example by G;
when two or more R⁹¹ to R⁹⁵ groups exist, the R⁹¹ to R⁹⁵ groups may be the same or different and adjacent R⁹¹ to R⁹⁵ groups may be bonded to each other to form a ring;
M² represents an aryl group having 6 to 30 ring carbon atoms, which may be unsubstituted or substituted for example by G or a heterocyclic group having 5 to 30 ring atoms, which may be unsubstituted or substituted for example by G; and
each of p1, q1, r1, and s1 independently represents an integer of 0 to 4, and
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen. E is preferably -OR⁶⁹; -SR⁶⁹; -NR⁶⁵R⁶⁶; -COR⁶⁸; -COOR⁶⁷; -CON⁶⁵R⁶⁶; or -CN; wherein R⁶⁵, R⁶⁶, R⁶⁷, R⁶⁸ and R⁶⁹ are preferably independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, triphenylyl or biphenylyl;
G is E, or a C₁-C₂₄alkyl group, a C₆-C₃₀aryl group, a C₆-C₃₀aryl group, which is substituted by F, C₁-C₂₄alkyl, or C₁-C₂₄alkyl which is interrupted by O; a C₂-C₆oheteroaryl group, or a C₂-C₆₀heteroaryl group, which is substituted by F, C₁-C₁₈alkyl, or C₁-C₁₈alkyl which is interrupted by O. G is preferably - OR69, -SR⁶⁹, -NR⁶⁵R⁶⁶; a C₁-C₁₈alkyl group, a C₆-C₁₈aryl group, a C₆-C₁₈aryl group, which is substituted by F, or C₁-C₁₈alkyl; a C₂-C₂₄heteroaryl group, or a C₂-C₂₄heteroaryl group, which is substituted by F, or C₁-C₁₈alkyl; wherein R⁶⁵, R⁶⁶ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, G is a C₆-C₁₈aryl group like phenyl, tolyl, triphenylyl or biphenylyl, or a C₆-C₂₄heteroaryl group like dibenzothiophenylyl, dibenzofuranyl, pyridyl, triazinyl, pyrimidinyl, azatriphenylyl, azadibenzofuryl, azadibenzothiophenyl, azacarbazolyl, quinolonyl, isoquinolinyl, quinoxalinyl, quinazolinyl, phenanthrolinyl, phenanthridinyl, benzo[h]quinolonyl, benz[h]isoquinolinyl, benzo[f]isoquinolinyl, benzo[f]quinolinyl, benzo[h]quinazolinyl, benzo[f]quinazolinyl, dibenzo[f,h]quinolonyl, dibenzo[f,h]isoquinolonyl, dibenzo[f,h]quinoxalinyl or dibenzo[f,h]quinazolinyl.

In formulae (KoH1) to (KoH4), (KoH1-1), and (KoH1-2), the groups represented by R⁵¹ to R⁵⁸, R⁹¹ to R⁹⁵, R⁷⁷ to R⁷⁹ are as defined above with respect to formula (KoH1).

Examples for preferred second host materials used as co-hosts mentioned in US2013234119 are:

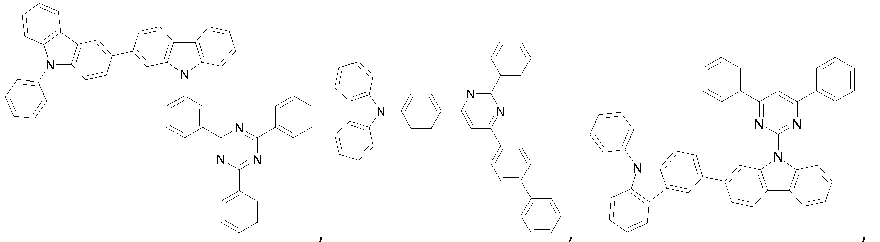

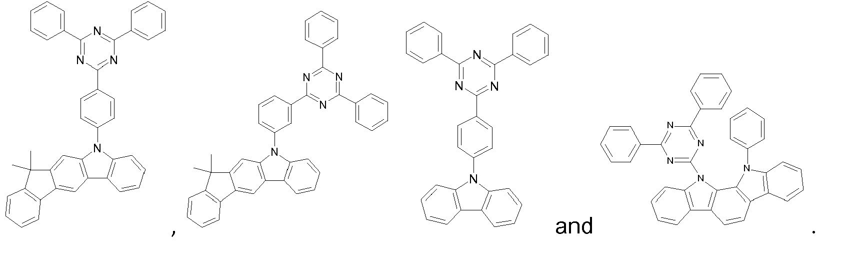

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAIq), phenothiazine S,S-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH**-**1**), (**SH**-**2**), (**SH**-**3**), **SH-4, SH-5, SH-6,** (**SH-7**), (**SH**-**8**), (**SH**-**9**), (**SH**-**10**) and (**SH**-**11**) may be used as hole/exciton blocking materials.

### Electron transport layer (g):

Electron transport layer may include a material capable of transporting electrons. Electron transport layer may be intrinsic (undoped), or doped. Doping may be used to enhance conductivity. Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

Further suitable electron transport materials are mentioned in Abhishek P. Kulkarni, Christopher J. Tonzola, Amit Babel, and Samson A. Jenekhe, Chem. Mater. 2004, 16, 4556-4573; G. Hughes, M. R. Bryce, J. Mater. Chem. 2005, 15, 94-107 and Yasuhiko Shirota and Hiroshi Kageyama, Chem. Rev. 2007, 107, 953-1010 *(ETM, HTM).*

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**XVI**) below, preferably a compound of the formula (**XVIa**) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (**formula XVII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**XVII**) 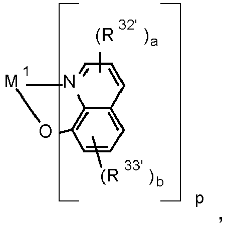 in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**XVII**) is 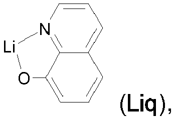 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**XVI**), 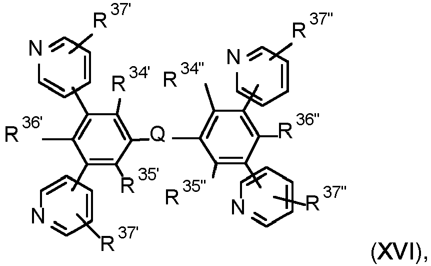 in which
R^{34"}, R^{35"}, R^{36"}, R^{37"}, R^{34'}, R^{35'}, R^{36'} and R^{37'} are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-;
E' is -OR^{44'}; -SR^{44'}; -NR^{40'}R^{41'}; -COR^{43'}; -COOR^{42'}; -CONR^{40'}R^{41'}; -CN; or F;
G' is E', C₁-C₁₈-alkyl, C₁-C₁₈-alkyl which is interrupted by D', C₁-C₁₈-perfluoroalkyl, C₁-C₁₈-alkoxy, or C₁-C₁₈-alkoxy which is substituted by E' and/or interrupted by D', in which R^{38'} and R^{39'} are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R^{40'} and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula (**XVI**) are compounds of the formula (**XVIa**) 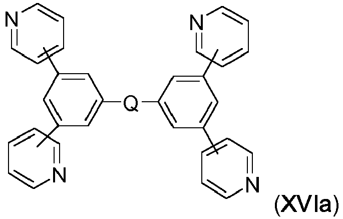
in which Q is: 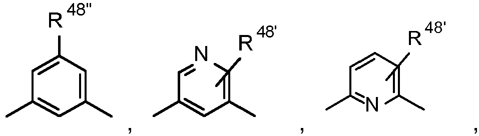 or 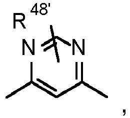
R^{48'} is H or C₁-C₁₈-alkyl and
R^{48"} is H, C₁-C₁₈-alkyl or 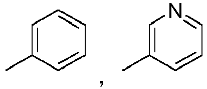 or

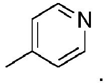

Particular preference is given to a compound of the formula

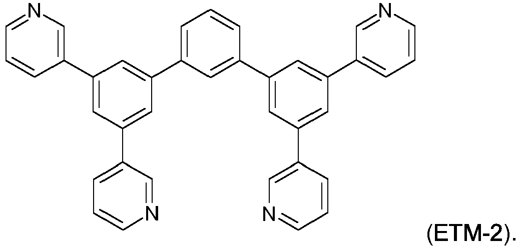

In a further, very particularly preferred embodiment, the electron-transport layer comprises a compound **Liq** and a compound **ETM-2.**

In a preferred embodiment, the electron-transport layer comprises at least one compound of the formula (**XVII**) in an amount of 99 to 1% by weight, preferably 75 to 25% by weight, more preferably about 50% by weight, and at least one compound of the formula (**XVI**) in an amount of 1 to 99% by weight, preferably 25 to 75% by weight, more preferably about 50% by weight, where the amount of the compounds of the formulae (**XVII**) and the amount of the compounds of the formulae (**XVI**) adds up to a total of 100% by weight.

The preparation of the compounds of the formula (**XVI**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 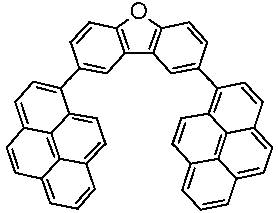 (**A-10; = ETM-1**) is most preferred.

In a preferred embodiment, the electron-transport layer comprises **Liq** in an amount of 99 to 1% by weight, preferably 75 to 25% by weight, more preferably about 50% by weight, and at least one dibenzofuran compound in an amount of 1 to 99% by weight, preferably 25 to 75% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 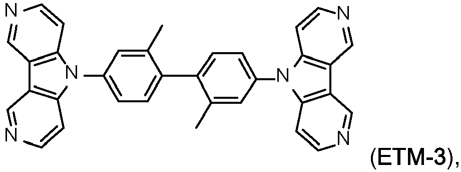 US2012/0261654 , such as, for example, a compound of formula 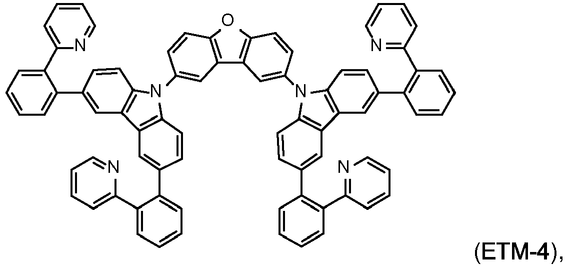 and WO2012/115034, such as for example, such as, for example, a compound of formula

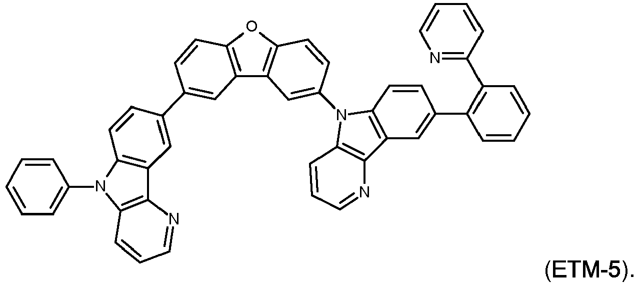

A further suitable electron transport material is:

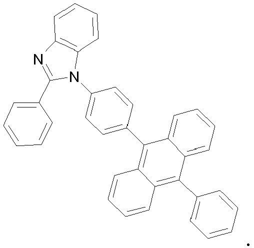

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula (**1**) in at least one layer of the OLED, preferably in the light-emitting layer (preferably as a matrix materialhole transport layer and/or in the electron/exciton blocking layer makes it possible to obtain OLEDs with high efficiency and with low use and operating voltage. Frequently, the OLEDs obtained by the use of the compounds of the formula (**1**) additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### I Preparation Example

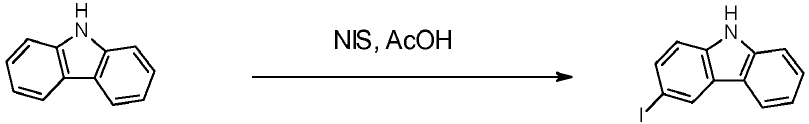

a) 76.9 g (0.460 mol) carbazole and 104 g (0.460 mol) 1-iodopyrrolidine-2,5-dione (NIS) in 100m ml acetic acid are stirred under nitrogen at 20 °C. After 5 h the product is filtered off. The product is crystalized from 900 ml ethanol using 2 g charcoal. The ethanol solution is filtered hot. The ethanol solution is cooled to 20 °C and the product is filtered off (yield: 59.5 g (44 %)). 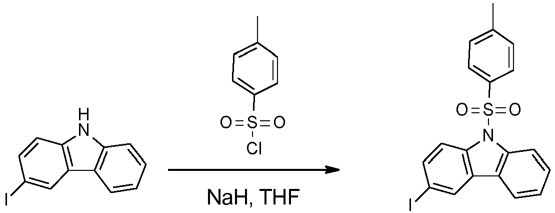
b) 19.7 g (67.0 mmol) 3-iodo-9H-carbazole and 2.95 g (73.7 mmol) sodium hydride 60 % dispersion in mineral oil in 500 ml tetrahydrofuran (THF) are stirred at 50 °C under nitrogen for 1h. 12.8 g (67.0 mmol) 4-methylbenzenesulfonyl chloride in 100 ml THF are added at 20 °C.
   The reaction mixture is stirred for 1 h at 20 °C and is then stirred for 1 h at 50 °C. The solution is filtered and the solvent is distilled off. 200 ml ethyl acetate are added and the organic phase is washed with a solution of citric acid, sodium hydrogen carbonate and water. The solvent is partly removed until the product starts to crystalize. The product is filtered off and washed with methanol (yield: 23 g (79 %)). 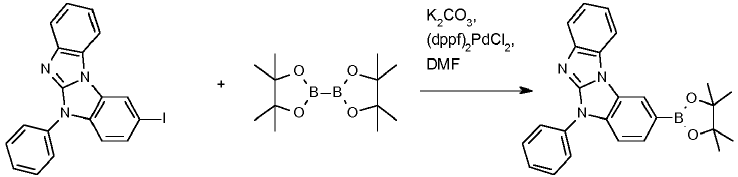
c) 2-iodo-5-phenyl-benzimidazolo[1,2-a]benzimidazole is prepared as describe in WO 2014/009317
   3.00 g (7.33 mmol) 2-iodo-5-phenyl-benzimidazolo[1,2-a]benzimidazole, 2.88 g (29.3 mmol) potassium acetate and 2.23 (8.80 mmol) 4,4,5,5-tetramethyl-2-(4,4,5,5 -tetramethyl-1,3,2-dioxaborolan- 2-yl)-1,3,2- dioxaborolane are degassed 3 times with argon The water free DMF is added and the reaction mixture is degassed 7 times with argon under stirring and at 25 °C. The catalyst is added and the reaction mixture is degassed 2 times with argon at 25 °C. Reaction mixture is stirred at 65 °C under argon for 18 h.
   The reaction mixture is cooled to 35 °C 5 ml diethyl ether and 400mg NaCN in 2 ml water is added simultaneously. The reaction mixture is stirred 10 min. The reaction mixture is poured in dichloromethane containing 20 % diethyl ether. The organic phase is washed with water and dried with magnesium sulfate and filtered on Hyflo. The solvent is removed in vacuum. Yield 2.93 g (97.7 %) 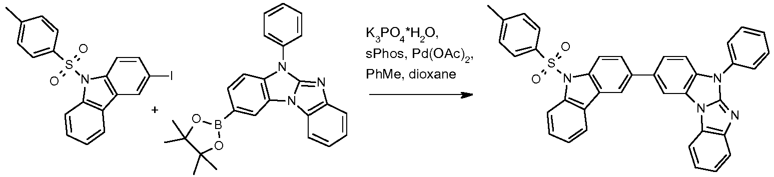
d) To 7.75 g (17.3 mmol) 3-iodo-9-(p-tolylsulfonyl)carbazole, 7.80 (19.1 mmol) 5-phenyl-2-(4,4,5,5-tetramethyl-1,3,2-dioxaborolan-2-yl)benzimidazolo[1,2-a] benzimidazole, 18.4 g (86.6 mmol) potassium phosphate tribasic monohydrate, 25 ml dioxane, 60 ml toluene and 25 ml water are added.
   The mixture is degassed with argon. 426 mg (0.250 mmol) 2-dicyclohexylphosphino-2',6'-dimethoxybiphenyl (SPhos) and 39 mg (0.17 mmol) palladium(II) acetate are added. The reaction mixture is degassed with argon and is stirred for 16 h at 90 °C under argon. 30 ml of a 1 % sodium cyanide solution are added and the reaction mixture is refluxed for 2 h. Toluene is added and organic phase is separated. The organic phase is dried with magnesium sulfate. The solvent is removed in vacuum. The product is crystalized from diethyl ether. 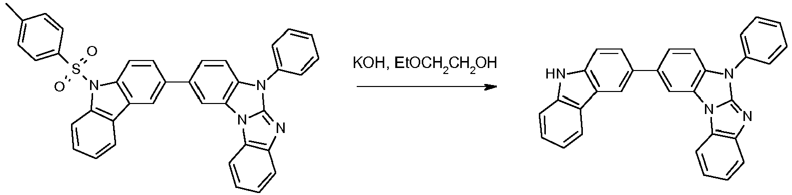
e) 9.30 g (15.4 mmol) 5-phenyl-2-[9-(p-tolylsulfonyl)carbazol-3-yl]benzimidazolo [1,2-a]benzimidazole and 2.14 g (32.4 mmol) potassium hydroxide in 200 ml 2-ethoxyethanol is refluxed for 2 h. The solvent is removed in vacuum. The product is decocted in ethanol and the product is filtered of. 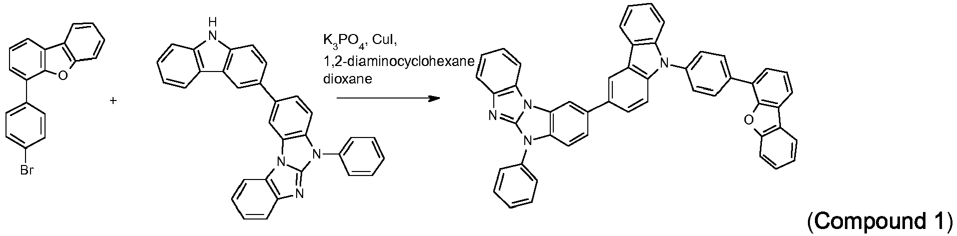
f) 500 mg (1.55 mmol) 4-(4-bromophenyl)dibenzofuran, 760 mg (1.70 mmol) 2-(9H-carbazol-3-yl)-5-phenyl-benzimidazolo[1,2-a]benzimidazole, 990 mg (4.64 mmol) potassium phosphate tribasic, 60 mg (0.31 mmol) cupper iodide in 10 ml dioxane are stirred under nitrogen at 90 °C. 1.24 g (36.1 mmol) cis,trans 1,2-diaminocyclohexane are added. The reaction mixture is stirred for 16 h. The reaction temperature is increased to 100 °C. The reaction mixture is stirred for 48 h at 100 °C under nitrogen.

The reaction mixture is poured in 40 ml methanol. The product is filtered of and is washed with water, methanol 10 % tatraic acid. Column chromatography on silica gel with chloroform give the product. Yield 780 mg (73 %)
**¹H NMR** (400 MHz, THF-d8): δ = 8.67 (d, 1H), 8.43 (d, 1H), 8.31-8.36 (m, 3H), 8.20-8.23 (m,1H), 8.11-8.16 (m, 2H), 8.05-8.08 (m, 2H), 7.32-7.95 (m, 20 H).

### II Application Examples

### 1. Comparative Application Example 1C

A glass substrate with 120 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As a hole injection layer, 40 nm-thick of compound A is applied. Then 20 nm-thick of compound B is applied as a hole transporting layer. Subsequently, a mixture of 20% by weight of an emitter compound, (Ir(Ph-ppy)₃), 40% by weight of a 1^{st} host (compound C) and 40% by weight of a 2^{nd} host (Comparative compound 1C) are applied to form a 40 nm-thick phosphorescent-emitting layer. On the emitting layer, 30 nm-thick compound D is applied as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

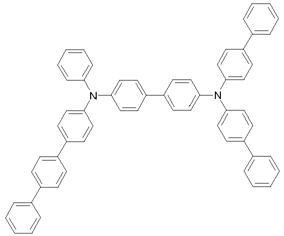

Compound A (described in WO2006073054, page 82 and US2012112176, H24, page 87)

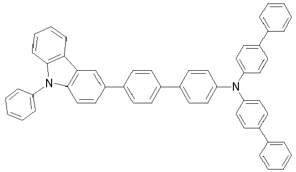

Compound B (described in WO09072587, page181)

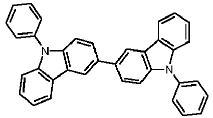

Compound C (described in US 2014/0197386, page 44)

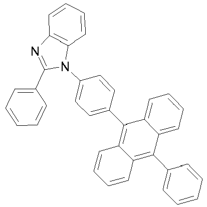

Compound D (described in US2009009067, page 101 and US 20090009065, page 79)

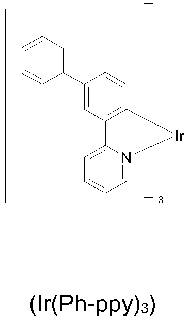

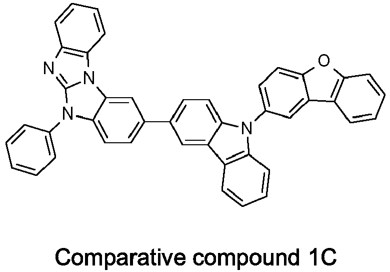

### Application Example 1

Comparative Application Example 1 is repeated except that the 2^{nd} host (Comparative compound 1C) is replaced by inventive Compound 1. The device results are shown in Table 1.

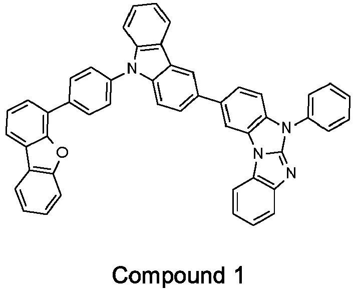

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U, EQE and Commission Internationale de l'Éclairage (CIE) coordinate are given at 10mA/cm² except otherwise stated.

**Table 1**

| **Appl. Ex.** | **2^{nd} Host** | **LT80 [hrs]** |
|---|---|---|
| **Comparative Appl. Ex. 1** | **Comparative compound 1C** | **90** |
| **Appl. Ex. 1** | **Compound 1** | **140** |

The results shown in Table 1 demonstrate that the lifetime is prolonged in an OLED comprising the inventive compound of formula (**1**).

## Claims

1. A heterocyclic derivative of formula (**1**);
**A-[(B₁)ₒ-(B₂)ₚ-(B₃)_{q}-(B₄)ᵣ-Z]_{z}** **(1)**
wherein
B₁, B₂, B₃ and B₄
are independently of each other a C₆-C₂₄arylene group, which can optionally be substituted by G, or a 6 membered heteroarylene group, which can optionally be substituted by G; o is 0 or 1, p is 0 or 1, q is 0 or 1, r is 0 or 1, wherein at least one of o, p, q and r is 1;
Z represents
a dibenzofuran group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G; or
a dibenzothiophene group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
and/or
two adjacent substituents of the dibenzofuran group or of the dibenzothiophene group may form together with the atoms to which they are bonded a ring structure, which can optionally be substituted by G;
A is a heterocyclic group represented by formula (2) or formula (3); 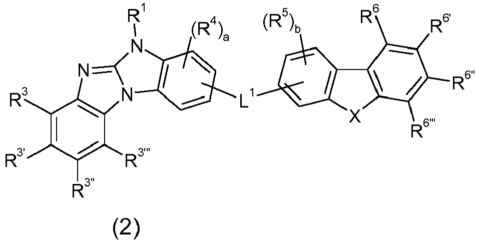 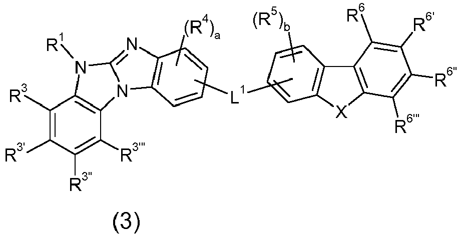
wherein X is NR⁷;
L¹ is single bond, a C₆-C₂₄arylene group, which can optionally be substituted by G, or a C₁-C₂₄heterocyclic group, which can optionally be substituted by G;
R¹, R³, R^{3'}, R^{3"}, R^{3'''}, R⁴, R⁵, R⁶, R^{6'}, R^{6"} and R^{6'''}, are independently of each other H or a group of formula -(B⁵)ₛ-(B⁶)ₜ-(B⁷)ᵤ-(B⁸)ᵥ-R¹⁰;
B⁵, B⁶, B⁷ and B⁸ are independently of each other a C₆-C₂₄arylene group, which can optionally be substituted by G, or a C₂-C₃₀heteroarylene group, which can optionally be substituted by G;
s is 0 or 1, t is 0 or 1, u is 0 or 1, v is 0 or 1;
R¹⁰ is H, a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G;
and/or
two adjacent groups of the groups R¹, R³, R^{3'}, R^{3"}, R^{3'''}, R⁴, R⁵, R⁶, R^{6'}, R^{6"}, and R6''' may form together with the atoms to which they are bonded a ring structure, which can optionally be substituted by G;
a is 1, 2 or 3;
b is 1, 2 or 3;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or-C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₂₄alkyl group, a C₆-C₆₀aryl group, a C₆-C₆₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
z is 1;
wherein R⁷ is replaced by -(B₁)ₒ-(B₂)ₚ-(B₃)_{q}-(B₄)ᵣ-Z.

2. The heterocyclic derivative according to claim 1, wherein L¹ is single bond.

3. The heterocyclic derivative according to claim 2, wherein A is a heterocyclic group represented by formula (4), formula (5), formula (6), formula (7), formula (8), formula (9), formula (10), formula (11), formula (12), formula (13), formula (14), formula (15) or formula (38):

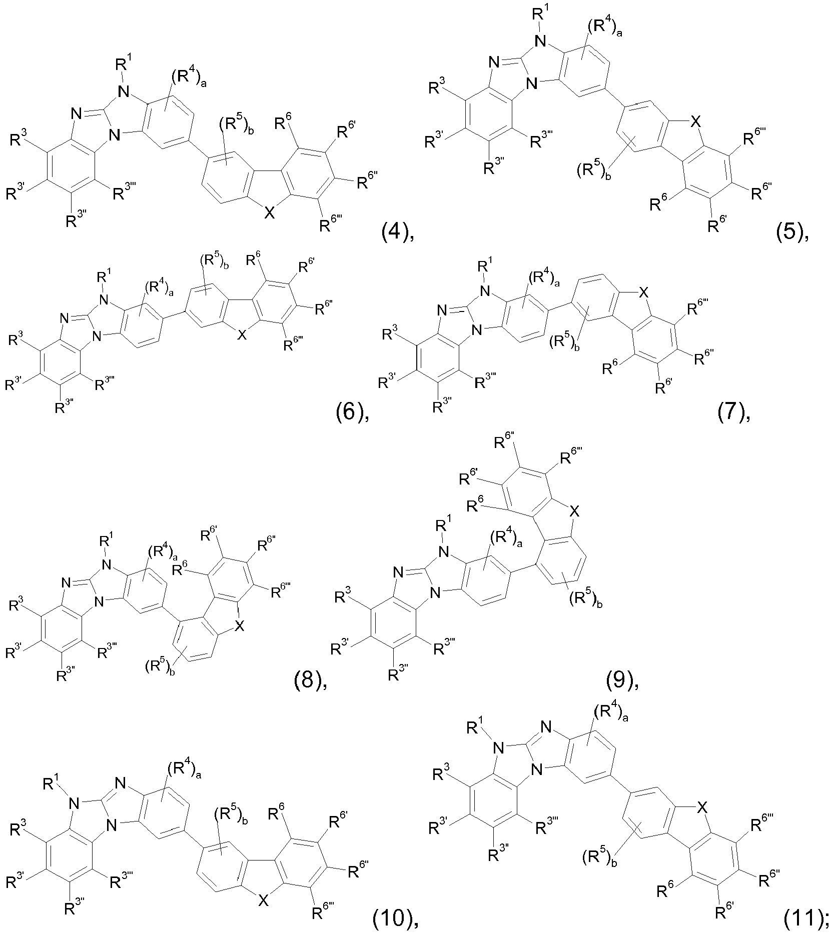

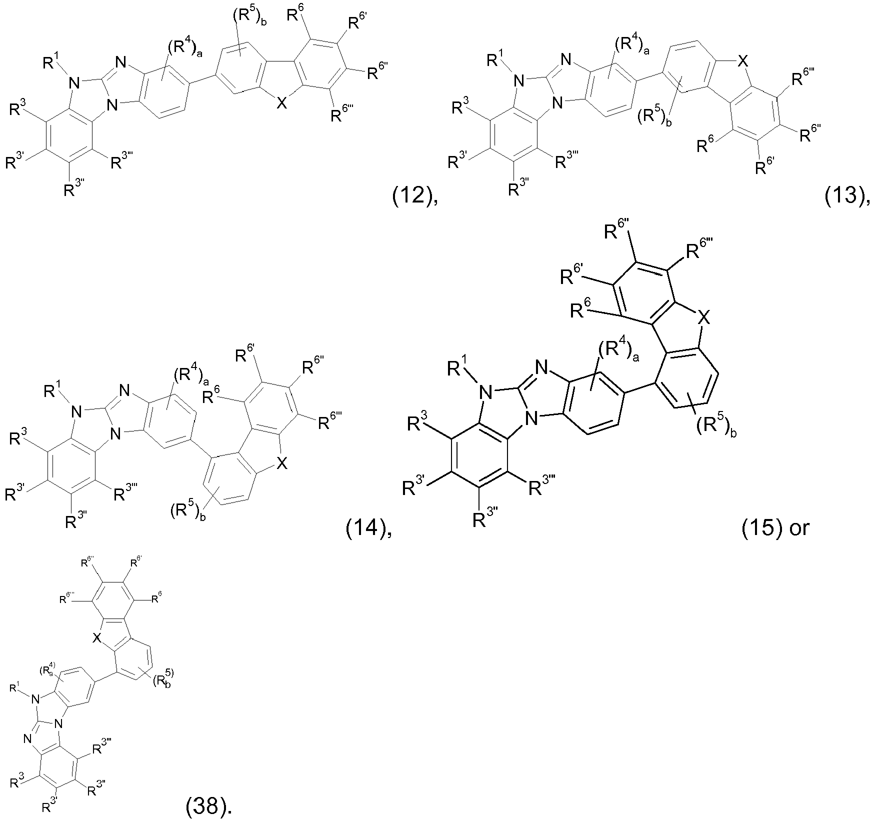

4. The heterocyclic derivative according to any one of claims 1 to 3, wherein B₁, B₂, B₃ and B₄
are independently of each other a C₆-C₂₄arylene group, which can optionally be substituted by G;
preferably 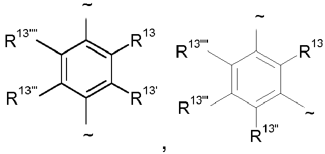 or 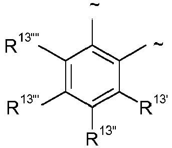 wherein
R¹³, R^{13'}, R^{13"}, R^{13'''}, or R^{13''''}
are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or-C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
∼ are bonding sites to the neighboring groups.

5. The heterocyclic derivative according to any one of claims 1 to 4, wherein o is 1, p is 0 or 1, preferably 0, and q and r are 0.

6. The heterocyclic derivative according to any one of claims 1 to 5, wherein Z is represented by one of the following formulae (16), (17), (18) or (19), preferably by one of formulae (16) or (18) 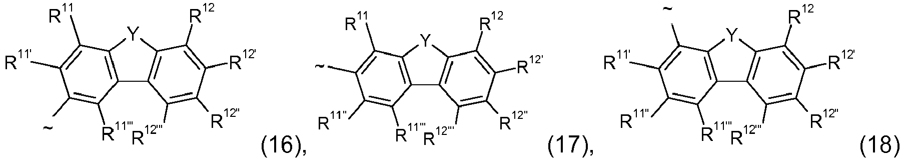 or 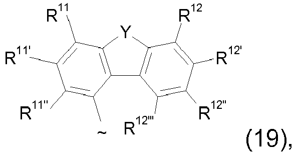 wherein
Y is O or S;
R¹¹, R^{11'}, R^{11"}, R^{11'''}, R¹², R^{12"} and R^{12'''}
are independently of each other H, a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
or
two adjacent groups R¹¹, R^{11'}, R^{11"}, R^{11'''}, R¹², R^{12"} and R^{12'''} may form together with the atoms to which they are bonded a ring structure;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or-C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
∼ is the bonding site to the group A-(B₁)ₒ-(B₂)ₚ-(B₃)_{q}-(B₄)ᵣ-.

7. An organic electronic device, comprising a compound according to any one of claims 1 to 6.

8. The electronic device according to claim 7, which is an organic electroluminescent device.

9. A hole transport layer, an electron/exciton blocking layer, or an emitting layer comprising a compound according to any one of claims 1 to 6.

10. The emitting layer according to claim 9, comprising a compound according to any one of claims 1 to 8 as host material in combination with a phosphorescent emitter.

11. An apparatus selected from the group consisting of stationary visual display units; mobile visual display units; illumination units; keyboards; items of clothing; furniture; wallpaper, comprising the organic electronic device according to claim 7 or 8, or the hole transport layer, the electron/exciton blocking layer, or the emitting layer according to claim 9.

12. Use of the compounds of formula (**1**) according to any one of claims 1 to 6 for organic electroluminescent devices, electrophotographic photoreceptors, photoelectric converters, organic solar cells, switching elements, organic light emitting field effect transistors, image sensors or dye lasers.

13. A process for the preparation of a heterocyclic derivative of formula (1) according to any one of claims 1 to 6 comprising:
a) Coupling of a group 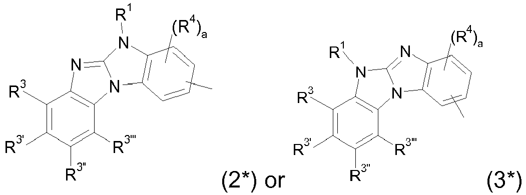 with a group 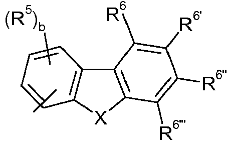 via a group L¹,
whereby a heterocyclic group A of formula (2) or formula (3) is obtained 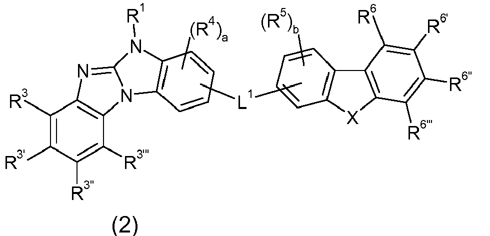 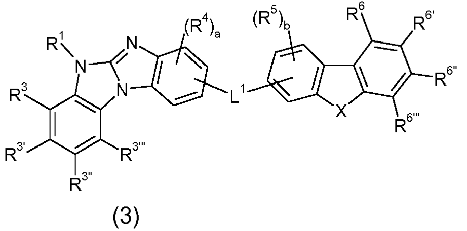 and
b) Introduction of one group -(B₁)ₒ-(B₂)ₚ-(B₃)_{q}-(B₄)ᵣ-Z into the heterocyclic group A of formula (2) or formula (3),
whereby a heterocyclic derivative of formula (1)
A-[(B₁)ₒ-(B₂)ₚ-(B₃)_{q}-(B₄)ᵣ-Z]_{z} (1)
is obtained,
wherein
B₁, B₂, B₃ and B₄
are independently of each other a C₆-C₂₄arylene group, which can optionally be substituted by G, or a 6 membered heteroarylene group, which can optionally be substituted by G; o is 0 or 1, p is 0 or 1, q is 0 or 1, r is 0 or 1, wherein at least one of o, p, q and r is 1;
Z represents
a dibenzofuran group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G; or
a dibenzothiophene group, which can optionally be substituted by a C₁-C₂₅alkyl group, which can optionally be substituted by E and/or interrupted by D; a C₆-C₂₄aryl group, which can optionally be substituted by G, or a C₁-C₂₄heteroaryl group, which can optionally be substituted by G;
and/or
two adjacent substituents of the dibenzofuran group or of the dibenzothiophene group may form together with the atoms to which they are bonded a ring structure, which can optionally be substituted by G;
A is a heterocyclic group represented by formula (2) or formula (3); 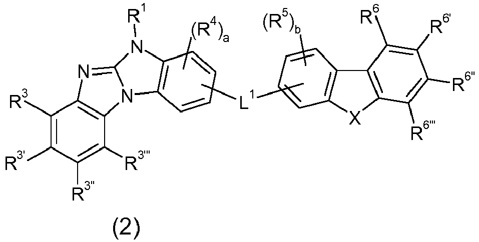 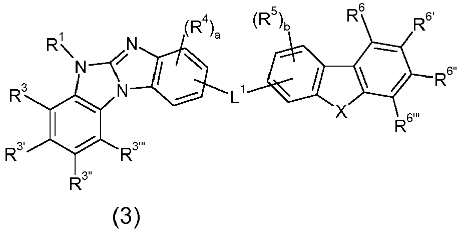
wherein X is NR⁷;
L¹ is single bond, a C₆-C₂₄arylene group, which can optionally be substituted by G, or a C₁-C₂₄heterocyclic group, which can optionally be substituted by G;
R¹, R³, R^{3'}, R^{3"}, R^{3'''}, R⁴, R⁵, R⁶, R^{6'}, R^{6"} and R^{6'''}
are independently of each other H or a group of formula -(B⁵)ₛ-(B⁶)ₜ-(B⁷)ᵤ-(B⁸)ᵥ-R¹⁰;
B⁵, B⁶, B⁷ and B⁸ are independently of each other a C₆-C₂₄arylene group, which can optionally be substituted by G, or a C₂-C₃₀heteroarylene group, which can optionally be substituted by G;
s is 0 or 1, t is 0 or 1, u is 0 or 1, v is 0 or 1;
R¹⁰ is H, a C₁-C₂₅alkyl group, which can optionally be substituted by E and or interrupted by D; a C₆-C₂₄aryl group, which can optionally be substituted by G, or a C₁-C₂₄heteroaryl group, which can optionally be substituted by G;
and/or
two adjacent groups of the groups R¹, R³, R^{3'}, R^{3"}, R^{3'''}, R⁴, R⁵, R⁶, R^{6'}, R^{6"}, and R6''' may form together with the atoms to which they are bonded a ring structure, which can optionally be substituted by G;
a is 1, 2 or 3;
b is 1, 2 or 3;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or-C≡C;
E is -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ or halogen;
G is E, or a C₁-C₂₄alkyl group, a C₆-C₆₀aryl group, a C₆-C₆₀aryl group, which is substituted by F, C₁-C₂₄alkyl, or C₁-C₂₄alkyl which is interrupted by O; a C₂-C₆₀heteroaryl group, or a C₂-C₆₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ may form together with the atom to which they are bonded a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl; z is 1;
wherein R⁷ is replaced by -(B₁)ₒ-(B₂)ₚ-(B₃)_{q}-(B₄)ᵣ-Z.

## Patentansprüche

1. Heterocyclisches Derivat der Formel (**1**);
**A-[(B₁)ₒ-(B₂)ₚ-(B₃)_{q}-(B₄)ᵣ-Z]_{z}** **(1)**
wobei
B₁, B₂, B₃ und B₄
unabhängig voneinander für eine C₆-C₂₄-Arylengruppe, die gegebenenfalls durch G substituiert sein kann, oder eine 6-gliedrige Heteroarylengruppe, die gegebenenfalls durch G substituiert sein kann, stehen;
o für 0 oder 1 steht, p für 0 oder 1 steht, q für 0 oder 1 steht, r für 0 oder 1 steht, wobei mindestens eine der Variablen o, p, q und r für 1 steht;
Z für
eine Dibenzofurangruppe, die gegebenenfalls durch eine C₁-C₂₅-Alkylgruppe, die gegebenenfalls durch E substituiert und/oder durch D unterbrochen sein kann, substituiert sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann, oder
eine Dibenzothiophengruppe, die gegebenenfalls durch eine C₁-C₂₅-Alkylgruppe, die gegebenenfalls durch E substituiert und/oder durch D unterbrochen sein kann, substituiert sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann,
steht und/oder
zwei benachbarte Substituenten der Dibenzofurangruppe oder der Dibenzothiophengruppe zusammen mit den Atomen, an die sie gebunden sind, eine Ringstruktur, die gegebenenfalls durch G substituiert sein kann, bilden können;
A für eine heterocyclische Gruppe steht, die durch Formel (2) oder Formel (3) wiedergegeben wird; 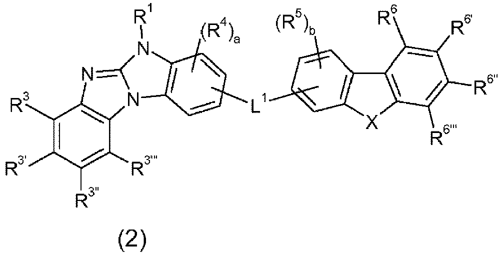 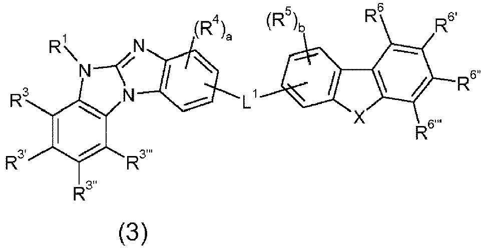
wobei X für NR⁷ steht;
L¹ für eine Einfachbindung, eine C₆-C₂₄-Arylengruppe, die gegebenenfalls durch G substituiert sein kann, oder eine C₁-C₂₄-heterocyclische Gruppe, die gegebenenfalls durch G substituiert sein kann, steht;
R¹, R³, R^{3'}, R^{3"}, R^{3'''}, R⁴, R⁵, R⁶, R^{6'}, R^{6"} und R^{6'''} unabhängig voneinander für H oder eine Gruppe der Formel -(B⁵)ₛ-(B⁶)ₜ-(B⁷)ᵤ-(B⁸)ᵥ-R¹⁰ stehen;
B⁵, B⁶, B⁷ und B⁸ unabhängig voneinander für eine C₆-C₂₄-Arylengruppe, die gegebenenfalls durch G substituiert sein kann, oder eine C₂-C₃₀-Heteroarylengruppe, die gegebenenfalls durch G substituiert sein kann, stehen;
s für 0 oder 1 steht, t für 0 oder 1 steht, u für 0 oder 1 steht, v für 0 oder 1 steht;
R¹⁰ für H, eine C₁-C₂₅-Alkylgruppe, die gegebenenfalls durch E substituiert und/oder durch D unterbrochen sein kann; eine C₆-C₂₄-Arylgruppe, die gegebenenfalls durch G substituiert sein kann, oder eine C₁-C₂₄-Heteroarylgruppe, die gegebenenfalls durch G substituiert sein kann, steht; und/oder
zwei benachbarte Gruppen der Gruppen R¹, R³, R^{3'}, R^{3"}, R^{3'''}, R⁴, R⁵, R⁶, R^{6'}, R^{6"} und R^{6'''} zusammen mit den Atomen, an die sie gebunden sind, eine Ringstruktur, die gegebenenfalls durch G substituiert sein kann, bilden können;
a für 1, 2 oder 3 steht;
b für 1, 2 oder 3 steht;
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C- steht;
E für -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ oder Halogen steht;
G für E oder eine C₁-C₂₄-Alkylgruppe, C₆-C₆₀-Arylgruppe, eine C₆-C₆₀-Arylgruppe, die durch F, C₁-C₂₄-Alkyl oder C₁-C₂₄-Alkyl, das durch O unterbrochen ist, substituiert ist, eine eine C₂-C₆₀-Heteroarylgruppe oder eine C₂-C₆₀-Heteroarylgruppe, die durch F, C₁-C₁₈-Alkyl oder C₁-C₁₈-Alkyl, das durch O unterbrochen ist, substituiert ist, steht; R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen; R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen; oder
R⁶⁵ und R⁶⁶ zusammen mit dem Atom, an das sie gebunden sind, einen fünf- oder sechsgliedrigen Ring bilden können;
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht;
z für 1 steht;
wobei R⁷ durch -(B₁)ₒ-(B₂)ₚ-(B₃)_{q}-(B₄)ᵣ-Z ersetzt ist.

2. Heterocyclisches Derivat nach Anspruch 1, wobei L¹ für eine Einfachbindung steht.

3. Heterocyclisches Derivat nach Anspruch 2, wobei A für eine heterocyclische Gruppe steht, die durch Formel (4), Formel (5), Formel (6), Formel (7), Formel (8), Formel (9), Formel (10), Formel (11), Formel (12), Formel (13), Formel (14), Formel (15) oder Formel (38) wiedergegeben wird: 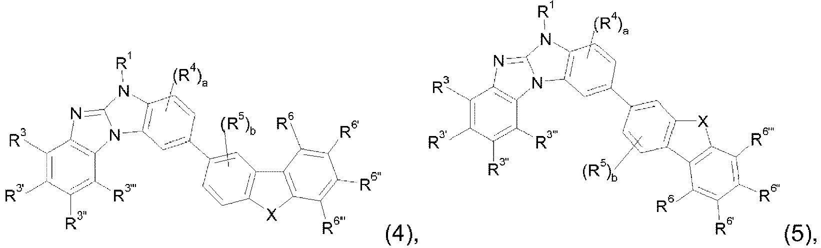 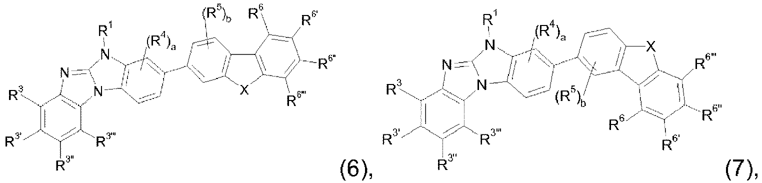 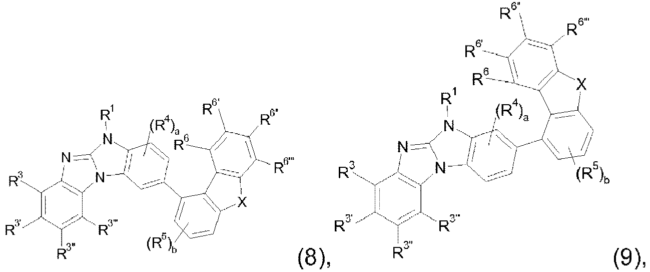 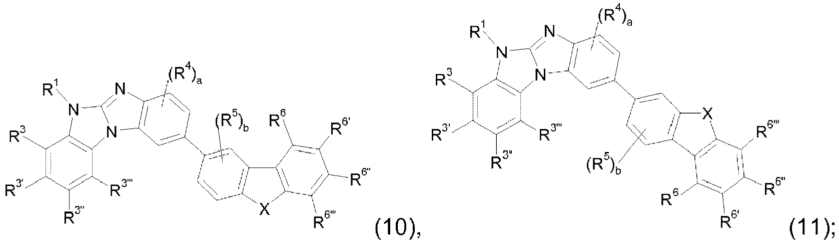 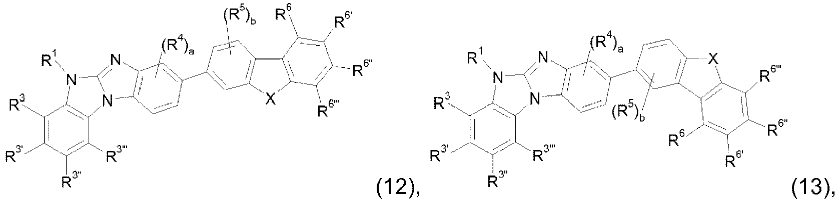 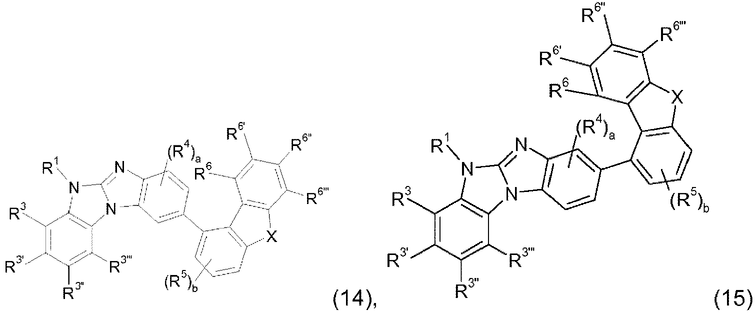 oder

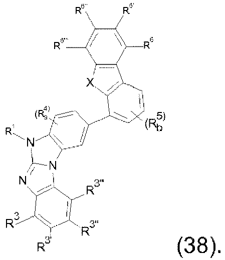

4. Heterocyclisches Derivat nach einem der Ansprüche 1 bis 3, wobei B₁, B₂, B₃ und B₄
unabhängig voneinander für eine C₆-C₂₄-Arylengruppe, die gegebenenfalls durch G substituiert sein kann;
vorzugsweise 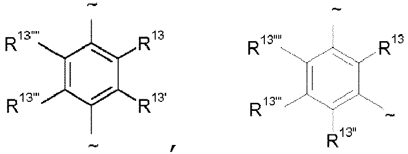 oder 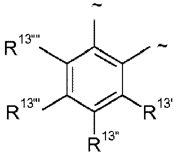 steht, wobei
R¹³, R^{13'}, R^{13"}, R^{13'''} oder R^{13''''} unabhängig voneinander für H, eine C₁-C₂₅-Alkylgruppe, die gegebenenfalls durch E substituiert und/oder durch D unterbrochen sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann, stehen;
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C- steht;
E für -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ oder Halogen steht;
G für E oder eine C₁-C₁₈-Alkylgruppe, eine C₆-C₂₄-Arylgruppe, eine C₆-C₂₄-Arylgruppe, die durch F, C₁-C₁₈-Alkyl oder C₁-C₁₈-Alkyl, das durch O unterbrochen ist, substituiert ist, eine C₂-C₃₀-Heteroarylgruppe oder eine C₂-C₃₀-Heteroarylgruppe, die durch F, C₁-C₁₈-Alkyl oder C₁-C₁₈-Alkyl, das durch O unterbrochen ist, substituiert ist, steht;
∼ für Bindungsstellen an die benachbarten Gruppen stehen.

5. Heterocyclisches Derivat nach einem der Ansprüche 1 bis 4, wobei o für 1 steht, p für 0 oder 1, vorzugsweise 0, steht und q und r für 0 stehen.

6. Heterocyclisches Derivat nach einem der Ansprüche 1 bis 5, wobei Z durch eine der folgenden Formeln (16), (17), (18) oder (19), vorzugsweise durch eine der Formeln (16) oder (18), wiedergegeben wird, 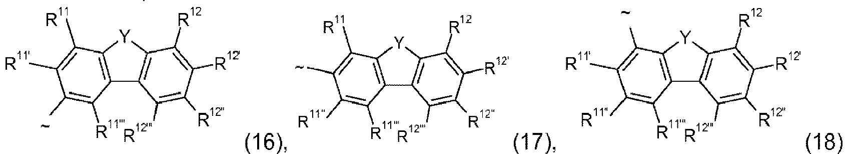 oder 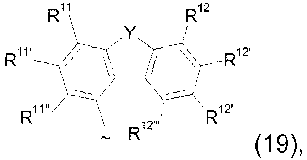 wobei
Y für O oder S steht;
R¹¹, R^{11'}, R^{11"}, R^{11'''}, R¹², R^{12"} und R^{12'''} unabhängig voneinander für H, eine C₁-C₂₅-Alkylgruppe, die gegebenenfalls durch E substituiert und/oder durch D unterbrochen sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann, stehen;
oder
zwei benachbarte Gruppen R¹¹, R^{11'}, R^{11"}, R^{11'''}, R¹², R^{12"} und R^{12'''} zusammen mit den Atomen, an die sie gebunden sind, eine Ringstruktur bilden können;
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C steht;
E für -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ oder Halogen steht;
G für E oder eine C₁-C₁₈-Alkylgruppe, eine C₆-C₂₄-Arylgruppe, eine C₆-C₂₄-Arylgruppe, die durch F, C₁-C₁₈-Alkyl oder C₁-C₁₈-Alkyl, das durch O unterbrochen ist, substituiert ist, eine C₂-C₃₀-Heter0-arylgruppe oder eine C₂-C₃₀-Heteroarylgruppe, die durch F, C₁-C₁₈-Alkyl oder C₁-C₁₈-Alkyl, das durch O unterbrochen ist, substituiert ist, steht;
∼ für die Bindungsstelle an die Gruppe A-(B₁)ₒ-(B₂)ₚ-(B₃)_{q}-(B₄)ᵣ steht.

7. Organische elektronische Vorrichtung, umfassend eine Verbindung nach einem der Ansprüche 1 bis 6.

8. Elektronische Vorrichtung nach Anspruch 7, bei der es sich um eine organische Elektrolumineszenzvorrichtung handelt.

9. Lochtransportschicht, Ladungs-/Exzitonen-Blockerschicht oder Emissionsschicht, umfassend eine Verbindung nach einem der Ansprüche 1 bis 6.

10. Emissionsschicht nach Anspruch 9, umfassend eine Verbindung nach einem der Ansprüche 1 bis 8 als Wirtsmaterial in Kombination mit einem phosphoreszierenden Emitter.

11. Apparatur, ausgewählt aus der Gruppe bestehend aus stationären Bildschirmen; mobilen Bildschirmen; Beleuchtungseinheiten; Tastaturen; Kleidungsstücken; Möbeln; Tapeten, umfassend die organische elektronische Vorrichtung nach Anspruch 7 oder 8 oder die Ladungstransportschicht, die Ladungs-/Exzitonen-Blockerschicht oder die Emissionsschicht nach Anspruch 9.

12. Verwendung der Verbindungen der Formel (**1**) nach einem der Ansprüche 1 bis 6 für Elektrolumineszenzvorrichtungen, elektrophotographische Photorezeptoren, photoelektrische Umwandler, organische Solarzellen, Schaltelemente, organische lichtemittierende Feldeffekttransistoren, Bildsensoren oder Farbstofflaser.

13. Verfahren zur Herstellung eines heterocyclischen Derivats der Formel (1) nach einem der Ansprüche 1 bis 6, umfassend:
a) Kuppeln einer Gruppe 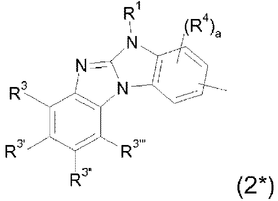 oder 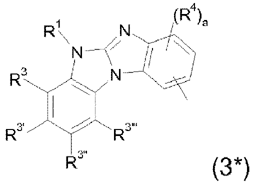 mit einer Gruppe 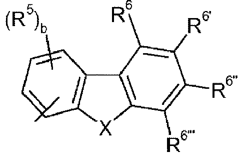 über eine Gruppe L¹, wodurch eine heterocyclische Gruppe A der Formel (2) oder Formel (3) erhalten wird, 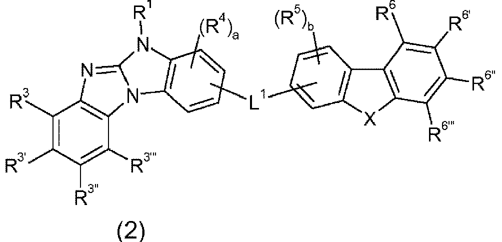 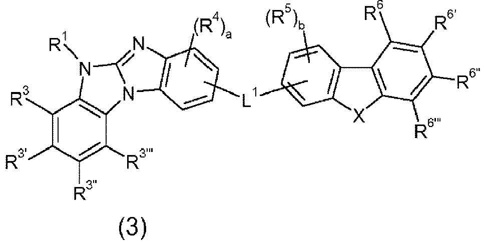 und
b) Einführung einer Gruppe -(B₁)ₒ-(B₂)ₚ-(B₃)_{q}-(B₄)ᵣ-Z in die heterocyclische Gruppe A der Formel (2) oder Formel (3),
wodurch ein heterocyclisches Derivat der Formel (1)
A-[(B₁)ₒ-(B₂)ₚ-(B₃)_{q}-(B₄)ᵣ-Z]_{z} (1)
erhalten wird,
wobei
B₁, B₂, B₃ und B₄
unabhängig voneinander für eine C₆-C₂₄-Arylengruppe, die gegebenenfalls durch G substituiert sein kann, oder eine 6-gliedrige Heteroarylengruppe, die gegebenenfalls durch G substituiert sein kann, stehen;
o für 0 oder 1 steht, p für 0 oder 1 steht, q für 0 oder 1 steht, r für 0 oder 1 steht, wobei mindestens eine der Variablen o, p, q und r für 1 steht;
Z für
eine Dibenzofurangruppe, die gegebenenfalls durch eine C₁-C₂₅-Alkylgruppe, die gegebenenfalls durch E substituiert und/oder durch D unterbrochen sein kann, substituiert sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann, oder
eine Dibenzothiophengruppe, die gegebenenfalls durch eine C₁-C₂₅-Alkylgruppe, die gegebenenfalls durch E substituiert und/oder durch D unterbrochen sein kann, substituiert sein kann, eine C₆-C₂₄-Arylgruppe, die gegebenenfalls durch G substituiert sein kann, oder eine C₁-C₂₄-Heteroarylgruppe, die gegebenenfalls durch G substituiert sein kann,
steht und/oder
zwei benachbarte Substituenten der Dibenzofurangruppe oder der Dibenzothiophengruppe zusammen mit den Atomen, an die sie gebunden sind, eine Ringstruktur, die gegebenenfalls durch G substituiert sein kann, bilden können;
A für eine heterocyclische Gruppe steht, die durch Formel (2) oder Formel (3) wiedergegeben wird; 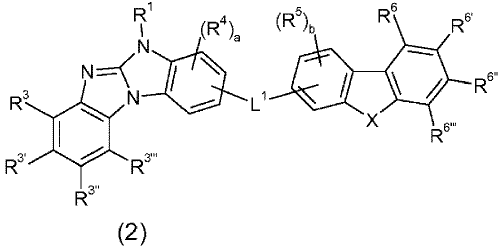 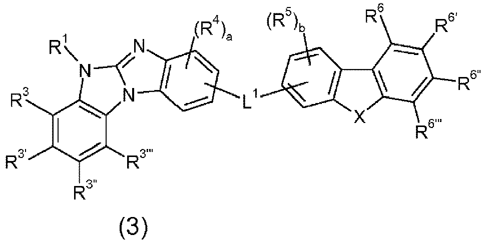
wobei X für NR⁷ steht;
L¹ für eine Einfachbindung, eine C₆-C₂₄-Arylengruppe, die gegebenenfalls durch G substituiert sein kann, oder eine C₁-C₂₄-heterocyclische Gruppe, die gegebenenfalls durch G substituiert sein kann, steht;
R¹, R³, R^{3'}, R^{3"}, R^{3'''}, R⁴, R⁵, R⁶, R^{6'}, R^{6"} und R^{6'''} unabhängig voneinander für H oder eine Gruppe der Formel -(B⁵)ₛ-(B⁶)ₜ-(B⁷)ᵤ-(B⁸)ᵥ-R¹⁰ stehen;
B⁵, B⁶, B⁷ und B⁸ unabhängig voneinander für eine C₆-C₂₄-Arylengruppe, die gegebenenfalls durch G substituiert sein kann, oder eine C₂-C₃₀-Heteroarylengruppe, die gegebenenfalls durch G substituiert sein kann, stehen;
s für 0 oder 1 steht, t für 0 oder 1 steht, u für 0 oder 1 steht, v für 0 oder 1 steht;
R¹⁰ für H, eine C₁-C₂₅-Alkylgruppe, die gegebenenfalls durch E substituiert und/oder durch D unterbrochen sein kann; eine C₆-C₂₄-Arylgruppe, die gegebenenfalls durch G substituiert sein kann, oder eine C₁-C₂₄-Heteroarylgruppe, die gegebenenfalls durch G substituiert sein kann, steht; und/oder
zwei benachbarte Gruppen der Gruppen R¹, R³, R^{3'}, R^{3"}, R^{3'''}, R⁴, R⁵, R⁶, R^{6'}, R^{6"} und R^{6'''} zusammen mit den Atomen, an die sie gebunden sind, eine Ringstruktur, die gegebenenfalls durch G substituiert sein kann, bilden können;
a für 1, 2 oder 3 steht;
b für 1, 2 oder 3 steht;
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C- steht;
E für -OR⁶⁹, -SR⁶⁹, -NR⁶⁵R⁶⁶, -COR⁶⁸, -COOR⁶⁷, -CONR⁶⁵R⁶⁶, -CN, -Si(R⁷⁰)₃ oder Halogen steht;
G für E oder eine C₁-C₂₄-Alkylgruppe, eine C₆-C₆₀-Arylgruppe, eine C₆-C₆₀-Arylgruppe, die durch F, C₁-C₂₄-Alkyl oder C₁-C₂₄-Alkyl, das durch O unterbrochen ist, substituiert ist, eine C₂-C₆₀-Heteroarylgruppe oder eine C₂-C₆₀-Heteroarylgruppe, die durch F, C₁-C₁₈-Alkyl oder C₁-C₁₈-Alkyl, das durch O unterbrochen ist, substituiert ist, steht;
R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen; R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen; oder
R⁶⁵ und R⁶⁶ zusammen mit dem Atom, an das sie gebunden sind, einen fünf- oder sechsgliedrigen Ring bilden können;
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht;
z für 1 steht;
wobei R⁷ durch -(B₁)ₒ-(B₂)ₚ-(B₃)_{q}-(B₄)ᵣ-Z ersetzt ist.

## Revendications

1. Dérivé hétérocyclique de formule (1) :
**A-[(B₁)ₒ-(B₂)ₚ-(B₃)_{q}-(B₄)ᵣ-Z]_{z}** **(1)**
B₁, B₂, B₃ et B₄
représentant indépendamment les uns des autres un groupe C₆₋₂₄-arylène qui peut éventuellement être substitué par G, ou un groupe hétéroarylène à 6 chaînons, qui peut éventuellement être substitué par G ;
o étant 0 ou 1, p étant 0 ou 1, q étant 0 ou 1, r étant 0 ou 1, au moins l'un parmi o, p, q et r étant 1 ;
Z représentant
un groupe dibenzofuranne, qui peut éventuellement être substitué par un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ; ou
un groupe dibenzothiophène, qui peut éventuellement être substitué par un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ;
et/ou
deux substituants adjacents du groupe dibenzofuranne ou du groupe dibenzothiophène pouvant former une structure cyclique conjointement avec les atomes auxquels ils sont liés, qui peut éventuellement être substituée par G ;
A représentant un groupe hétérocyclique représenté par la formule (2) ou la formule (3) ; 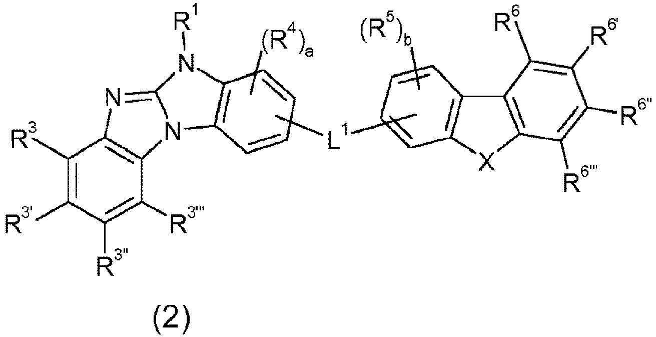 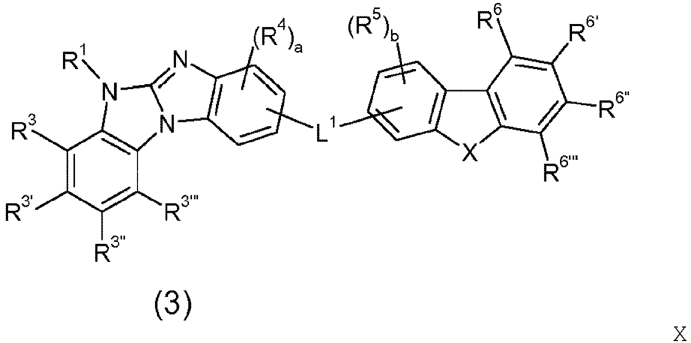
représentant NR⁷ ;
L¹ représentant une simple liaison, un groupe C₆₋₂₄-arylène, qui peut éventuellement être substitué par G, ou un groupe C₁₋₂₄-hétérocyclique, qui peut éventuellement être substitué par G ;
R¹, R³, R^{3'}, R^{3"}, R^{3'''}, R⁴, R⁵, R⁶, R^{6'}, R^{6"} et R^{6'''} représentant indépendamment les uns des autres H ou un groupe de formule -(B⁵)ₛ-(B⁶)ₜ-(B⁷)ᵤ-(B⁸)ᵥ-R¹⁰ ; B⁵, B⁶, B⁷ et B⁸ représentant indépendamment les uns des autres un groupe C₆₋₂₄₋arylène, qui peut éventuellement être substitué par G, ou un groupe C₂₋₃₀-hétéroarylène, qui peut éventuellement être substitué par G ;
s étant 0 ou 1, t étant 0 ou 1, u étant 0 ou 1, v étant 0 ou 1 ;
R¹⁰ représentant H, un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ;
et/ou
deux groupes adjacents aux groupes R¹, R³, R^{3'}, R^{3"}, R^{3'''}, R⁴, R⁵, R⁶, R^{6'}, R^{6"} et R^{6'''} pouvant former une structure cyclique conjointement avec les atomes auxquels ils sont liés, qui peut éventuellement être substituée par G ;
a étant 1, 2 ou 3 ;
b étant 1, 2 ou 3 ;
D représentant -CO-, -COO-, -S-, -SO-, -SO₂-, -O-, -NR⁶⁵-, -SiR⁷⁰R⁷¹-, -POR⁷²-, -CR⁶³=CR⁶⁴- ou -C≡C ;
E représentant -OR⁶⁹, -SR⁶⁹, -NR⁶⁵R⁶⁶, -COR⁶⁸,-COOR⁶⁷, -CONR⁶⁵R⁶⁶, -CN, -Si(R⁷⁰)₃ ou halogène ;
G représentant E ou un groupe C₁₋₂₄-alkyle, un groupe C₆₋₆₀-aryle, un groupe C₆₋₆₀-aryle, qui est substitué par F, C₁₋₂₄-alkyle ou C₁₋₂₄-alkyle qui est interrompu par O ; un groupe C₂₋₆₀-hétéroaryle ou un groupe C₂₋₆₀-hétéroaryle, qui est substitué par F, C₁₋₁₈-alkyle ou C₁₋₁₈-alkyle qui est interrompu par O ;
R⁶³ et R⁶⁴ représentant indépendamment l'un de l'autre H, C₆₋₁₈-aryle ; C₆₋₁₈-aryle qui est substitué par C₁₋₁₈-alkyle ou C₁₋₁₈-alcoxyle ; C₁₋₁₈-alkyle ; ou C₁₋₁₈-alkyle qui est interrompu par -O- ;
R⁶⁵ et R⁶⁶ représentant indépendamment l'un de l'autre un groupe C₆₋₁₈-aryle ; un C₆₋₁₈-aryle qui est substitué par C₁₋₁₈-alkyle ou C₁₋₁₈-alcoxyle ; un groupe C₁₋₁₈-alkyle ; ou un groupe C₁₋₁₈-alkyle qui est interrompu par -O- ; ou
R⁶⁵ et R⁶⁶ pouvant former un cycle à cinq ou six chaînons conjointement avec l'atome auquel ils sont liés,
R⁶⁷ représentant un groupe C₆₋₁₈-aryle ; un groupe C₆₋₁₈-aryle, qui est substitué par C₁₋₁₈-alkyle ou C₁₋₁₈-alcoxyle ; un groupe C₁₋₁₈-alkyle ; ou un groupe C₁₋₁₈-alkyle qui est interrompu par -O-,
R⁶⁸ représentant H ; un groupe C₆₋₁₈-aryle ; un groupe C₆₋₁₈-aryle, qui est substitué par C₁₋₁₈-alkyle ou C₁₋₁₈-alcoxyle ; un groupe C₁₋₁₈-alkyle ; ou un groupe C₁₋₁₈-alkyle qui est interrompu par -O-,
R⁶⁹ représentant un C₆₋₁₈-aryle ; un C₆₋₁₈-aryle, qui est substitué par C₁₋₁₈-alkyle ou C₁₋₁₈-alcoxyle ; un groupe C₁₋₁₈-alkyle ; ou un groupe C₁₋₁₈-alkyle, qui est interrompu par -O-,
R⁷⁰ et R⁷¹ représentant indépendamment l'un de l'autre un groupe C₁₋₁₈-alkyle, un groupe C₆₋₁₈-aryle ou un groupe C₆₋₁₈-aryle, qui est substitué par C₁₋₁₈-alkyle et
R⁷² représentant un groupe C₁₋₁₈-alkyle, un groupe C₆₋₁₈-aryle ou un groupe C₆₋₁₈-aryle, qui est substitué par C₁₋₁₈-alkyle ;
z étant 1 ;
R⁷ étant remplacé par -(B₁)ₒ-(B₂)ₚ-(B₃)_{q}-(B₄)ᵣ-Z.

2. Dérivé hétérocyclique selon la revendication 1, L¹ représentant une simple liaison.

3. Dérivé hétérocyclique selon la revendication 2, A représentant un groupe hétérocyclique représenté par la formule (4), la formule (5), la formule (6), la formule (7), la formule (8), la formule (9), la formule (10), la formule (11), la formule (12), la formule (13), la formule (14), la formule (15) ou la formule (38) : 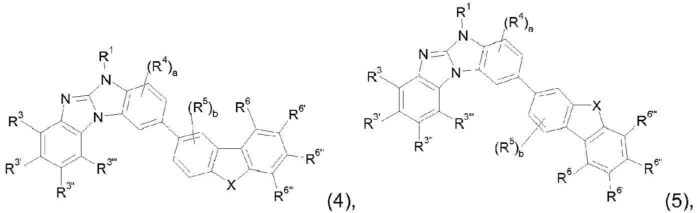 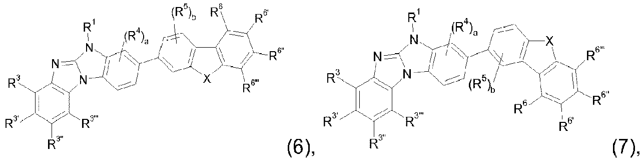 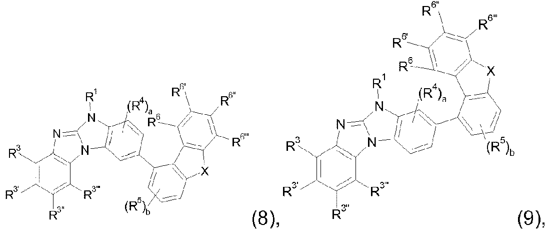 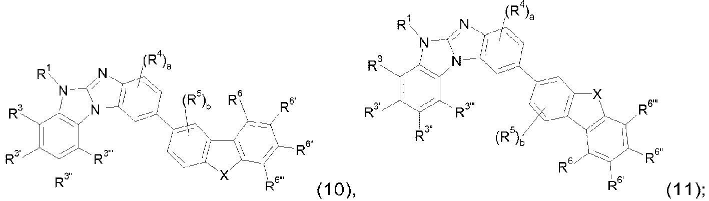 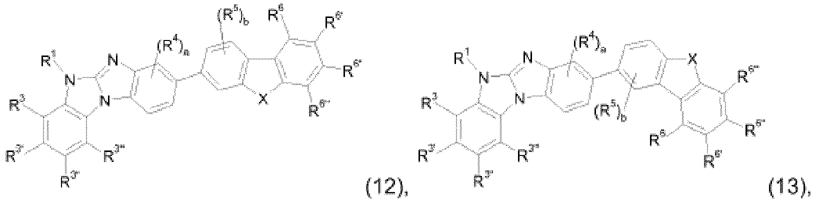 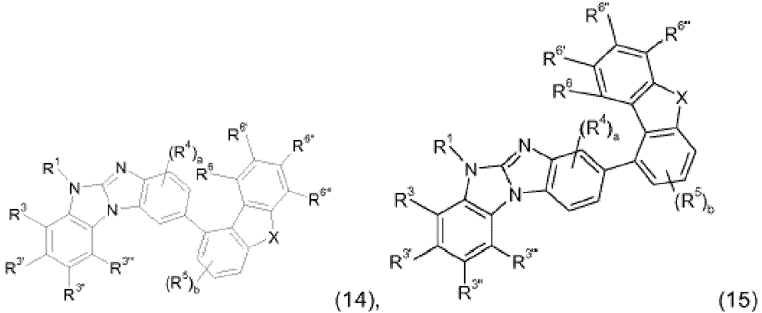 ou

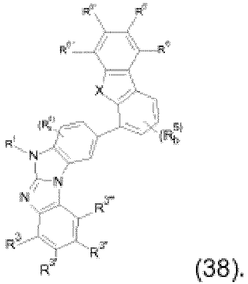

4. Dérivé hétérocyclique selon l'une quelconque des revendications 1 à 3, B₁, B₂, B₃ et B₄ représentant indépendamment les uns des autres un groupe C₆₋₂₄₋arylène, qui peut éventuellement être substitué par G ;
préférablement 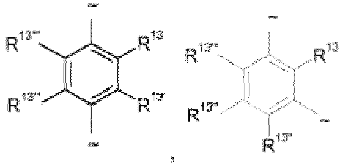 ou 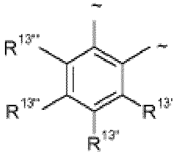
R¹³, R^{13'}, R^{13"}, R^{13'''} ou R^{13''''}
représentant indépendamment les uns des autres H, un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ;
D représentant -CO-, -COO-, -S-, -SO-, -SO₂-, -O-, -NR⁶⁵-, -SiR⁷⁰R⁷¹-, -POR⁷²-, -CR⁶³=CR⁶⁴- ou -C≡C ;
E représentant -OR⁶⁹, -SR⁶⁹, -NR⁶⁵R⁶⁶, -COR⁶⁸,-COOR⁶⁷, -CONR⁶⁵R⁶⁶, -CN, -Si(R⁷⁰)₃ ou halogène ;
G représentant E ou un groupe C₁₋₁₈-alkyle, un groupe C₆₋₂₄-aryle, un groupe C₆₋₂₄-aryle, qui est substitué par F, C₁₋₁₈-alkyle ou C₁₋₁₈-alkyle qui est interrompu par O ; un groupe C₂₋₃₀-hétéroaryle ou un groupe C₂₋₃₀-hétéroaryle, qui est substitué par F, C₁₋₁₈-alkyle ou C₁₋₁₈-alkyle qui est interrompu par O ;
∼ représentant des sites de liaison aux groupes voisins.

5. Dérivé hétérocyclique selon l'une quelconque des revendications 1 à 4, o étant 1, p étant 0 ou 1, préférablement 0 et q et r étant 0.

6. Dérivé hétérocyclique selon l'une quelconque des revendications 1 à 5, Z étant représenté par une des formules suivantes (16), (17), (18) ou (19), préférablement par une des formules (16) ou (18) 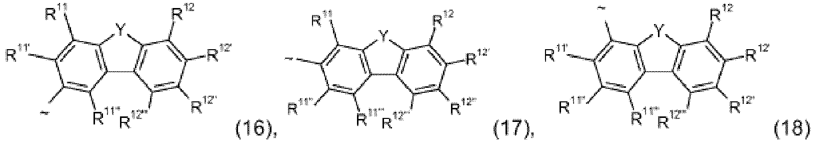 ou 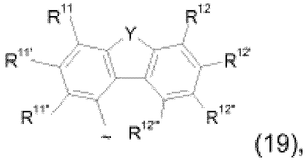
Y représentant O ou S ;
R¹¹, R^{11'}, R^{11"}, R^{11'''}, R¹², R^{12"} et R^{12'''} représentant indépendamment les uns des autres H, un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ;
ou
deux groupes adjacents R¹¹, R^{11'}, R^{11"}, R^{11'''}, R¹², R^{12"} et R^{12'''} pouvant former une structure cyclique conjointement avec les atomes auxquels ils sont liés ;
D représentant -CO-, -COO-, -S-, -SO-, -SO₂-, -O-, -NR⁶⁵-, -SiR⁷⁰R⁷¹-, -POR⁷²-, -CR⁶³=CR⁶⁴- ou -C≡C ;
E représentant -OR⁶⁹, -SR⁶⁹, -NR⁶⁵R⁶⁶, -COR⁶⁸,-COOR⁶⁷, -CONR⁶⁵R⁶⁶, -CN, -Si (R⁷⁰)₃ ou halogène ;
G représentant E ou un groupe C₁₋₁₈-alkyle, un groupe C₆₋₂₄-aryle, un groupe C₆₋₂₄-aryle, qui est substitué par F, C₁₋₁₈-alkyle ou C₁₋₁₈-alkyle qui est interrompu par O ; un groupe C₂₋₃₀-hétéroaryle ou un groupe C₂₋₃₀-hétéroaryle, qui est substitué par F, C₁₋₁₈-alkyle ou C₁₋₁₈-alkyle qui est interrompu par O ;
∼ représentant le site de liaison au groupe A-(B₁)ₒ-(B₂)ₚ-(B₃)_{q}-(B₄)ᵣ-.

7. Dispositif électronique organique, comprenant un composé selon l'une quelconque des revendications 1 à 6.

8. Dispositif électronique selon la revendication 7, qui est un dispositif électroluminescent organique.

9. Couche de transport de trou, couche de blocage d'électron/exciton ou couche émettrice comprenant un composé selon l'une quelconque des revendications 1 à 6.

10. Couche émettrice selon la revendication 9, comprenant un composé selon l'une quelconque des revendications 1 à 8 en tant que matériau hôte en combinaison avec un émetteur phosphorescent.

11. Appareil choisi dans le groupe constitué par les unités d'affichage visuel stationnaires ; les unités d'affichage visuel mobiles ; les unités d'éclairage ; les claviers ; les articles d'habillement ; les meubles ; le papier peint, comprenant le dispositif électronique organique selon la revendication 7 ou 8 ou la couche de transport de trou, la couche de blocage d'électron/exciton ou la couche émettrice selon la revendication 9.

12. Utilisation des composés de formule (1) selon l'une quelconque des revendications 1 à 6 pour des dispositifs électroluminescents organiques, des photorécepteurs électrophotographiques, des convertisseurs photoélectriques, des cellules solaires organiques, des éléments de commutation, des transistors organiques luminescents à effet de champ, des capteurs d'image ou des lasers à colorant.

13. Procédé pour la préparation d'un dérivé hétérocyclique de formule (1) selon l'une quelconque des revendications 1 à 6 comprenant :
a) le couplage d'un groupe 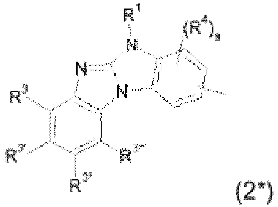 ou 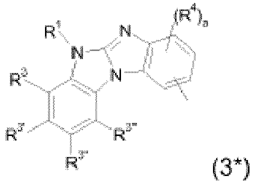 avec un groupe 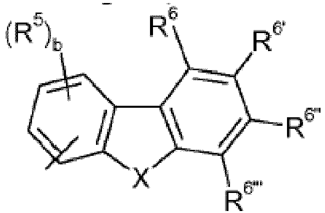 via un groupe L¹,
un groupe hétérocyclique A de formule (2) ou de formule (3) étant obtenu 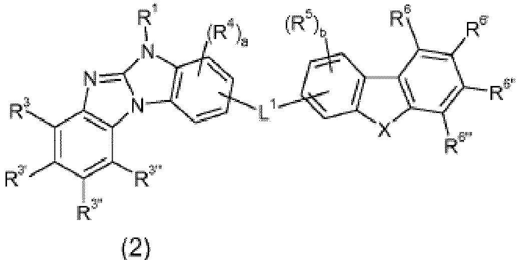 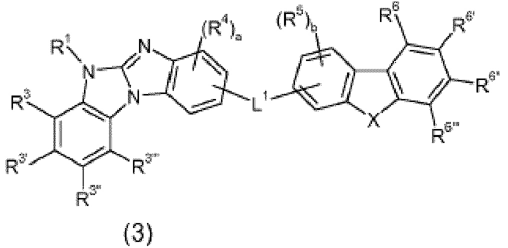 et
b) l'introduction d'un groupe - (B₁)ₒ-(B₂)ₚ-(B₃)_{q}-(B₄)ᵣ-Z dans le groupe hétérocyclique A de la formule (2) ou de la formule (3),
un dérivé hétérocyclique de formule (1)
**A-[(B₁)ₒ-(B₂)ₚ-(B₃)_{q}-(B₄)ᵣ-Z]_{z}** **(1)**
étant obtenu,
B₁, B₂, B₃ et B₄
représentant indépendamment les uns des autres un groupe C₆₋₂₄-arylène qui peut éventuellement être substitué par G, ou un groupe hétéroarylène à 6 chaînons, qui peut éventuellement être substitué par G ;
o étant 0 ou 1, p étant 0 ou 1, q étant 0 ou 1, r étant 0 ou 1, au moins l'un parmi o, p, q et r étant 1 ;
Z représentant
un groupe dibenzofuranne, qui peut éventuellement être substitué par un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ; ou
un groupe dibenzothiophène, qui peut éventuellement être substitué par un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ;
et/ou
deux substituants adjacents du groupe dibenzofuranne ou du groupe dibenzothiophène pouvant former une structure cyclique conjointement avec les atomes auxquels ils sont liés, qui peut éventuellement être substituée par G ;
A représentant un groupe hétérocyclique représenté par la formule (2) ou la formule (3) ; 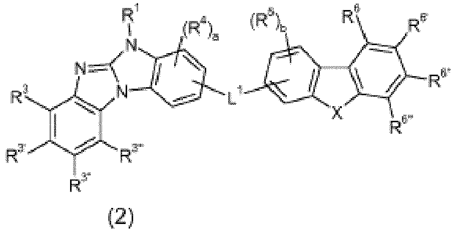 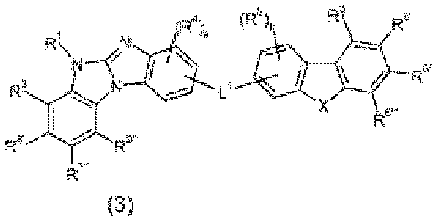
X représentant NR⁷ ;
L¹ représentant une simple liaison, un groupe C₆₋₂₄-arylène, qui peut éventuellement être substitué par G, ou un groupe C₁₋₂₄-hétérocyclique, qui peut éventuellement être substitué par G ;
R¹, R³, R^{3'}, R^{3"}, R^{3'''}, R⁴, R⁴, R⁶, R^{6'}, R^{6"} et R^{6'''} représentant indépendamment les uns des autres H ou un groupe de formule - (B⁵)ₛ-(B⁶)ₜ-(B⁷)ᵤ-(B⁸)ᵥ-R¹⁰ ; B⁵, B⁶, B⁷ et B⁸ représentant indépendamment les uns des autres un groupe C₆₋₂₄₋arylène, qui peut éventuellement être substitué par G, ou un groupe C₂₋₃₀-hétéroarylène, qui peut éventuellement être substitué par G ;
s étant 0 ou 1, t étant 0 ou 1, u étant 0 ou 1, v étant 0 ou 1 ;
R¹⁰ représentant H, un groupe C₁₋₂₅-alkyle, qui peut éventuellement être substitué par E et/ou interrompu par D ; un groupe C₆₋₂₄-aryle, qui peut éventuellement être substitué par G, ou un groupe C₁₋₂₄-hétéroaryle, qui peut éventuellement être substitué par G ;
et/ou
deux groupes adjacents aux groupes R¹, R³, R^{3'}, R^{3"}, R^{3'''}, R⁴, R⁵, R⁶, R^{6'}, R^{6"} et R^{6'''} pouvant former une structure cyclique conjointement avec les atomes auxquels ils sont liés, qui peut éventuellement être substituée par G ;
a étant 1, 2 ou 3 ;
b étant 1, 2 ou 3 ;
D représentant -CO-, -COO-, -S-, -SO-, -SO₂-, -O-, -NR⁶⁵-, -SiR⁷⁰R⁷¹-, -POR⁷²-, -CR⁶³=CR⁶⁴- ou -C≡C ;
E représentant -OR⁶⁹, -SR⁶⁹, -NR⁶⁵R⁶⁶, -COR⁶⁸,-COOR⁶⁷, -CONR⁶⁵R⁶⁶, -CN, -Si(R⁷⁰)₃ ou halogène ;
G représentant E ou un groupe C₁₋₂₄-alkyle, un groupe C₆₋₆₀-aryle, un groupe C₆₋₆₀-aryle, qui est substitué par F, C₁₋₂₄-alkyle ou C₁₋₂₄-alkyle qui est interrompu par O ; un groupe C₂₋₆₀-hétéroaryle ou un groupe C₂₋₆₀-hétéroaryle, qui est substitué par F, C₁₋₁₈-alkyle ou C₁₋₁₈-alkyle qui est interrompu par O ;
R⁶³ et R⁶⁴ représentant indépendamment l'un de l'autre H, C₆₋₁₈-aryle ; C₆₋₁₈-aryle qui est substitué par C₁₋₁₈-alkyle ou C₁₋₁₈-alcoxyle ; C₁₋₁₈-alkyle ; ou C₁₋₁₈-alkyle qui est interrompu par -O- ;
R⁶⁵ et R⁶⁶ représentant indépendamment l'un de l'autre un groupe C₆₋₁₈-aryle ; un C₆₋₁₈-aryle qui est substitué par C₁₋₁₈-alkyle ou C₁₋₁₈-alcoxyle ; un groupe C₁₋₁₈-alkyle ; ou un groupe C₁₋₁₈-alkyle qui est interrompu par -O- ; ou
R⁶⁵ et R⁶⁶ pouvant former un cycle à cinq ou six chaînons conjointement avec l'atome auquel ils sont liés,
R⁶⁷ représentant un groupe C₆₋₁₈-aryle ; un groupe C₆₋₁₈-aryle, qui est substitué par C₁₋₁₈-alkyle ou C₁₋₁₈-alcoxyle ; un groupe C₁₋₁₈-alkyle ; ou un groupe C₁₋₁₈-alkyle qui est interrompu par -O-,
R⁶⁸ représentant H ; un groupe C₆₋₁₈-aryle ; un groupe C₆₋₁₈-aryle, qui est substitué par C₁₋₁₈-alkyle ou C₁₋₁₈-alcoxyle ; un groupe C₁₋₁₈-alkyle ; ou un groupe C₁₋₁₈-alkyle qui est interrompu par -O-,
R⁶⁹ représentant un C₆₋₁₈-aryle ; un C₆₋₁₈-aryle, qui est substitué par C₁₋₁₈-alkyle ou C₁₋₁₈-alcoxyle ; un groupe C₁₋₁₈-alkyle ; ou un groupe C₁₋₁₈-alkyle, qui est interrompu par -O-,
R⁷⁰ et R⁷¹ représentant indépendamment l'un de l'autre un groupe C₁₋₁₈-alkyle, un groupe C₆₋₁₈-aryle, ou un groupe C₆₋₁₈-aryle qui est substitué par C₁₋₁₈-alkyle et
R⁷² représentant un groupe C₁₋₁₈-alkyle, un groupe C₆₋₁₈-aryle, ou un groupe C₆₋₁₈-aryle qui est substitué par C₁₋₁₈-alkyle ;
z étant 1 ;
R⁷ étant remplacé par -(B₁)ₒ-(B₂)ₚ-(B₃)_{q}-(B₄)ᵣ-Z.
